(19) Europäisches Patentamt
European Patent Office
Office européen des brevets

(11) Publication number: **0 005 349**
**A1**

(12) # EUROPEAN PATENT APPLICATION

(21) Application number: **79300712.1**

(22) Date of filing: **26.04.79**

(51) Int. Cl.[2]: **C 07 D 487/04,** A 61 K 31/40 // (C07D487/04, 209/00, 205/00),(A61K31/40, 31/43), (A61K31/40, 31/545)

(30) Priority: **06.05.78 GB 1810178**
**30.05.78 GB 2435878**
**22.12.78 GB 4984178**
**26.01.79 GB 7902920**

(43) Date of publication of application: **14.11.79**
**Bulletin 79/23**

(84) Designated Contracting States: **BE CH DE FR GB IT LU NL SE**

(71) Applicant: **BEECHAM GROUP LIMITED, Beecham House Great West Road, Brentford, Middlesex (GB)**

(72) Inventor: **Corbett, David Francis, Dingle Bank Pilgrims Way West Humble, Dorking Surrey (GB)**
Inventor: **Eglington, Alfred John, 66 Nutwood Avenue Brockham, Betchworth Surrey (GB)**

(74) Representative: **Cole, William Gwyn et al, Beecham Pharmaceuticals Yew Tree Bottom Road, Epsom Surrey KT18 5XQ (GB)**

(54) **Alkylcarbapenems, their preparation, use in pharmaceutical compositions and intermediates.**

(57) This invention provides the antibacterial compounds of the formula (I):

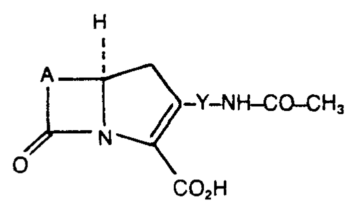

(I)

and salts and cleavable esters thereof wherein A is a $C{=}CH{-}CH_3$ or $CH{-}CH_2{-}CH_3$ group and Y is a $-S-CH_2-CH_2-$, $-S-CH{=}CH-$ or $-SO-CH{=}CH-$ group.

The invention also relates to process for the preparation of the said compounds and pharmaceutical compositions containing them.

EP 0 005 349 A1

0005349



# Alkylcarbapenems, their preparation, use in pharmaceutical compositions and intermediates

British Patent Specifications Nos. 1467413 1489235 and 1483142 disclose carbapenem antibiotics designated MM4550, MM13902 and MM17880. United States Patent Specification No. 3950375 discloses a further carbapenem antibiotic designated thienamycin. Belgian Patent Specification No. 864570 discloses substantially pure carbapenem antibiotics containing 6-hydroxyethyl substituents. Belgian Patent No. 865578 discloses that a 2-(2-acetamidoethylthio)-3-carboxy-6-ethyl-7-oxo-1-carba-2-penem is a metabolite of Streptomyces A271. A group of carbapenem antibiotics which were produced semi-synthetically have now been found.

The present invention provides the compounds of the formula (I):

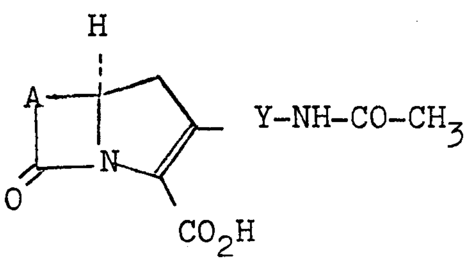

(I)

and salts and cleavable esters thereof wherein A is a $C{=}CH{-}CH_3$ or $CH{-}CH_2{-}CH_3$ group and Y is a $-S-CH_2-CH_2-$, $-S-CH{=}CH-$ or $-SO-CH{=}CH-$ group excluding substantially pure biologically produced (5R, 6R)-3-(2-acetamidoethylthio)-6-ethyl-7-oxo-1-azabicyclo [3.2.0]hept-2-ene-2-carboxylic acid and its salts. When used herein the term "naturally occuring" means obtained as a product of a microbiological process without subsequent chemical modification of the 6-position substituent.

The compounds of the formula (I) and their salts and esters wherein A is a $CH-CH_2-CH_3$ group are envisaged as antibacterial agents whereas the compounds of the formula (I) and their salts and esters wherein A is a $C=CH-CH_3$ group are envisaged primarily as chemical intermediates although they also possess antibacterial activity. Aptly Y is a $-S-CH_2-CH_2-$ , trans -S-CH=CH- or trans -SO-CH=CH-, or cis -SCH=CH- group.

Certain favoured anti-bacterial agents within formula (I) include the compounds of the formulae (II) - (V)

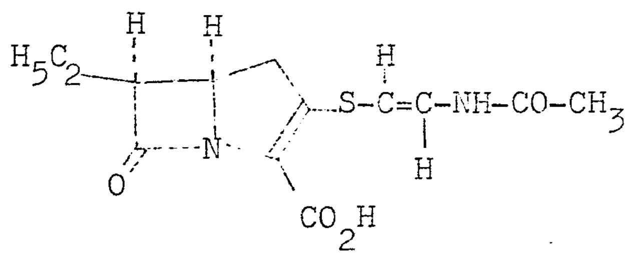

(II)

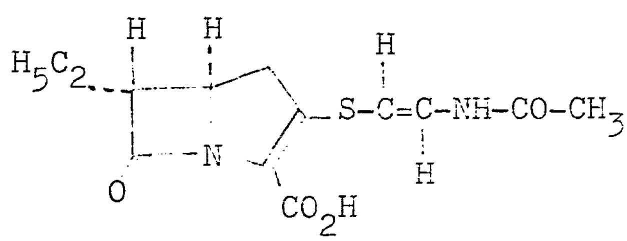

(III)

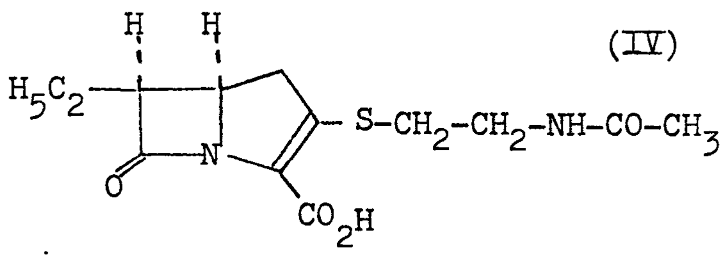

(IV)

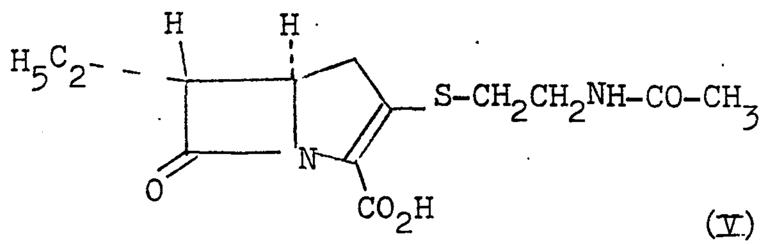

(V)

and pharmaceutically acceptable salts and in-vivo hydrolysable esters thereof with the proviso that the compound of the formula (V) or its salt or ester has been chemically produced (that is produced by chemical modification of the 6-position substituent).

The compounds of the formulae (II) - (V) are suitably provided in the form of an in-vivo hydrolysable ester.

The compounds of the formulae (II) - (V) are favourably provided in the form of a pharmaceutically acceptable salt or the free acid. The compounds of the formulae (II) - (V) are preferably in the form of a pharmaceutically acceptable salt, for example as a pharmaceutically acceptable alkali metal or alkaline earth metal salt. Preferred salts of the compounds of the formulae (II) - (V) include the sodium and potassium salts.

The compounds of the formulae (II) and (III) are particularly apt so they and their pharmaceutically acceptable salts and in-vivo hydrolysable esters form a particularly suitable aspect of this invention.

Since the compounds of the formula (II) - (V) and more especially their pharmaceutically acceptable salts are for use in medicaments it is preferably that they are provided in substantially pure form, for example at least 80% w/w and more suitably at least 90% w/w pure.

The compound of the formula (I) wherein A is a $CH-CH_2-CH_3$ group can have either the <u>cis</u>- or <u>trans</u>-configuration about the β-lactam ring. It is preferred that the <u>cis</u>- form of such compounds is provided substantially free of the <u>trans</u>- form and the <u>trans</u>-form is provided substantially free of the <u>cis</u>- form.

Certain favoured intermediates within formula (I) include the compounds of the formulae (VI) - (IX)

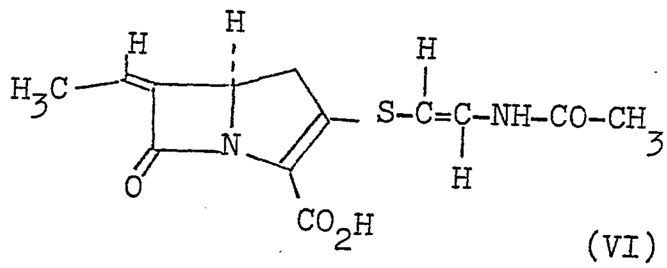

(VI)

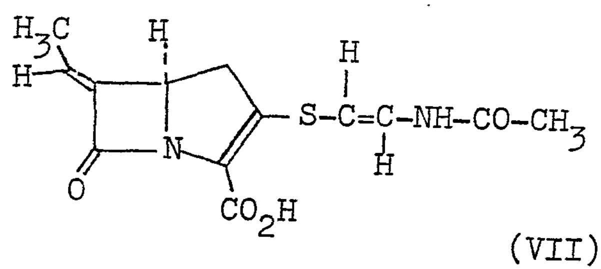

(VII)

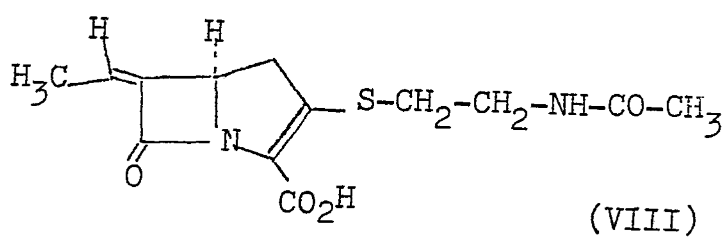

(VIII)

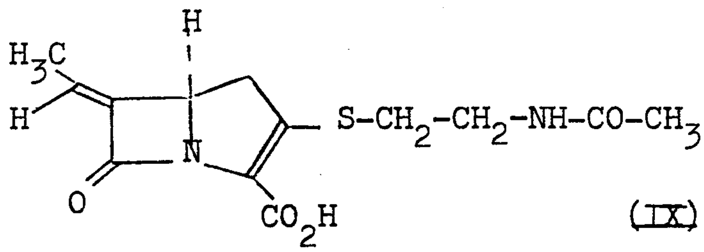

(IX)

or a salt or cleavable ester thereof.

The compounds of the formulae (VI) - (IX) are most suitably in the form of a cleavable ester.

In reference to the compounds of the formulae (I) - (IX) the term ester includes those compounds wherein the 2-position substituent is of the sub-formulae (a) - (d):

$-CO-O-A_1$ (a)

$-CO-O-CA_2A_3A_4$ (b)

$-CO-O-CHA_5-O-CO-A_6$ (c)

$-CO-O-CH-A_7$ (d)

$\quad\quad\quad$ | $\quad$ |

$\quad\quad\quad O - CO$

wherein $A_1$ is an alkyl group of up to 4 carbon atoms, an alkenyl group of up to 4 carbon atoms, an alkynyl group of up to 4 carbon atoms, or an alkyl group of up to 6 carbon atoms substituted by an alkoxyl group of up to 4 carbon atoms, a benzyloxyl group, a phenoxyl group, an acyl group of up to 4 carbon atoms, an alkoxycarbonyl group of up to 4 carbon atoms, a benzyloxycarbonyl group or by up to three halogen atoms; $A_2$ is a hydrogen atom, a methyl group, a phenyl group or a methoxyphenyl group; $A_3$ is a hydrogen atom, a methyl phenyl, methoxyphenyl group or a nitrophenyl group; $A_4$ is a phenyl, halophenyl, nitrophenyl or methoxyphenyl group; $A_5$ is a hydrogen atom or methyl group; $A_6$ is an alkyl group of up to 6 carbon atoms, an alkoxyl group of up to 6 carbon atoms, a phenyl group,

a phenoxy group, a benzyl group or a benzyloxy group; and $A_7$ is a $CH_2CH_2$ group, a CH=CH group, an o-phenylene group or an o-phenylene group substituted by one or two methyl or methoxyl groups.

Favoured values for $A_1$ include the methyl, ethyl, propyl, butyl, allyl, ethynyl, methoxymethyl and benzyloxymethyl groups.

Favoured values for $CA_2A_3A_4$ include the benzyl, p-nitrobenzyl, 2-p-nitrophenylpropyl, benzhydryl and trityl groups.

Favoured values for $A_6$ include the methyl, t-butyl and ethoxy groups.

Favoured values for $A_7$ include the o-phenylene and dimethoxy-o-phenylene groups.

Certain preferred esters may be selected from the methyl ester, the methoxymethyl ester, the benzyl ester, the p-nitrobenzyl ester, the acetoxymethyl ester, the pivaloyloxymethyl ester, the phthalidyl ester and the α-ethoxycarbonyloxyethyl ester.

A particularly preferred in-vivo hydrolysable ester is the phthalidyl ester.

Preferred ester cleavable by chemical means is the p-nitrobenzyl ester.

Many of the preceding esters may be converted to the free acid or its salt by chemical methods such as hydrolysis or hydrogenolysis. For example esters of the sub-formulae (b) - (d) may be subjected to hydrogenation and esters of the sub-formula (a), (c) and (d) may be subjected to hydrolysis. Similarly esters, particularly those of the sub-formulae (c) and (d) are biologically hydrolysed, for example in-vivo.

In addition to the preceding conventional esters, the term ester when used herein also extends to silyl

esters wherein the 2-position substituent is of the sub-formula(e):

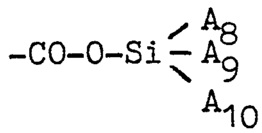

(e)

wherein $A_8$ is an alkyl group of up to 4 carbon atoms or a phenyl group, $A_9$ is an alkyl group of up to 4 carbon atoms or a phenyl group and $A_{10}$ is an alkyl group of up to 4 carbon atoms.

One particularly suitable value for the $SiA_8A_9A_{10}$ moiety is the trimethyl silyl group.

A further particularly suitable value for the $SiA_8A_9A_{10}$ moiety is the tertbutyldimethylsilyl group.

Another particularly suitable value for the $SiA_8A_9A_{10}$ moiety is the tertbutyldiphenylsilyl group.

Silyl esters may be prepated by the reaction of a carboxylate slat with an appropriate silyl chloride.

A further group of useful cleavable esters are those wherein the esterified carboxyl group is of the sub-formula (f):

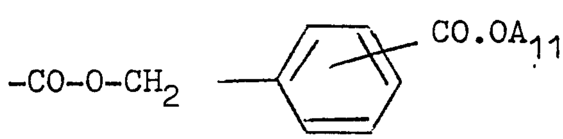

(f)

wherein $A_{11}$ is a lower alkyl group.

Preferably, the alkoxycarbonyl group $CO_2A_{11}$ in the sub-formula (f) is at the 2- or 4-position in the benzene ring, more preferably at the 4-position.

Preferably $A_{11}$ is a methyl group.

Esters of the compounds of the formula (I) wherein the esterified carboxyl group is of the sub-formula (f) may be de-esterified by electrolysis as hereinafter described.

p-Nitrobenzyl esters may also be conveniently de-esterified by electrolysis.

The present invention also provides a pharmaceutical composition which comprises a compound of the formula (I) or a pharmaceutically acceptable salt or in-vivo hydrolylsable ester thereof and a pharmaceutically acceptable carrier. Preferably, A is $CH-CH_2-CH_3$.

The compound of the invention present in such composition will most suitably be in the form of a pharmaceutically acceptable salt such as a pharmaceutically acceptable alkali metal salt, pharmaceutically acceptable alkaline earth metal salt or salt with a pharmaceutically acceptable nitrogenous base. Particularly suitable salts include the sodium salt and the potassium salt.

One group of particularly suitable compounds for inclusion in the compositions of this invention include those of the formulae (II) - (V) and pharmaceutically acceptable salts and in-vivo hydrolysable esters thereof.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit dose composition adapted for administration by injection.

Unit-dose forms according to this invention will normally contain from 50 to 500 mgs of a compound of this invention, for example about 62.5, 100, 125, 150, 200, 250 or 300 mgs. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compostion of this invention may be used to treat infections of the respiratory tract, urinary tract or soft tissues in humans, mastitis in cattle or the like.

The carriers used in the compositions of this invention may include diluent, binders, disintegrants, lubricants, colours, flavouring agents, preservatives or the like in conventional manner. Thus suitable agents include lactose, starch, sucrose, calcium phosphate, sorbitol or the like, polyvinylpyrrolidone, acacia, gelatin, tragacanth or the like, potato starch or polyvinylpolypyrrolidone, magnesium stearate, sodium

lauryl sulphate or the like.

Orally administrable forms of the compositions of this invention are most suitably in the form of unit-dose units such as tablets or capsules.

The present invention also provices synergistic pharmaceutical compositions which comprises a pharmaceutical compositions as hereinbefore described which also contains a penicillin or a cephalosporin.

Suitable penicillins for inclusion in the compositions of this invention include benzyl penicillin, phenoxymethylpenicillin, ampicillin or a pro-drug therefor, amoxycillin or a pro-drug therefor, carbenicillin or a pro-drug therefor, ticarcillin or a pro-drug therefor, suncillin, sulbenicillin, azlocillin, mezlocillin or the like.

Particularly suitable penicillins for inclusion in orally administrable compositions of this invention include ampicillin and its orally administrable prodrugs, amoxycillin and its orally administrable prodrugs of carbenicillin. Thus particularly suitable penicillins include ampicillin anhydrate, ampicillin trihydrate, sodium ampicillin, talampicillin hydrochloride, bacampicillin hydrochloride and the like; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. Afurther preferred penicillin for inclusion in the orally administrable compositions of this invention is ampicillin trihydrate.

Particularly suitable penicillins for inclusion in injectably administrable compositions of this invention include injectable salts such as the sodium salt of ampicillin, amoxycillin, carbenicillin and

and ticarcillin.

A preferred penicillin for inclusion in the injectably administrable composition of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusion in the injectably administrable compositions of this invention include cephaloridine, cefazolin and cephradine, generally as their pharmaceutically acceptable salt such as the sodium salt.

The weight ratio between compound of this invention and pencillin or cephalosporin is generally from 10:1 to 1:10, or more usually from 5:1 to 1:5 and normally from 3:1 to 1:3.

The penicillin or cephalosporin is generally utilised in its conventionally administered amount.

The compounds of the formula (I) wherein A is a $CH-CH_2-CH_3$ group and salts and esters thereof may be prepared by the reduction of the corresponding compound of the formula (I) wherein A is a $C=CH-CH_3$ group.

Accordingly this invention provides a process for the preparation of the compounds of the formula (I) wherein A is a $CH-CH_2-CH_3$ group or a salt or cleavable ester thereof, which process comprises the reduction of a compound of the formula (I) wherein A is a $C=CH-CH_3$ group or a salt or cleavable ester thereof and thereafter, if desired, de-esterifying the esterified carboxyl group.

The reduction is generally performed on an ester of the ethylidene compound of the formula (I).

The reduction may be effected by hydrogenation in the presence of a noble metal catalyst, such as palladium or platinum oxide. The preferred catalyst is platinum oxide.

A low, medium or high pressure of hydrogen may be used, and we have found it convenient to use an approximately atmospheric pressure of hydrogen.

Suitable solvents for the hydrogenation reaction include tetrahydrofuran, ethyl acetate and the like, or mixtures thereof, optionally in admixture with water.

The hydrogenation reaction leads predominantly to the ethyl compound of the formula (I) having the cis configuration about the β-lactam ring [for example, the compounds of the formulae (II) and (IV)] with only a minor proportion of the trans isomer.

The desired compound may be obtained from the reaction mixture by evaporation of the filtered reaction mixture in vacuo. The initial product may be further purified chromatographically, for example, over silica using cyclohexane/ethyl acetate or the like as eluant.

The reduction of an ethylidene compound of the formula (I) may also be effected using a borohydride.

Sodium borohydride has proved to be particularly convenient in this process.

The reaction is normally effected at a slightly depressed temperature, such as from -10 to +10°C, in a solvent such as ethanol/pyridine or aqueous tetrahydrofuran.

The borohydride reduction leads to the formation

of the ethyl compound of the formula (I) having predominantly the *trans* configuration about the β-lactam ring [for example, the compounds of the formulae (III) and (V)] with only a minor proportion of the *cis* isomer.

The required product may be obtained from the reaction mixture in conventional manner, for example, by adding ethyl acetate, washing with water, and evaporating the organic layer. The product may be further purified if desired, for example, by chromatography on silica, eluting with ethyl acetate and/or petroleum ether or the like.

The *cis* ethyl compounds of the formula (I) may be separated from any small amounts of the *trans* isomers (or vice versa) by repeated chromatography.

The required compounds may be distinguished by their nuclear magnetic resonance spectra. The 5-CH/6-CH proton coupling constants are of the order of 2 - 3 Hz in the *trans* compounds, and 4 - 6 Hz in the *cis* compounds.

Alternatively, the individual isomers may be identified by structure determination techniques such as X-ray crystallography.

If the compound of the formula (I) per se or a salt thereof is required it may be prepared from a hydrogenolysable or hydrolysable ester by mild hydrogenation or mild hydrolysis. In general, it is preferred to hydrogenate a p-nitrobenzyl ester in order to produce the parent acid or its salt. Such hydrogenation is conveniently effected using an atmospheric pressure of hydrogen and a palladium on charcoal catalyst. Suitable solvents include aqueous dioxane and other similar solvents. If a salt is required, this may be generated in conventional manner

by the addition of the desired amount of a base, for example an aqueous solution of a bicarbonate, carbonate or the like. The desired product may be obtained by evaporation of the solvents.

The salts of a compound of the formula (I) may be purified by chromatography, for example over a gel-filtration agent such as Biogel P2 (Biogel P2 is a registered trade mark which refers to a polyacrylamide gel supplied by Biorad). Suitable solvents for use include water, aqueous lower alkanols such as aqueous methanol, aqueous ethanol or the like.

This invention further provides a process for the preparation of a compound of the formula (I) or a salt thereof which process comprises the electrolysis of a corresponding ester, wherein the esterified carboxyl group is a group of the sub-formula (f) as hereinbefore defined or a p-nitrobenzyloxycarbonyl group.

Preferably, the electrolysis is carried out on a p-methoxycarbonylbenzyl ester.

The electrolysis will be effected using standard conditions, for example, in an inert aprotic solvent, using mercury, lead, platinum or carbon as the cathode and lead, platinum or carbon as the anode, and using a tetra-alkylammonium halide or tetrafluoroborate as electrolyte.

We have found it particularly convenient to carry out the electrolysis in the cathodic compartment of a divided cell with a mercury pool electrode using DMF as solvent, 0·1M tetrabutylammonium iodide or tetrafluoroborate as electrolyte, in the presence of acetic acid to quench any radical anions formed in the electrolysis, at a potential of about - 1·9v relative to the standard calomel electrode for cleavage of

p-methoxycarbonylbenzyl esters, or about -1·3v for p-nitrobenzyl esters.

The product of the electrolysis reaction may be recovered in conventional manner, for example by converting the compound to the sodium salt using an ion exchange resin (e.g. Amberlite IR 120, Na form) and further purifying the salt by, for example, gel chromatography on a material such as Biogel P-2. Alternatively, the product of the electrolysis may be adsorbed on a resin such as Amberlite IRA 458, then eluted with sodium chloride solution, the eluate being de-salted to provide a solution of the sodium salt of the compound of the formula (I). The salt of the compound of the formula (I) may be obtained in solid form by removing the solvent(for example, by freeze-drying or spray-drying) or by solvent precipitation.

This invention also provides processes for the preparation of a compound of the formula (I) as hereinbefore defined wherein A is $C{=}CH.CH_3$ or a salt or cleavable ester thereof which processes comprise either:

(a) the elimination of the elements of a compound of the formula (XV):

$$R_7\text{-}(O)_n\text{-}SO_3\text{-}H \qquad (XV)$$

wherein $R_7$ is a lower alkyl, aryl or lower aralkyl group and n is 0; or $R_7$ is a lower alkyl, aryl or lower aralkyl group or a cation and n is 1, from a compound of the formula (XVI):

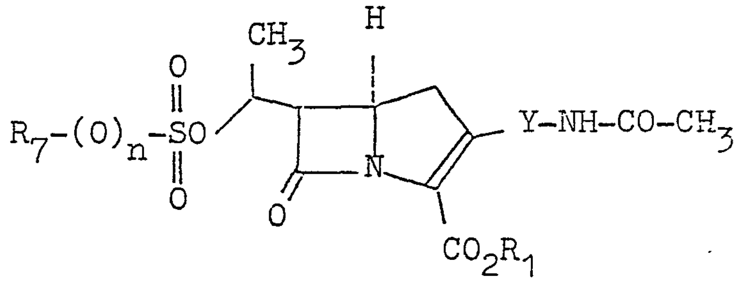

(XVI)

wherein X is as defined in relation to formula (I); $CO_2R_1$ is a cleavable ester group; and $R_7$ and n are as defined in relation to formula (XV).

The elimination of the elements of a compound of the formula (XV) is most conveniently brought about by treatment of a compound of the formula (XVI) with a base in an aprotic medium. The base employed will be a base of low nucleophilicity so that in general primary and secondary amines will not be suitable. Suitable bases include powdered inorganic bases such as alkali metal carbonates, bicarbonates or hydroxides, for example powdered potassium carbonate. or hydrides or 1,5-diazabicyclo[5.4.0]undec-5-ene. Suitable solvents for use in this reaction include dimethylformamide, hexamethylphosphoramide, dichloromethane tetrahydrofuran and the like.

BAD ORIGINAL

The elimination may be effected at a non-extreme temperature such as -20 - 70°C, for example 10° to 25°C. The reaction may be conveniently effected at ambient temperature.

Elimination of a compound of the formula (XV) from an ester of a trans compound of the formula (XVI) leads to the preparation of a Z- or EZ- isomer of the ethylidene compound whereas elimination from an ester of a cis-compound of the formula (XVI) leads to the preparation of the corresponding E- or EZ-isomer.

The desired product may be obtained from the reaction mixture by concentrating in vacuo, diluting with a water-immiscible organic solvent such as ethyl acetate, and removing impurities by washing with water. The organic layer may be re-evaporated in vacuo to yield the compound of the formula (VIII) or (IX) or a mixture thereof. These compounds may then be purified and/or separated chromatographically if desired, for example on silica gel using ethyl acetate, petroleum ether, chloroform, ethanol or the like or mixtures thereof as eluant.

Or

(b) the elimination of the elements of water from a compound of the formula (XVII):

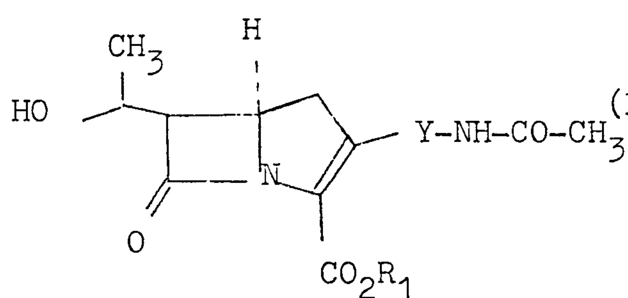

(XVII)

wherein $Y-NH-CO-CH_3$ is as defined in relation to formula (I) and $CO_2R_1$ is a cleavable ester group, in the presence of a compound of the formula (XVIII):

$R_8O_2C-N=N-CO_2R_9$ (XVIII)

wherein $R_8$ and $R_9$ are independently lower alkyl, aryl or lower alkyl-aryl, and a compound of the formula (XIX):

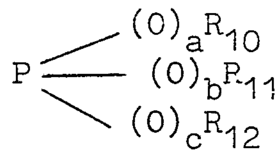

(XIX)

wherein a, b and c are independently 0 or 1, and $R_{10}$, $R_{11}$ and $R_{12}$ are independently lower alkyl, aryl or lower alkyl-aryl.

In the compounds of the formula (XVIII) $R_8$ and $R_9$ are preferably selected from methyl, ethyl, propyl, butyl, phenyl and benzyl, the ethyl and t-butyl groups being preferred.

It is often convenient that $R_8$ and $R_9$ represent the same group.

Preferred compounds of the formula (XIX) include triarylphosphines and trialkylphosphites. Preferred groups $R_{10}$, $R_{11}$ and $R_{12}$ include methyl, ethyl, n-propyl, n-butyl, benzyl, phenyl and methoxyphenyl. Conveniently, $R_{10}$, $R_{11}$ and $R_{12}$ represent the same group. A particularly preferred compound of the formula (XIX) is triphenylphosphine.

Generally, approximately two equivalents of the compounds of the formulae (XVII), (XVIII) and (XIX) are used per mole of compound (XVII).

The elimination reaction is generally carried out at a non-extreme temperature, such as -20 to +100°C. We have found it convenient to begin the reaction at a depressed temperature, such as 0°C, and then to allow the temperature to rise to about room temperature.

The reaction is performed in an inert aprotic organic solvent. Suitable solvents include tetrahydrofuran, dioxane, ethyl acetate, benzene and the like.

Once the reaction is complete, the product may be obtained by washing with water, evaporation of the solvent and chromatography as hereinbefore described.

The compounds of the formula (XVI) wherein n is 0 may be prepared by the reaction of a compound of the formula (XVII) with a compound of the formula (XX):

$$R_7-SO_2-Cl \qquad (XX)$$

or the chemical equivalent thereof wherein $R_7$ is as defined in relation to formula (XVI).

Suitable chemical equivalents of the compounds of the formula (XX) include the corresponding bromide, anhydride and the like.

In general, the reaction is performed in the presence of an acid acceptor to take up the acid generated by the reaction. Suitable acid acceptors include tertiary amines such as triethylamine and the like and powdered solid inorganic bases such as potassium carbonate or the like.

The reaction is normally carried out in a non-hydroxylic medium such as a halohydrocarbon such as dichloromethane or the like at a depressed temperature such as $-20^{o}$ to $10^{o}C$, for example $-10^{o}$ to $0^{o}C$.

When the reaction is over, the desired product may be obtained by washing the organic phase to remove water-soluble impurities and then evaporating the dried organic phase. The product may be further purified chromatographically if desired.

The di-esters within formula (XVI) wherein n is 1

may be prepared by the esterification of a salt of the formula (XXI):

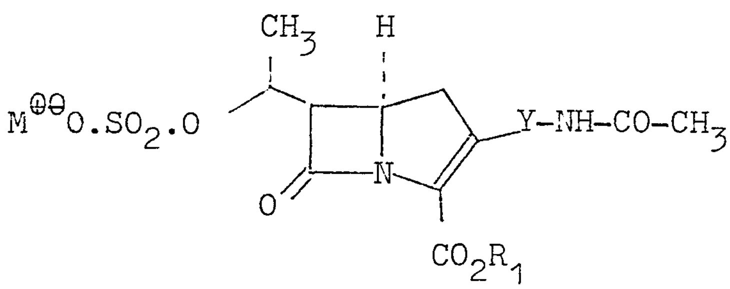

(XXI)

wherein Y-NH-CO-$CH_3$ and $R_1$ are as defined in relation to formulae (VIII) and (IX) and $M^{\oplus}$ is a cation.

Conveniently, $M^{\oplus}$ is an alkali metal ion, such as a sodium ion, or a quaternary ammonium ion.

The esterification is generally carried out using a powerful esterifying agent such as a compound of the formula (XXII):

$$(R_7)_3O^{\oplus} \qquad BF_4^{\ominus} \qquad \text{(XXII)}$$

in a non-hydroxylic solvent, such as dichloromethane.

Compounds (I) where Y is a cis -SCH=CH- group may be prepared by the isomerisation of a corresponding compound wherein Y is a <u>trans</u> -S-CH=CH- group by contacting the compound with a mercuric salt in the presence of an inert solvent.

Suitable mercuric salts include the chloride, bromide, iodide, sulphate and acetate.

Suitable solvents include acetonitrile, acetone, dichloromethane, chloroform and water.

The reaction is generally carried out at a moderate temperature, such as -30 to + 50°C, room temperature being convenient.

Example 1

p-Nitrobenzyl (5R,6S)-3-[(E)-2-acetamidoethenylthio]-6-[(S)-1-methylsulphonyloxyethyl]-7-oxo-1-azabicyclo[3.2.0]hept-2-ene-2-carboxylate

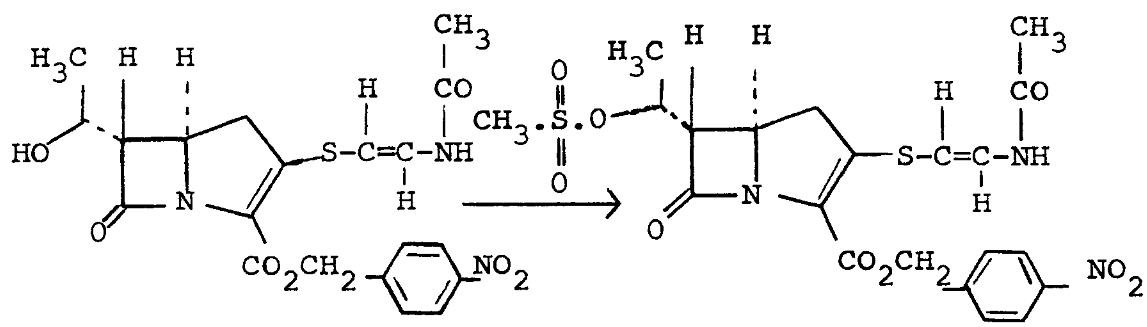

(e1) (e2)

p-Nitrobenzyl (5R,6S)-3-[(E)-2-acetamidoethenylthio]-6-[(S)-1-hydroxyethyl]-7-oxo-1-azabicyclo[3.2.0]hept-2-ene-2-carboxylate (e1) (147 mg) was suspended in dichloromethane (5 ml) containing triethylamine (100 mg), and the mixture was cooled to $0^{o}$. Whilst stirring the mixture at $0^{o}$, methanesulphonyl chloride (75 mg in 2.6 ml dichloromethane) was added dropwise over 5 minutes and the solution then stirred at a temperature of between $0^{o}$ and $-10^{o}$ for a further 25 minutes. The solution was diluted with dichloromethane (10 ml) and washed with cold water (20 ml) phosphate buffer (pH 3.1) (20 ml) and 5% $NaHCO_3$ solution (20 ml). The organic layer was dried ($MgSO_4$) and evaporated down to afford a solid which was triturated with ether and collected by filtration to yield p-nitrobenzyl (5R,6S)-3-[(E)-2-acetamidoethenylthio]-6-[(S)-1-methylsulphonyloxyethyl]-7-oxo-1-azabicyclo[3.2.0]hept-2-ene-2-carboxylate (e2) (130 mg); $\nu_{max}$ (KBr) 1775, 1690, 1620, 1520, 1340, 1270 and 1175 $cm^{-1}$.

Example 2

p-Nitrobenzyl (5R,6Z)-3-[(E)-2-acetamidoethenylthio]-6-ethylidene-7-oxo-1-azabicyclo[3.2.0]hept-2-ene-2-carboxylate

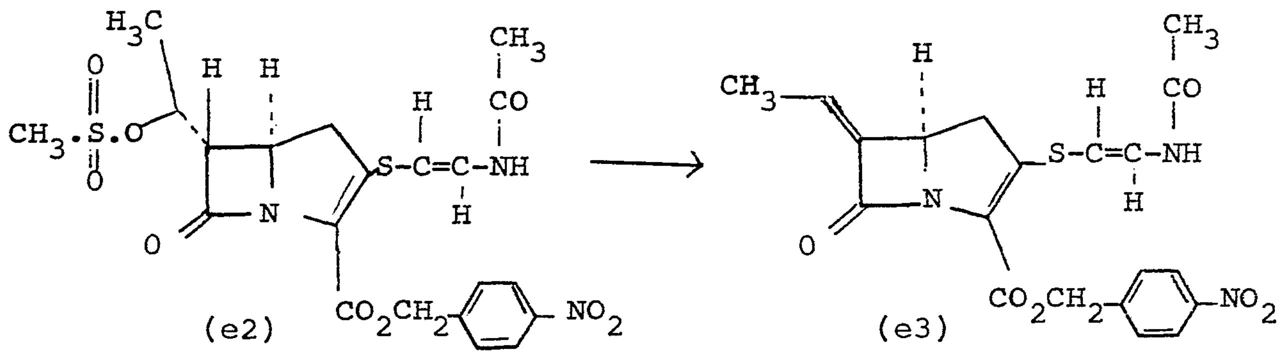

(e2) (e3)

p-Nitrobenzyl (5R,6S)-3-[(E)-2-acetamidoethenylthio]-6-[(S)-1-methylsulphonyloxyethyl]-7-oxo-1-azabicyclo [3.2.0]hept-2-ene-2-carboxylate (e2) (100 mg) and powdered anhydrous potassium carbonate (62 mg) were stirred in dimethylformamide (2 ml) at room temperature for 30 minutes. The mixture was concentrated in vacuo, and the product was then partitioned between ethyl acetate (20 ml) and water (20 ml). The organic layer was washed with water ( 2 x 20 ml) and brine (20 ml), then dried ($MgSO_4$) and evaporated in vacuo. The residue was rapidly chromatographed on silica gel using ethyl acetate as eluant to afford p-nitrobenzyl (5R,6Z)-3-[(E)-2-acetamido-ethenylthio]-6-ethylidene-7-oxo-1-azabicyclo [3.2.0]hept-2-ene-2-carboxylate (e3) (39 mg) as a foam; $\nu$max ($CH_2Cl_2$) 1770, 1705 and 1625 $cm^{-1}$; $\delta$ ($CDCl_3$) 2.05 (6H, br, s+d, C<u>H</u>$_3$CO and C<u>H</u>$_3$CH), 3.04 (2H, m, centre of AA'X, 4-$CH_2$) 4.61 (1H, br t, <u>J</u> 8.5 Hz, 5-C<u>H</u>), 5.18 and 5.46 (each 1H, d, <u>J</u> 14 Hz, C<u>H</u>$_2$Ar), 5.82 (1H, d, <u>J</u> 13.5Hz, = CH.S), 5.90 (1H, m, $CH_3$C<u>H</u>), 7.15 (1H, dd, <u>J</u> 10.5 and 13.5Hz, NHC<u>H</u>=), 7.58 and 8.13 (each 2H, d, <u>J</u> 9Hz, <u>Ar</u>$CH_2$), and about 8.05 (1H, br d, N<u>H</u>).

Example 3

p-Nitrobenzyl (5R,6R)-3-[(E)-2-acetamidoethenylthio]-6-[(S)-1-methylsulphonyloxyethyl]-7-oxo-1-azabicyclo[3.2.0]hept-2-ene-2-carboxylate

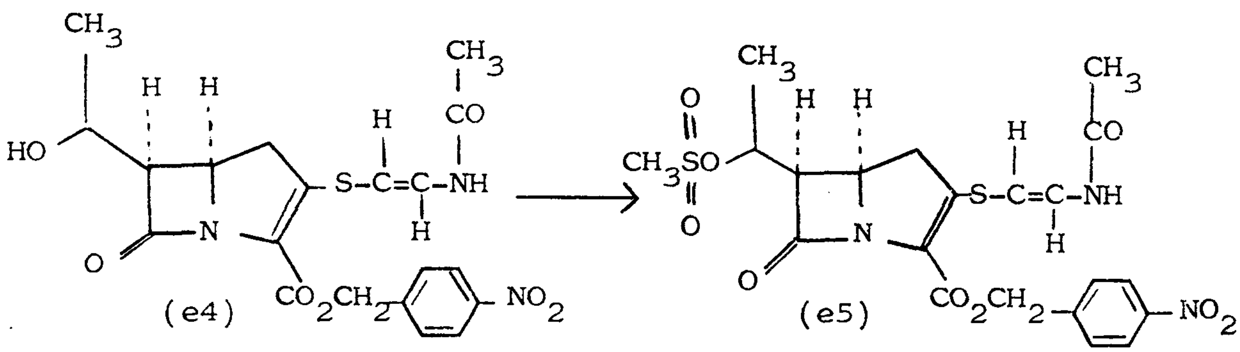

(e4) (e5)

p-Nitrobenzyl (5R,6R)-3-[(E)-2-acetamidoethenylthio]-6-[(S)-1-hydroxyethyl]-7-oxo-1-azabicyclo[3.2.0]hept-2-ene-2-carboxylate (e4) (100mg) was suspended in dichloromethane (6 ml) containing triethylamine (45 mg). The mixture was cooled to 0° and methanesulphonyl chloride (38.4 mg in 1.3 ml dichloromethane) was added dropwise over 5 minutes. After 25 minutes at 0° further quantities of triethylamine (15 mg) and methanesulphonyl chloride (13 mg) were added to the solution. After a further 10 minutes the solution was diluted with dichloromethane (10 ml) and was washed with cold water (20 ml), phosphate buffer (pH 3.1) (20 ml) and 5% $NaHCO_3$ solution (20 ml). The organic layer was dried ($MgSO_4$) and evaporated in vacuo to afford p-nitrobenzyl (5R,6R)-3-[(E)-2-acetamidoethenylthio]-6-[(S)-1-methylsulphonyloxyethyl]-7-oxo-1-azabicyclo[3.2.0]hept-2-ene-2-carboxylate (e5) as a yellow foam (107 mg); $\nu_{max}$ ($CH_2Cl_2$) 1780, 1700, 1625, 1525, 1350, 1330 and 1175 $cm^{-1}$.

Example 4

p-Nitrobenzyl (5R,6E)-3-[(E)-2-acetamidoethenylthio]-6-ethylidene-7-oxo-1-azabicyclo[3.2.0]hept-2-ene-2-carboxylate

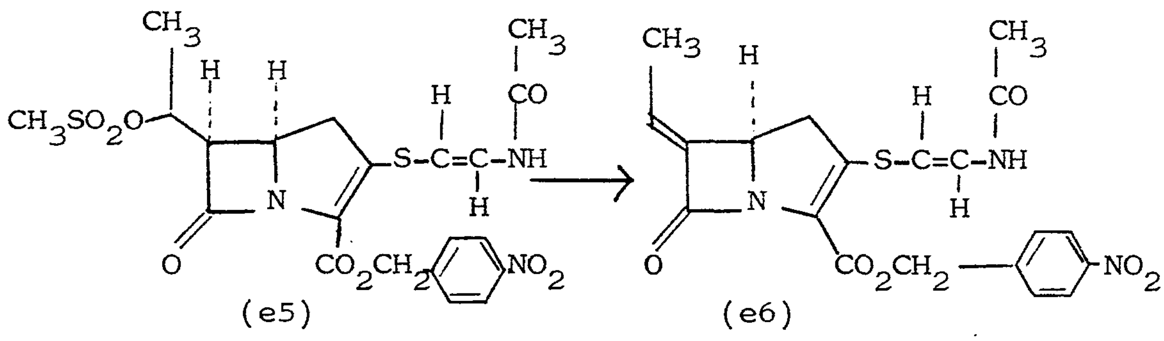

(e5) (e6)

p-Nitrobenzyl (5R,6R)-3-[(E)-2-acetamidoethenylthio]-6-[(S)-1-methylsulphonyloxyethyl]-7-oxo-1-azabicyclo [3.2.0]hept-2-ene-2-carboxylate (e5) (100 mg) was treated with potassium carbonate in dimethylformamide as described in Example 2. The product (18 mg) was p-nitrobenzyl(5R,6E)-3-[(E)-2-acetamidoethenylthio]-6-ethylidene-7-oxo-1-azabicyclo[3.2.0]hept-2-ene-2-carboxylate (e6); ʏmax ($CH_2Cl_2$) 1775, 1700 and 1625 $cm^{-1}$; δ($CDCl_3$) 1.81 (3H, d, J 7Hz, $CH_3CH$), 2.05 (3H, s, $CH_3CO$), 3.05 (2H, m, centre of ABX, 4-$CH_2$), 4.70 (1H, br t, J 9Hz, 5-CH), 5.20 and 5.48 (each 1H, d, J 14 Hz, $CH_2Ar$), 5.85 (1H, d, J 13.5Hz, S.CH=), 6.37 (1H, qd, J 7 and ca. 1Hz, $CH_3CH$), 7.15 (1H, dd, J 13.5 and 10 Hz. NH.CH=), ca. 7.6 (1H, NH), 7.60 and 8.15 (each 2H, d, J 9Hz $ArCH_2$).

Example 5

p-Nitrobenzyl (5R,6S)-3-(2-acetamidoethylthio)-6- [(S)-1-methylsulphonyloxyethyl] -7-oxo-1-azabicyclo [3.2.0] hept-2-ene-2-carboxylate

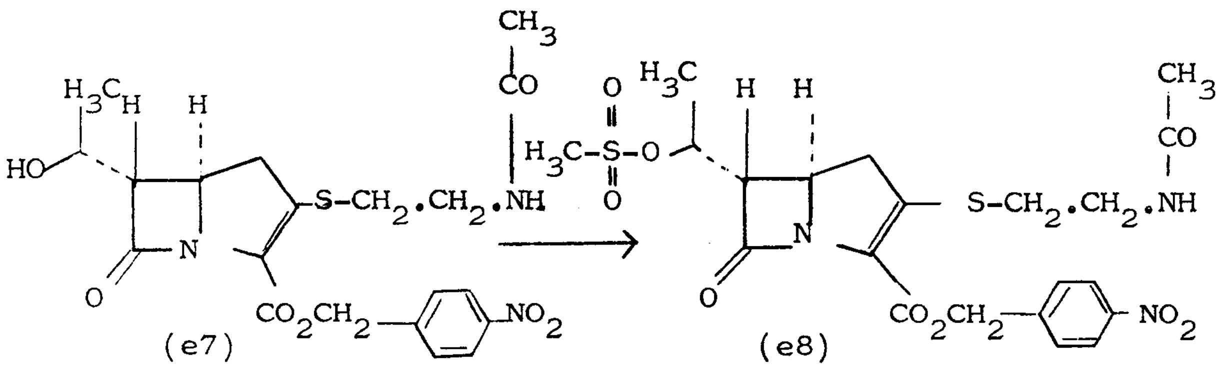

(e7) (e8)

p-Nitrobenzyl (5R,6S)-3-(2-acetamidoethylthio)-6-[(S)-1-hydroxyethyl]-7-oxo-1-azabicyclo[3.2.0]hept-2-ene-2-carboxylate (e7) (100 mg) was treated with methanesulphonyl chloride and triethylamine in dichloromethane as described in Example 1. After work-up, evaporation of the dichloromethane solution afforded p-nitrobenzyl (5R,6S)-3-(2-acetamidoethylthio)-6- [(S)-1-methylsulphonyloxyethyl]-7-oxo-1-azabicyclo [3.2.0]hept-2-ene-2-carboxylate (e8) (112 mg) as a foam; $\nu$ max ($CH_2Cl_2$) 1785, 1700 sh, 1680, 1525, 1350, 1335 and 1175 $cm^{-1}$.

Example 6

p-Nitrobenzyl (5R,6Z)-3-(-2-acetamidoethylthio)-6-ethylidene-7-oxo-1-azabicyclo [3.2.0]hept-2-ene-2-carboxylate.

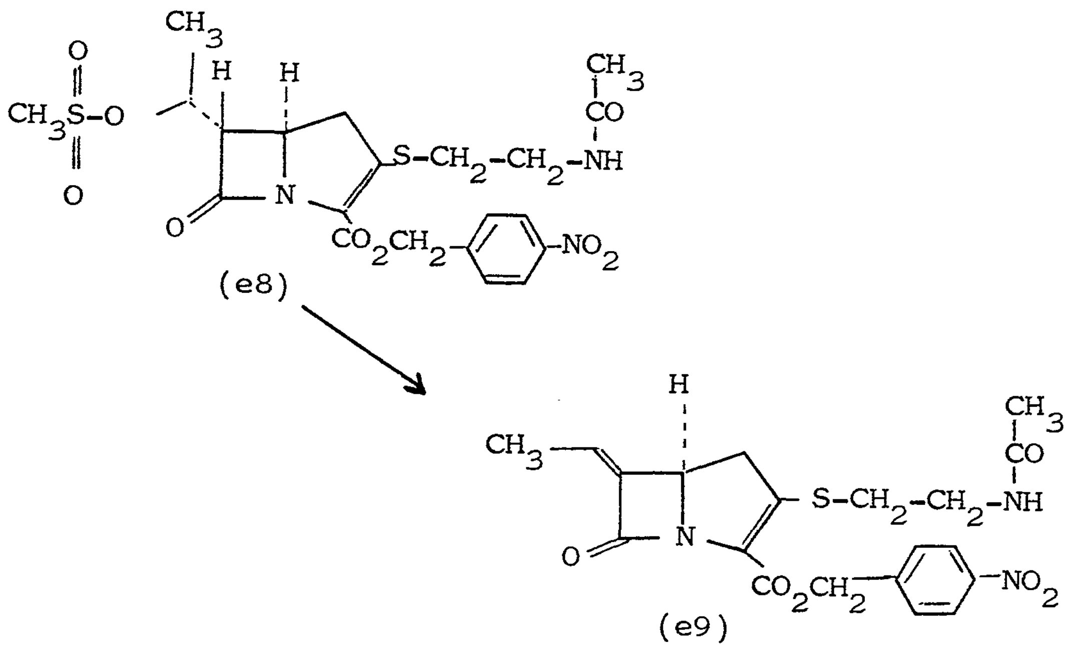

(e8)

(e9)

p-Nitrobenzyl (5R,6S)-3-(-2-acetamidoethylthio)-6-[(S)-1-methylsulphonyloxyethyl]-7-oxo-1-azabicyclo [3.2.0]hept-2-ene-2-carboxylate (e8) (110 mg) was treated with $K_2CO_3$ (100 mg) in dimethylformamide (3 ml) for 1 hour. Work-up and chromatography as described in Example 2 afforded p-nitrobenzyl (5R,6Z)-3-(-2-acetamidoethylthio)-6-ethylidene-7-oxo-1-azabicyclo[3.2.0]hept-2-ene-2-carboxylate (e9) as a gum (33 mg); $\nu$ max ($CH_2Cl_2$) 1770, 1700 and 1680 $cm^{-1}$; $\delta$ ($CDCl_3$) 1.92 (3H, s, $CH_3CO$), 2.04 (3H, d, *J* 7Hz, $C\underline{H}_3CH$), *ca.* 2.9 - 3.5 (6H, m, $NC\underline{H}_2C\underline{H}_2S$ and 4-$C\underline{H}_2$), 4.67 (1H, br t, *J* 8.5 Hz, 5-$C\underline{H}$), 5.17 and 5.46 (each 1H, d, *J* 14Hz, $ArC\underline{H}_2$), 5.92 (1H, q, *J* 7Hz, $CH_3C\underline{H}$) *ca.* 6.05 (1H, br, $N\underline{H}$), 7.58 and 8.13 (each 2H, d, *J* 9Hz, $\underline{Ar}CH_2$).

Example 7

p-Nitrobenzyl (5R,6R)-3-(-2-acetamidoethylthio)-6-[(S)-1-methylsulphonyloxyethyl]-7-oxo-1-azabicyclo[3.2.0]hept-2-ene-2-carboxylate

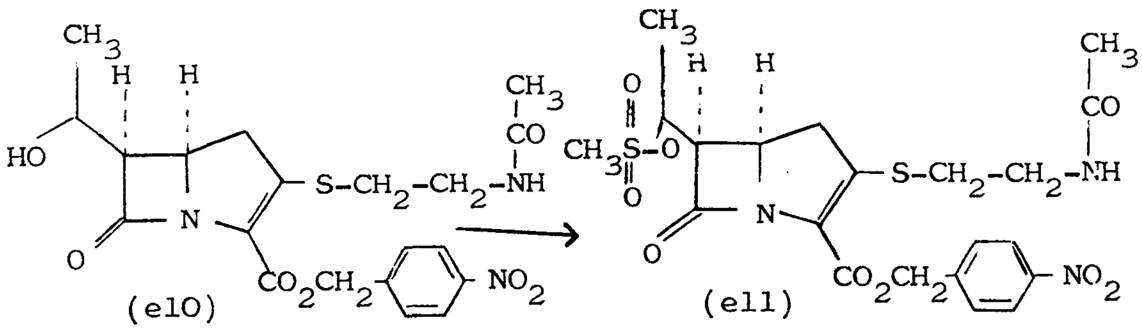

(e10) (e11)

p-Nitrobenzyl (5R,6R)-3-(2-acetamidoethylthio)-6-(1-hydroxyethyl)-7-oxo-1-azabicyclo[3.2.0]hept-2-ene-2-carboxylate (e10) (80 mg) was treated with methanesulphonyl chloride and triethylamine in dichloromethane as described in Example 1. After work-up, evaporation of the dichloromethane solution afforded p-nitrobenzyl (5R,6R)-3-(2-acetamidoethylthio)-6-[(S)-1-methylsulphonyloxyethyl]-7-oxo-1-azabicyclo[3.2.0]hept-2-ene-2-carboxylate (e11) as a pale-yellow foam (67 mg); $\nu$max ($CH_2Cl_2$) 1780, 1700, 1675, 1425, 1245 and 1175 $cm^{-1}$.

Example 8

p-Nitrobenzyl (5R,6E)-3-(2-acetamidoethylthio)-6-ethylidene-7-oxo-1-azabicyclo[3.2.0]hept-2-ene-2-carboxylate

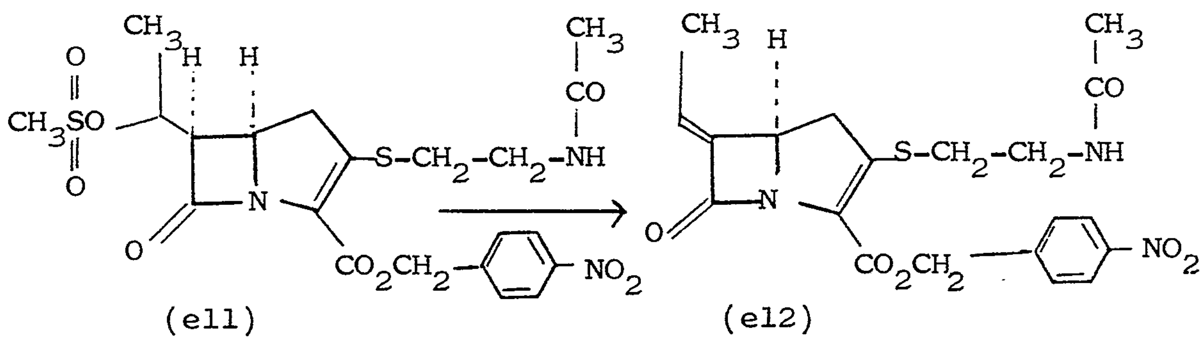

(e11) (e12)

p-Nitrobenzyl (5R,6R)-3-(2-acetamidoethylthio)-6-[(S)-1-methylsulphonyloxyethyl]-7-oxo-1-azabicyclo[3.2.0]hept-2-ene-2-carboxylate (e11) (67 mg) was stirred with $K_2CO_3$ (70 mg) in dimethylformamide (2 ml) at room temperature for 1 hour. Work-up as in Example 2 gave a residue which was triturated with ethyl acetate/ether to afford a solid which was collected by filtration. The solid (10 mg) was identified as p-nitrobenzyl (5R,6E)-3-(2-acetamidoethylthio)-6-ethylidene-7-oxo-1-azabicyclo[3.2.0]hept-2-ene-2-carboxylate (e12); $\delta$ (DMF-d7) ca. 1.85 (3H, d, C$\underline{H}_3$CH), 1.87 (3H, s, C$\underline{H}_3$CO) ca. 2.7-3.5 (6H, m, $NCH_2CH_2S$ and 4-$CH_2$), 4.85 (1H, br t, *J* 9Hz, 5-C$\underline{H}$), 5.29 and 5.53 (each 1H, d, *J* 14Hz, C$\underline{H}_2$Ar), 6.38 (1H, br q, *J* 7 Hz, $CH_3C\underline{H}$ ), 7.76 and 8.20 (each 2H, d, *J* 9Hz, $\underline{Ar}CH_2$), and ca. 8.0 (1H, NH).

Example 9

Methyl (5R,6S)-3-[(E)-2-acetamidoethenylthio]-6-ethyl-7-oxo-1-azabicyclo[3.2.0]hept-2-ene-2-carboxylate and methyl (5R,6R)-3-[(E)-2-acetamidoethenylthio]-6-ethyl-7-oxo-1-azabicyclo[3.2.0]hept-2-ene-2-carboxylate

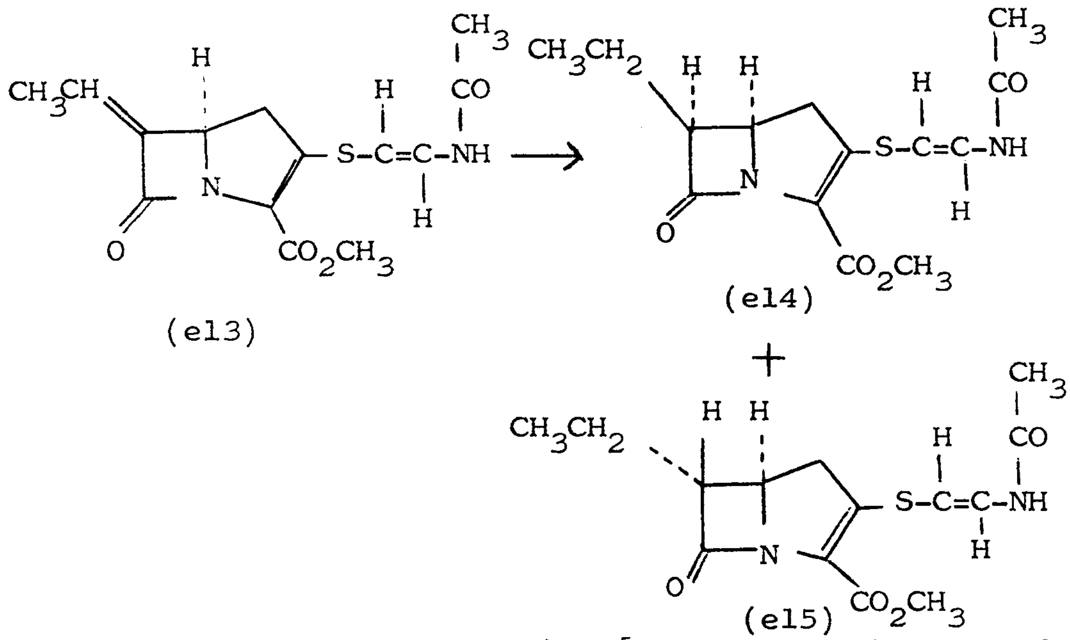

(e13) (e14) + (e15)

Methyl (5R,)-3-[(E)-2-acetamidoethenylthio]-6-thylidene-7-oxo-1-azabicyclo [3.2.0]hept-2-ene-2-carboxylate (e13) (200 mg) was dissolved in tetrahydrofuran (20 ml), and the solution was hydrogenated at room temperature and atmospheric pressure for 15 hours in the presence of platinum oxide catalyst (200 mg). The mixture was filtered over Celite, and the filtrate was evaporated in vacuo. The residue was chromatographed on silica gel using ethyl acetate/cyclohexane mixtures to elute. The major product consisted of a mixture of methyl (5R,6S)-3-[(E)-2-acetamidoethenylthio]-6- ethyl-7-oxo-1-azabicyclo[3.2.0]hept-2-ene-2-carboxylate (e14) and the (5R,6R)-isomer (e15) (ca. 4:1); $\nu$max ($CHCl_3$)

3300 br, 1775, 1700 and 1625 $cm^{-1}$; $\nu$ max (EtOH) 322 and 225 nm; $\delta$ ($CDCl_3$) [(5R,6R)isomer] 0.98 (3H, t, *J* 7.5Hz, $CH_3CH_2$), *ca*. 1.75 (2H, m, $CH_3C\underline{H}_2CH$), 2.04 (3H, s, $CH_3CO$) 2.90 (2H, d, *J* 9Hz, 4-$CH_2$), 3.46 (1H, m, 6-C$\underline{H}$) 3.78 (3H, s, $CO_2CH_3$), 4.20 (1H, dt, *J* 9 and 6Hz, 5-CH), 5.87 (1H, d, *J* 14 Hz, S.C$\underline{H}$=CH), 7.18 (1H, dd, *J* 14 and 10Hz, CH=C$\underline{H}$.NH), 8.25 (1H, broad d, *J* 10 Hz, NH). $\delta$ ($CDCl_3$) [(5R,6S)-isomer] as for (5R,6R)-isomer except 1.01 (3H, t, *J* 7.5Hz, $C\underline{H}_3CH_2$), *CA*. 3.05 (3H, m, 4-$C\underline{H}_2$ and 6-C$\underline{H}$), 3.85 (1H, td, *J* 9 and 3Hz, 5-C$\underline{H}$) and 5.85 (1H, d, *J* 14Hz, SC$\underline{H}$=CH).

Example 10

Benzyl (5R,6S)-3-[(E)-2-acetamidoethenylthio]-6-ethyl-7-oxo-1-azabicyclo[3.2.0]hept-2-ene-2-carboxylate and benzyl (5R,6R)-3-[(E)-2-acetamidoethenylthio]-6-ethyl-7-oxo-1-azabicyclo[3.2.0]hept-2-ene-2-carboxylate

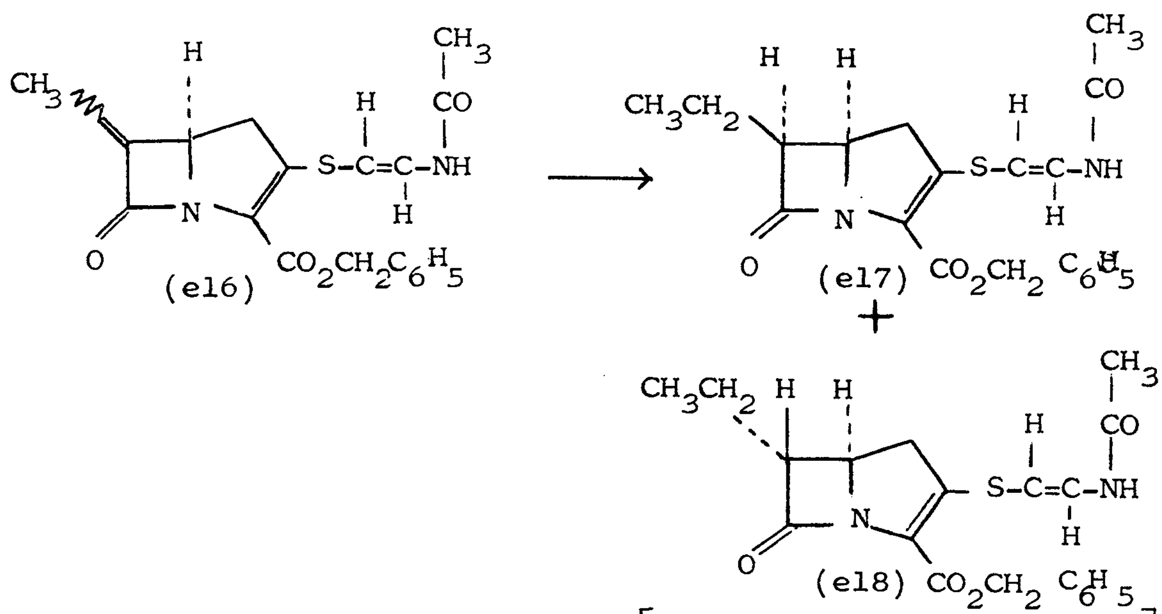

(e16) (e17) + (e18)

Benzyl (5R)-3-[(E)-2-acetamidoethenylthio]-6-ethylidene-7-oxo-1-azabicyclo[3.2.0]hept-2-ene-2-carboxylate (e16) (159 mg) was hydrogenated over platinum oxide catalyst (160 mg) in ethyl acetate (20 ml) for 18 hours. The mixture was filtered, and the filtrate was evaporated in vacuo to afford a gum which was chromatographed on silica gel using petroleum ether - ethyl acetate in a gradient elution. The first eluted product was benzyl (5R,6S)-3-[(E)-2-acetamidoethenylthio]-6-ethyl-7-oxo-1-azabicyclo[3.2.0]hept-2-ene-2-carboxylate (e17) as a colourless oil (41 mg); $\nu_{max}$ ($CHCl_3$) 3300 br, 1775, 1695 and 1625 $cm^{-1}$; $\lambda_{max}$ (EtOH) 322 and 226 nm, ($CDCl_3$) 0.94 (3H, t, $J$ 7Hz, $CH_3CH_2$), 1.3-1.9 (2H, m, $CH_3CH_2CH$), 1.98 (3H, s, $CH_3CO$), 2.85 (2H, d, $J$ 9Hz, 4-$CH_2$), 3.40 (1H, m, 6-CH), 4.14

(1H, dt, J 6 and 9Hz, 5-CH), 5.20 (2H, centre of AA', wings at $\delta$ 5.05 and 5.35, $CH_2Ph$), 5.81 (1H, d, J 14, SCH=), 7.03 (1H, dd, J 14 and 11Hz, NHCH=), 7.28 (5H, m, $PhCH_2$) and 8.03 (1H, broad d, J 11 Hz, NH).

Later fractions from the column were combined to afford a mixture of the (5R,6S)-isomer (e17) and the (5R,6R)-isomer (e18), (*ca.* 1:1) as an oil (48 mg). The p.m.r. spectrum of the mixture showed the following signals due to (e18) as well as those characteristic of (e17); $\delta$ ($CDCl_3$) *inter alia* 0.97 (3H, t, J 7Hz, $CH_2CH_3$), *ca.* 3.0 (2H, m, 6-CH and 4-$CH_2$), 3.81 (1H, dt, J 2.5 and 9Hz, 5-CH), and 5.78 (1H, d, J 14Hz, S.CH=).

Example 11

Methyl (5R,6R)-3-[(E)-2-acetamidoethenylthio]-6-[(S)-1-methoxysulphonyloxyethyl]-7-oxo-1-azabicyclo[3.2.0]hept-2-ene-2-carboxylate

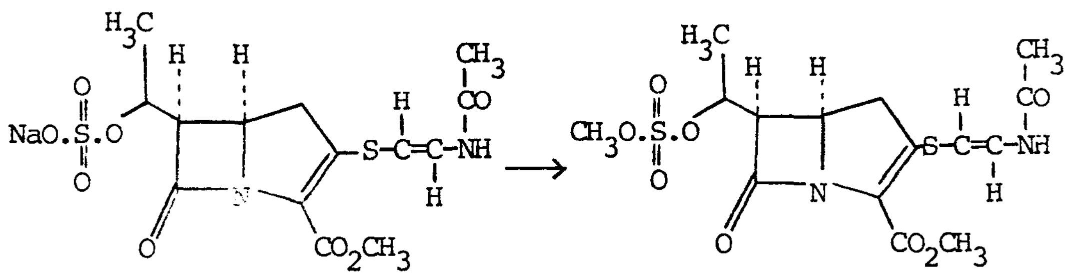

(e19) (e20)

Sodium (5R,6R)-3-[(E)-2-acetamidoethenylthio]-6-[(S)-1-sulphonatooxyethyl]-7-oxo-2-methoxycarbonyl-1-azabicyclo[3.2.0]hept-2-ene (e19) (100 mg) in water (2 ml) was treated with cetylbenzyldimethylammonium chloride (93 mg) in dichloromethane (2 ml). After shaking and separating, the dichloromethane layer was dried ($MgSO_4$) and the dichloromethane removed by evaporation. Toluene was added to the residue and the toluene then evaporated to leave the dry benzyldimethyl-n-hexadecylammonium (5R,6R)-3-[(E)-2-acetamidoethenylthio]-6-[(S)-1-sulphonatooxyethyl]-7-oxo-2-methoxycarbonyl-1-azabicyclo[3.2.0]hept-2-ene (e21). This was then taken up in dry dichloromethane (2 ml) and treated with trimethyloxonium tetrafluoroborate (42 mg) and the mixture stirred. The trimethyloxonium tetrafluoroborate slowly dissolved, and after about 1 hour a solid was deposited from the reaction mixture. The dichloromethane was then removed by evaporat- ethyl acetate (10 ml) was added to the residue and the mixture triturated and filtered. The ethyl acetate was subsequently washed with water (5 ml), followed by brine (5 ml) and then dried ($MgSO_4$) and evaporated. The resid-

was chromatographed on silica gel, using ethyl acetate as eluting solvent to give methyl (5R,6R)-3-[(E)-2-acetamidoethenylthio]-6-[(S)-1-methoxysulphonyloxyethyl]-7-oxo-1-azabicyclo[3.2.0]hept-2-ene-2-carboxylate (e20) (10 mg). [$\nu_{max}$ ($CHCl_3$) 1785, 1705, 1625 $cm^{-1}$.]

In a further experiment the intermediate cetylbenzyldimethylammonium salt was dissolved in dry dichloromethane (2 ml) and treated with trimethyloxonium hexafluorophosphate (50 mg). After stirring for 1½ hours tlc indicated the formation of the dimethyl ester (e20).

Example 12

Methyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate

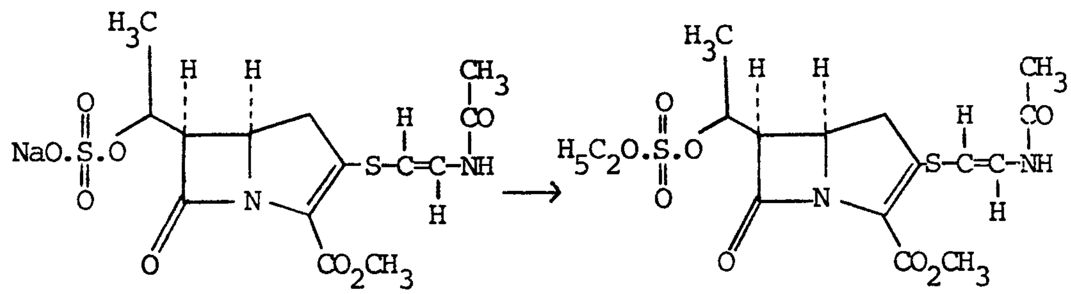

(e19) (e22)

The benzyldimethyl-n-hexadecylammonium salt (e21) was prepared from the sodium salt (e19) as described in Example 11. This was taken up in dry dichloromethane (2 ml), cooled in an ice bath and triethyloxonium tetrafluoroborate under ether (55 mg) was added. The mixture was stirred in the cold for 30 minutes. The dichloromethane was then evaporated. Ethyl acetate (1 ml) was added to the residue and the resultant suspension was placed on a silica gel (230-400 mesh ASTM) column (6 g). The column was eluted with ethyl acetate. Early fractions yielded the required product, contaminated by a material which would not redissolve in ethyl acetate. The impurity was filtered off, and the resultant ethyl acetate solution was evaporated to yield methyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e22) (50 mg). $\nu_{max}$ ($CHCl_3$) 1785, 1700, 1625, 1260 and 1195 $cm^{-1}$; $\nu_{max}$ ($CH_2Cl_2$) 1790, 1705,1625, 1200 $cm^{-1}$; $\delta$ ($CDCl_3$) 1.44 (3H, t, _J_ 7Hz, $C\underline{H}_3CH_2$), 1.64 (3H, d, _J_ 6Hz, 9-$C\underline{H}_3CH$), 2.08 (3H, s, $C\underline{H}_3CO$), 3.01 (1H, dd, _J_ 18 and 9Hz, 4-$C\underline{H}_AH_B$-CH), 3.23 (1H, dd, _J_ 18Hz and 9Hz, 4-$CH_A\underline{H}_B$-CH), 3.84 (3H, s, $CO_2C\underline{H}_3$), 3.73-3.91 (1H, m, 6-C$\underline{H}$), 4.35 (2H, q, _J_ 7Hz,

$OCH_2CH_3$), 4.28-4.47 (1H, m, 5-CH), 5.03 (1H, dq, J 9 and 6Hz, 8-CH), 5.85 (1H, d, J 14Hz, S.CH=CH), 7.30 (1H, dd, J 14 and 10Hz, NH.CH=CH), 8.18 (1H, d, J 10Hz, CONHCH=) p.p.m.

In an alternative experiment the sodium salt (e19) (110 mg) in suspension in dichloromethane (5 ml) was dreated with triethyloxonium tetrafluoroborate in dichloromethane (0.5 ml of a 100 mg/ml solution).

After 45 minutes a further 0.1 ml of the triethyloxonium tetrafluoroborate solution was added, and then after a further 10 minutes 0.1 ml of the solution was added. After another 1 minute the reaction mixture was diluted to 10 ml, washed with brine (5 ml), dried ($MgSO_4$) and evaporated to leave methyl (5R,6R)-3-[(E)-2-acetamidoethenylthio]-6-[(S)-1-ethoxysulphonyloxyehtyl]-7-oxo-1-azabicyclo[3.2.0]hept-2-ene-2-carboxylate (e22) (75 mg).

Example 13

Benzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate

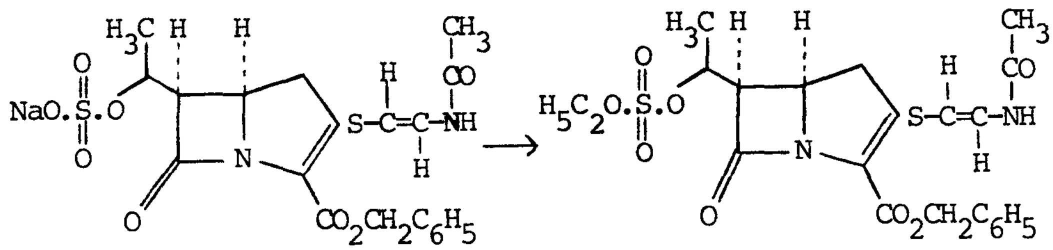

(e23) (e24)

Sodium (5R,6R)-3-[(E)-2-acetamidoethenylthio]-6-[(S)-1-sulphonatooxyethyl]-7-oxo-2-benzyloxycarbonyl-1-azabicyclo[3.2.0]hept-2-ene (e23) (580 mg; containing some inorganic impurities) in water (10 ml) was treated with cetylbenzyldimethylammonium chloride (440 mg) in dichloromethane. After shaking and separating the dichloromethane layer was dried ($MgSO_4$) and evaporated to gvie cetylbenzyldimethylammonium (5R,6R)-3-[(E)-2-acetamidoethenylthio]-6-[(S)-1-sulphonatooxyethyl]-7-oxo-2-benzyloxycarbonyl-1-azabicyclo[3.2.0]hept-2-ene (e25). This was dissolved in dry dichloromethane (10 ml), cooled to $0^{o}$ and triethyloxonium tetrafluoroborate under ether (250 mg) and dichloromethane (2 ml) added. The mixture was stirred at $0^{o}$ for 30 minutes and then at room temperature for 1½ hours. The solvent was removed by evaporation and the residue chromatographed on silica gel (230-400 mesh ASTM) (6 g) using ethyl acetate as eluant. This failed to effect complete purification so the product was rechromatographed on silica gel (230-400 mesh ASTM)

(30 g) eluting with dichloromethane (5 ml), followed by ethyl acetate/petroleum ether (b.p. $60^{o}$-$80^{o}$) mixtures; 1:1 (100 ml), 3:1 (100 ml) and then with neat ethyl acetate. Combination and evaporation of relevant fractions yielded benzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e24) (153 mg). $\nu_{max}$ ($CH_2Cl_2$) 1785, 1705, 1620 and 1200 $cm^{-1}$. $\delta$ ($CDCl_3$), 1.38 (3H, t, $\underline{J}$ 7Hz, $C\underline{H}_3CH_2$), 1.63 (3H, d, $\underline{J}$ 6Hz, 9-$C\underline{H}_3CH$), 2.03 (3H, s, $COC\underline{H}_3$), 2.95 (1H, dd, $\underline{J}$ 18 and 9Hz, 4-$C\underline{H}_AH_BCH$), 3.27 (1H, dd, $\underline{J}$ 18 and 9Hz, 4$CH_A\underline{H}_BCH$), 3.76 (1H, dd, $\underline{J}$ 10 and 6Hz, 6-C$\underline{H}$), ca. 4.1-4.4 (1H, m, S-C$\underline{H}$), 4.28 (2H, q, $\underline{J}$ 7Hz, $OC\underline{H}_2CH_3$), 4.8-5.15 (1H, m, 8-C$\underline{H}$), 5.23 (2H, s, $OC\underline{H}_2Ph$), 5.78 (1H, d, $\underline{J}$ 14Hz, SC$\underline{H}$=CH), 7.08-7.50 (6H, m, NHC$\underline{H}$=CH, 5 x Ar-$\underline{H}$), 8.22 (1H, d, $\underline{J}$ 11Hz, CON$\underline{H}$).

In a further experiment the sodium salt (e21) (100 mg) in dry dichloromethane (5 ml) was treated with a solution of triethyloxonium tetrafluoroborate in dichloromethane (0.40 ml of a 100 mg/ml solution). After 20 minutes a further 0.1 ml aliquot of the triethyloxonium tetrafluoroborate solution was added and the mixture stirred for a further 15 minutes. The mixture was then washed with brine, dried ($MgSO_4$) and evaporated to give the mono benzyl monoethyl diester (e25) (90 mg).

Example 14

p-Nitrobenzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate

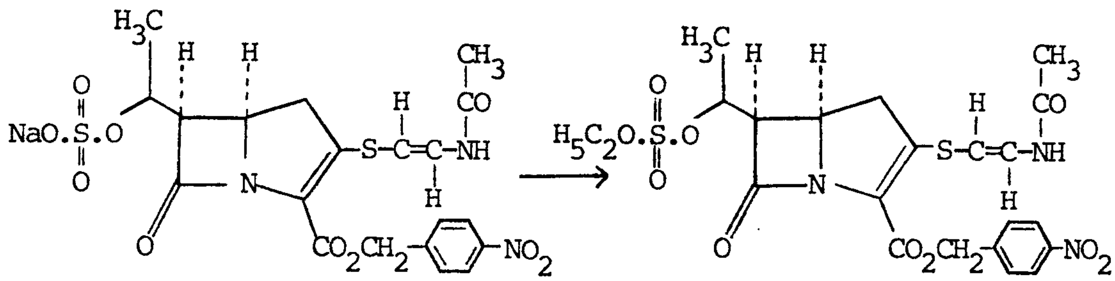

(e26) (e27)

Sodium (5R,6R)-3-[(E)-2-acetamidoethenylthio]-6-[(S)-1-sulphonatoxyethyl]-7-oxo-2-p-nitrobenzyloxycarbonyl-1-azabicyclo[3.2.0]hept-2-ene (e26) (210 mg) and cetylbenzyldimethylammonium chloride (160 mg) were shaken together in dichloromethane (10 ml) and water (10 ml), the dichloromethane layer was separated, dried ($MgSO_4$) and evaporated to give cetylbenzyldimethylammonium (5R,6R)-3-[(E)-2-acetamidoethenyl-thio]-6-[(S)-1-sulphonatooxyethyl]-7-oxo-2-p-nitrobenzyloxycarbonyl-1-azabicyclo[3.2.0]hept-2-ene (e28). This was taken up in dry dichloromethane (2 ml) and treated with triethyloxonium tetrafluoroborate (0.7 ml of a 100 mg/ml solution in $CH_2Cl_2$). After 30 minutes a further 0.1 ml of the tetrafluoroborate solution was added and then after 5 minutes the resultant mixture was chromatographed on silica gel (230-400 mesh ASTM) (10 g) eluting with ethyl acetate/cyclohexane. This gave the required diester. Some of the fractions were evaporated and residual oil was crystallised from ethyl acetate/cyclohexane to give p-nitrobenzyl (5R,6R)-3-[(E)-2-acetamidoethenylthio]-6-[(S)-1-ethoxysulphonyloxyethyl]-7-oxo-1-azabicyclo

[3.2.0]hept-2-ene-2-carboxylate (e27) (61 mg), m.p. 125 - 128° (dec). $[\alpha]_{20}^{D}$-183.9° (C 1.0%, $CHCl_3$). (Found: C, 47.56; H, 4.54; N, 7.59. $C_{22}H_{25}N_3O_{10}S_2$ requires C, 47.56; H, 4.54; N, 7.56%), $\nu_{max}$ ($CH_2Cl_2$) 1780, 1700 and 1620 $cm^{-1}$; δ ($CDCl_3$) 1.38 (3H, t, *J* 7Hz, $C\underline{H}_3CH_2$), 1.62 (3H, d, *J* 6Hz, $C\underline{H}_3CH$), 2.04 (3H, s, $C\underline{H}_3CO$), 2.99 (1H, dd, *J* 19 and 10Hz, $\underline{H}_A$ of ABX), 3.25 (1H, dd, *J* 19 and 9Hz, $\underline{H}_B$ of ABX), 3.78 (1H, d, *J* 10 and 5.5Hz, CH.C<u>H</u>.CH), 4.28 (2H, q, *J* 7Hz, $CH_3C\underline{H}_2$) <u>ca</u>. 5.2(1H, m, $C\underline{H}CH_2$), 4.98 (1H, m, $CH_3C\underline{H}CH$), 5.16 and 5.42 (each 1H, d, *J* 14Hz, $C\underline{H}_2Ar$), 5.78 (1H, d, *J* 13.5Hz, CH=C<u>H</u>S), 7.20 (1H, dd, *J* 13.5 and 10Hz, NHC<u>H</u>=C), 7.53 and 8.11 (each 2H, d, *J* 9Hz, aromatic protons), and 8.26 (1H, d, *J* 101 NH), ppm.

Example 15

p-Bromobenzyl (5R, 6R)-3-[(E)-2-acetamidoethenylthio]-6-[(S)-1-ethoxysulphonyloxyethyl]-7-oxo-1-azabicyclo [3.2.0]hept-2-ene-2-carboxylate

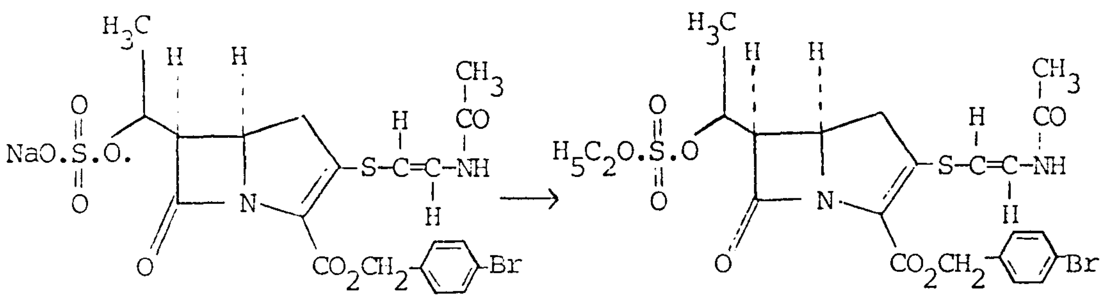

(e29) (e30)

Sodium (5R,6R)-3-[(E)-2-acetamidoethenylthio]-6-[(S)-1-sulphonatooxyethyl]-7-oxo-2-p-bromobenzyloxycarbonyl-1-azabicyclo[3.2.0]hept-2-ene (e29) (200 mg), suspended in dichloromethane (10 ml), was stirred and a solution of triethyloxonium tetrafluoroborate in dichloromethane (0.65 ml of a 100 mg/ml solution) was added. The mixture was stirred for 35 minutes when a further 0.1 ml aliquot of the triethyloxonium tetrafluoroborate solution was added, and after stirring for a further 10 minutes the reaction mixture was placed on a silica gel (230-400 mesh ASTM) column (10 g) and eluted with ethyl acetate/cyclohexane (8:2). Evaporation yielded p-bromobenzyl (5R,6R)-3-[(E)-2-acetamidoethenylthio]-6-[(S)-1-ethoxysulphonyloxyethyl]-7-oxo-1-azabicyclo [3.2.0]hept-2-ene-2-carboxylate (e30) (92 mg) which was crystallised from ethylacetate/cyclohexane to give crystals, m.p. 121-124$^{o}$ (dec); Found: C,44.79; H,4.40, N,4.86. $C_{22}H_{25}BrN_2O_8S_2$ requires C, 44.82; H, 4.27;

N, 4.76%), $[\alpha]_D^{20}$ - 124.9 ($CHCl_3$ c. 1.0) $\nu_{max}$ (EtOH) 325 ($\varepsilon_{max}$ 17340), 227 (24,300)nm, $\nu_{max}$ ($CH_2Cl_2$) 1785, 1705, 1625, 1195 $cm^{-1}$; $\delta$ ($CDCl_3$) 1.41 (3H, t, *J* 7Hz, $C\underline{H}_3CH_2$), 1.63 (3H, d, *J* 6Hz, $C\underline{H}_3CH$), 2.04 (3H, s, $COC\underline{H}_3$), 2.98 (1H, dd, *J* 18.5 and 10Hz, $\underline{H}_A$ of ABX system), 3.24 (1H, dd, *J* 18.5 and 9Hz, $\underline{H}_B$ of ABX system), 3.76 (1H, dd, *J* 10 and 5.5Hz, CH.C$\underline{H}$CH), 4.1-4.4 (1H, m, CH.C$\underline{H}$.$CH_2$) 4.28 (2H, q, *J* 7Hz, $OC\underline{H}_2CH_3$), 4.7-5.2 (1H, m, $CH_3$.C$\underline{H}$.CH), 5.07 and 5.24 (2H, $AB_q$, *J* 13Hz), 5.76 (1H, d, *J* 13.5Hz, SC$\underline{H}$=CH), 7.08-7.5 (1H, m, NHC$\underline{H}$=CH), 7.22 (2H, d, *J* 9Hz, 2 x Ar-$\underline{H}$), 7.41 (2H, d, *J* 9Hz, 2 x Ar-$\underline{H}$), 7.98 (1H, d, *J* 10Hz, CON$\underline{H}$CH=) p.p.m.

Example 16

p-Nitrobenzyl (5R,6R)-3-(2-acetamidoethylthio)-6-[(S)-1-ethoxysulphonyloxyethyl]-7-oxo-1-azabicyclo[3.2.0]hept-2-ene-2-carboxylate

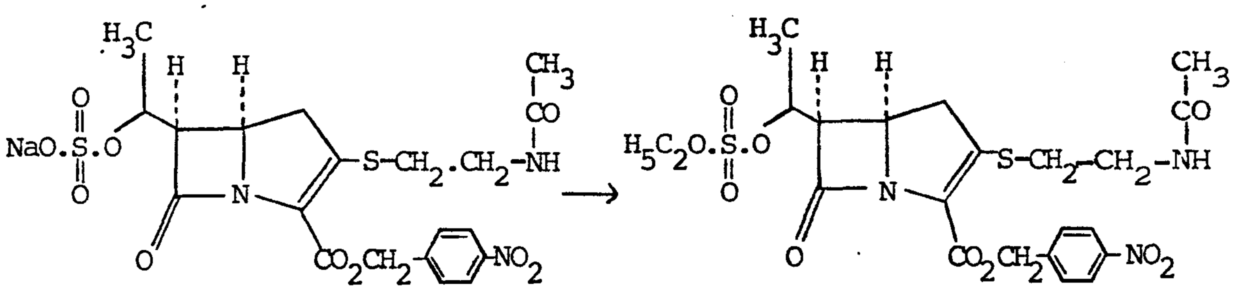

(e31) (e32)

p-Nitrobenzyl (5R,6R)-3-(2-acetamidoethylthio)-6-[(S)-1-ethoxysulphonyloxyethyl]-7-oxo-1-azabicyclo[3.2.0]hept-2-ene-2-carboxylate (e32) was prepared (31% yield) by a process analogous to that described in Example 14. The diester (e32) was crystallised from hot ethanol to afford small needles m.p. 126-128$^{o}$ (Found: C, 47.5; H, 5.0; N, 7.5%. $C_{22}H_{27}N_{3}O_{10}S_{2}$ requires C, 47.4; H, 4.9; N, 7.5%); $\lambda_{max}$ (EtOH) 318 ($\varepsilon_{max}$ 13,600) and 270 (11,900) nm; $\nu_{max}$ (KBr) 1765, 1690 and 1645 cm$^{-1}$.

Example 17

Benzyl (5R,6R)-3-[(E)-2-acetamidoethenylthio]-6-[(R)-1-ethylthioethyl]-7-oxo-1-azabicyclo[3.2.0]hept-2-ene-carboxylate and benzyl (5R,6R)-3-[(E)-2-acetamidoethenylthio] 6-[(S)-1-ethylthioethyl]-7-oxo-1-azabicyclo[3.2.0]hept-2-ene-carboxylate

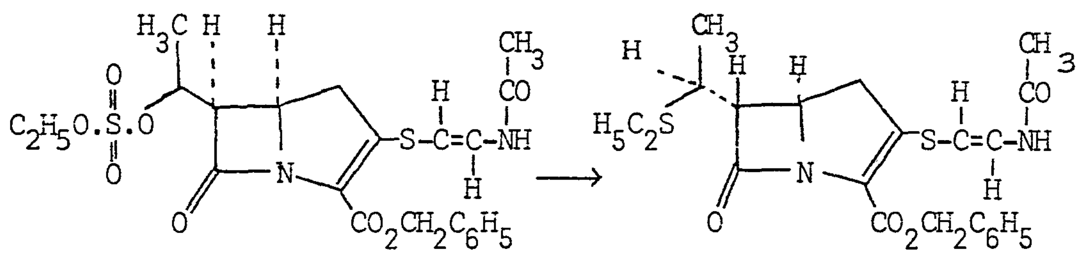

(e24) (e33)

Benzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e24) (235 mg) in dimethylformamide (4 ml) was treated with finely powdered potassium carbonate (32 mg) and cooled to -60°. Ethane thiol in dimethylformamide (1.43 ml of a 20 mg/ml solution) was added to the mixture and stirring was continued as the reaction mixture was allowed to reach room temperature. After 4 hours at room temperature the solution was added to ethyl acetate (50 ml) and washed with a mixture of water (15 ml) and brine (30 ml) (3 x). The organic layer was dried ($MgSO_4$) and evaporated to leave a gum which was chromatographed on silica gel (230-400 mesh ASTM) (20 g), eluting with ethyl acetate/cyclohexane mixtures thus: 3:7 (100 ml); 4:6 (50 ml), 1:1 (200 ml), 3:1 (200 ml) and finally ethyl acetate. Eventually one isomer of the title compound (e33) (17 mg) was eluted followed by a mixture of 2 isomers of the title compound (e33) (25 mg) and then the second isomer of the title compound (e33) (21 mg).

Example 18

Methyl (5R,6E)-3-[(E)-2-acetamidoethenylthio]-6-ethylidene-7-oxo-1-azabicyclo[3.2.0]hept-2-ene-2-carboxylate

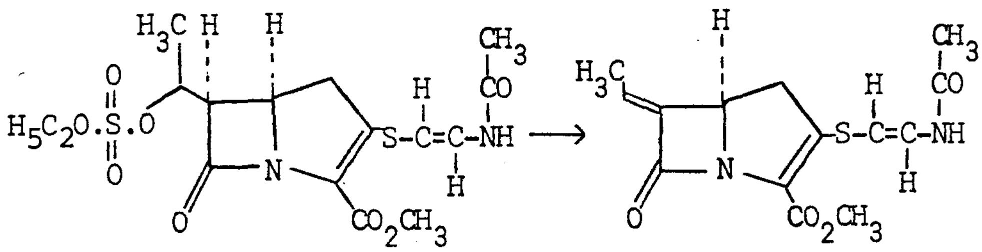

(e22) (e34)

Methyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e22) (42 mg) was taken up in ethanol (2 ml) and treated with anhydrous potassium acetate (50 mg). After 90 minutes the solvent was removed by evaporation and the product dissolved in ethyl acetate (10 ml) and washed with water (10 ml), and with brine (10 ml) and then dried ($MgSO_4$) and evaporated. The i.r. and u.v. spectra of crude material showed that the ethylidene compound had been formed. This was purified by chromatography on silica gel to give methyl (5R,6E)-3-[(E)-2-acetamidoethenylthio]-6-ethylidene-7-oxo-1-azabicyclo[3.2.0]hept-2-ene-2-carboxylate (e34) (14 mg). $\nu_{max}$ ($CH_2Cl_2$) 1780, 1705,1625 $cm^{-1}$. $\delta$ [$CDCl_3$ + 1 drop $(CD_3)_2CO$] 1.79 (3H, d, *J* 7Hz, $CH_3CH=$), 2.02 (3H, s, $CH_3CO$), 2.90 (1H, dd, *J* 18Hz and 8.5Hz, $H_A$ of ABX), 3.16 (1H, dd, *J* 18 and 9.5Hz, $H_B$ of ABX), 3.78 (3H, s, $OCH_3$), 4.69 (1H, broad t, $CH.CH_2$), 5.84 (1H, d, *J* 14Hz SCH:CH), 6.33 (1H, broad q, *J* 7Hz, $CH_3CH=$), 7.18 (1H, dd, *J* 14 and 10Hz, NH.CH=CH), 8.86 (1H, broad d, *J* 10Hz, CONH.CH) p.p.m.

Example 19

Methyl (5R,6E)-3-[(E)-2-acetamidoethenylthio]-6-ethylidene-7-oxo-1-azabicyclo[3.2.0]hept-2-ene-2-carboxylate and methyl (5R,6Z)-3-[(E)-2-acetamidoethenylthio]-6-ethylidene-7-oxo-1-azabicyclo[3.2.0]hept-2-ene-2-carboxylate

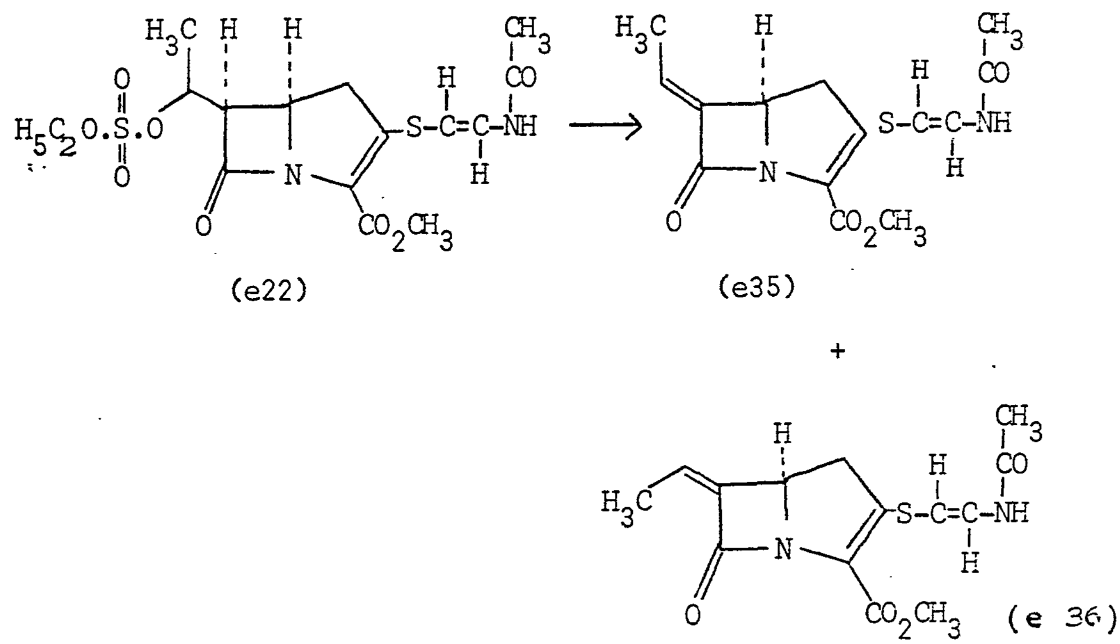

(e22) (e35)

+

(e 36)

Methyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e22) (86 mg) was taken up in dichloromethane (3 ml) and treated with 1,5-diazabicyclo[5.4.0] undec-5-ene (DBU) (33 mg) in dichloromethane (3.3 ml) added dropwise. After 15 minutes more DBU (3 mg) in dichloromethane (0.3 ml) was added and stirring was continued for 10 minutes. The solution was then washed with water (5 ml) and then chromatographed on silica gel to give methyl (5R,6E)-3-[(E)-2-acetamidoethenylthio]-6-ethylidene-7-oxo-1-azabicyclo[3.2.0]hept-2-ene-2-carboxylate and methyl (5R,6Z)-3-[(E)-2-acetamidoethenylthio]-6-ethylidene-7-oxo-1-azabicyclo[3.2.0]hept-2-ene-2-carboxylate as a mixture of Z and E isomers (e35 and e36) (21 mg), $\nu_{max}$ ($CH_2Cl_2$) 1775 (broad), 1705, 1620 $cm^{-1}$.

Example 20

Benzyl (5R,6E)-3-[(E)-2-acetamidoethenylthio]-6-ethylidene-7-oxo-1-azabicyclo[3.2.0]hept-2-ene-2-carboxylate and benzyl (5R,6Z)-3-[(E)-2-acetamidoethenylthio]-6-ethylidene-7-oxo-1-azabicyclo[3.2.0]hept-2-ene-2-carboxylate

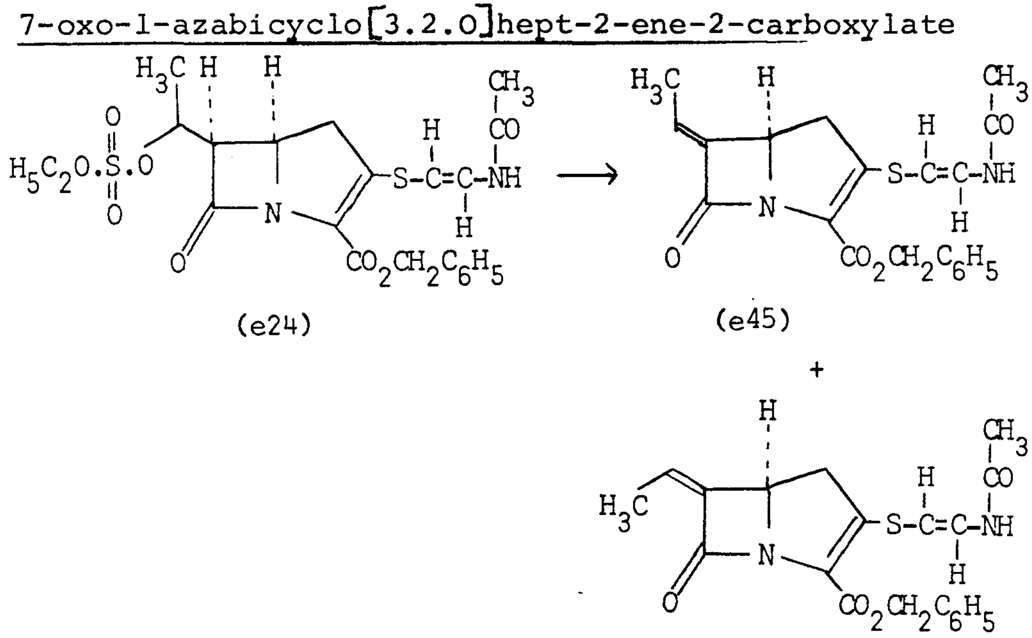

(e24) (e45)

+

(e37)

Benzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e24) prepared from sodium (5R,6R)-3-[(E)-2-acetamidoethenylthio]-6-[(S)-1-sulphonatooxyethyl]-7-oxo-2-benzyloxycarbonyl-1-azabicyclo[3.2.0]hept-2-ene (e23) (100 mg) as described in Example 13 was treated with 1,5-diazabicyclo[5.4.0]-undec-5-ene (35 mg) in an analogous manner to that described in Example 19 to give benzyl (5R,6E)-3-[(E)-2-acetamidoethenylthio]-6-ethylidene-7-oxo-1-azabicyclo[3.2.0]hept-2-ene-2-carboxylate and benzyl (5R,6Z)-3-[(E)-2-acetamidoethenylthio]-6-ethylidene-7-oxo-1-azabicyclo[3.2.0]hept-2-ene-2-carboxylate (e45 and e37) (30 mg). The n.m.r. spectrum [$CDCl_3$ + $(CD_3)_2SO$] indicated that the ethylidene group was largely in the E- form (e45) but that some Z- form (e37) was also present.

Example 21

Benzyl (5R,6E)-3-[(E)-2-acetamidoethenylthio]-6-ethylidene-7-oxo-1-azabicyclo[3.2.0]hept-2-ene-2-carboxylate and benzyl (5R,6Z)-3- (E)-2-acetamidoethenylthio -6-ethylidene-7-oxo-1-azabicyclo[3.2.0]hept-2-ene-2-carboxylate

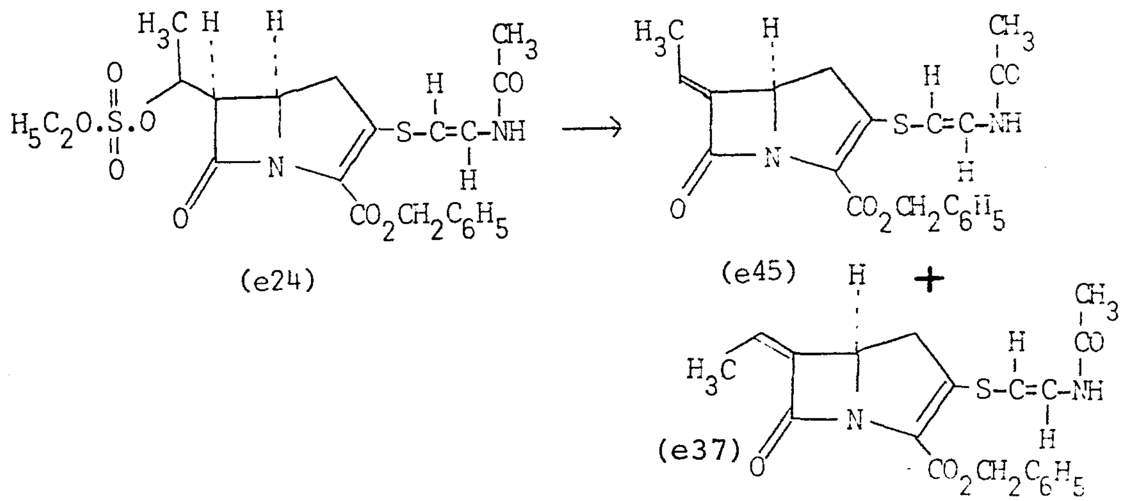

(e24) (e45) + (e37)

Benzyl (5R,6R)-3-[(E)-2-acetamidoethenylthio]-6-[(S)-1-ethoxysulphonyloxyethyl]-7-oxo-1-azabicyclo [3.2.0] hept-2-ene-2-carboxylate (18 mg) in dichloromethane (0.75 ml) was treated with a total of 0.16 ml of a 100 mg/ml solution of triethylamine in dichloromethane, added in equal aliquots over 90 minutes. The mixture was stirred for a further 90 minutes, washed with water (5 ml) and dried ($MgSO_4$). The solvent was then removed by evaporation to give benzyl (5R,6E)-3-[(E)-2-acetamidoethenylthio]-6-ethylidene-7-oxo-1-azabicyclo [3.2.0]hept-2-ene-2-carboxylate and benzyl (5R,6Z)-3-[(E)-2-acetamidoethenylthio]-6-ethylidene-7-oxo-1-azabicyclo [3.2.0]hept-2-ene-2-carboxylate (e45 and e37) (12 mg) as a 1:2 mixture.

Example 22

p-Nitrobenzyl (5R,6E)-3-[(E)-2-acetamidoethenylthio]-6-ethylidene-7-oxo-1-azabicyclo[3.2.0]hept-2-ene-2-carboxylate

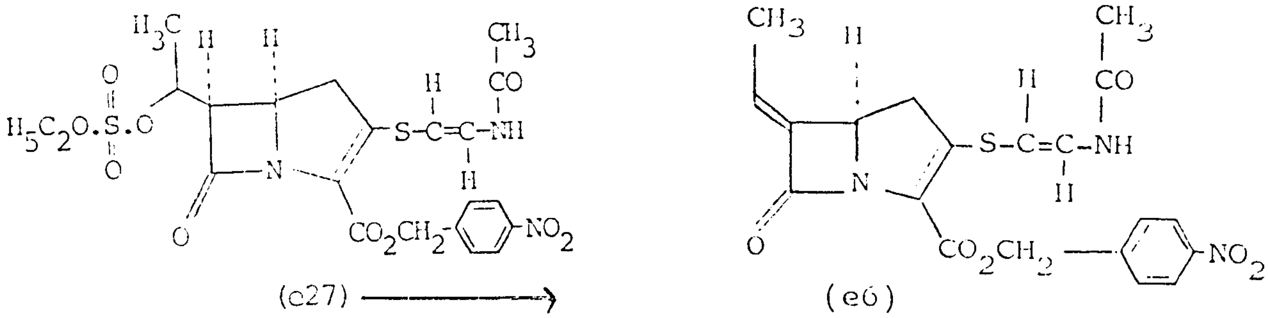

(e27) (e6)

p-Nitrobenzyl (5R,6R)-3-[(E)-2-acetamidoethenylthio]-6-[(S)-1-ethoxysulphonyloxyethyl]-7-oxo-1-azabicyclo[3.2.0]hept-2-ene-2-carboxylate (e32) (109 mg) in dimethylformamide (2 ml) was treated with potassium carbonate (100 mg) for 90 minutes.

The solvent was removed by evaporation, the residue dissolved in ethyl acetate (10 ml) and washed with water (10 ml). The organic layer was dried ($MgSO_4$) and the solvent removed by evaporation. The residue was chromatographed on silica gel (230-400 mesh ASTM) to give p-nitrobenzyl (5R,6E)-3-[(E)-2-acetamidoethenylthio]-6-ethylidene-7-oxo-1-azabicyclo[3.2.0]hept-2-ene-2-carboxylate (e6) (60 mg). $\nu$ max. ($CHCl_3$) 1770 (br), 1700, 1625 $cm^{-1}$. $\delta$ (DMF-$d_7$) 1.82 (3H, d, <u>J</u> 7Hz, $C\underline{H}_3CH$), 1.98 (3H, s, $C\underline{H}_3CO$), 3.0-3.5 (2H, ABX, $C\underline{H}_2CH$), 4.83 (1H, broad t, <u>J</u> 9Hz, $CH_2C\underline{H}$), 5.30 and 5.54 (each 1H, d, <u>J</u> 14Hz, $C\underline{H}_2Ar$), 5.96 (1H, d, <u>J</u> 13.5Hz, CH=C<u>H</u>S), 6.38 (1H, dq, <u>J</u> 1 and 7Hz, $CH_3C\underline{H}$=), 7.16 (1H, dd, <u>J</u> 13.5 and 10Hz, CH=C<u>H</u>NH), 7.77 and 8.21 (each 2H, d, <u>J</u> 9Hz, aromatic protons).

The above conversion of (e27) into (e6) was also carried out by a method analogous to that of Example 18 with a yield of 67%.

Example 23

p-Bromobenzyl (5R,6E)-3-[(E)-2-acetamidoethenylsulphinyl-6-ethylidene-7-oxo-1-azabicyclo[3.2.0]hept-2-ene-2-carboxylate and p-bromobenzyl (5R,6Z)-3-(E)-2-acetamido-ethenylsulphinyl-6-ethylidene-7-oxo-1-azabicyclo[3.2.0]hept-2-ene-2-carboxylate

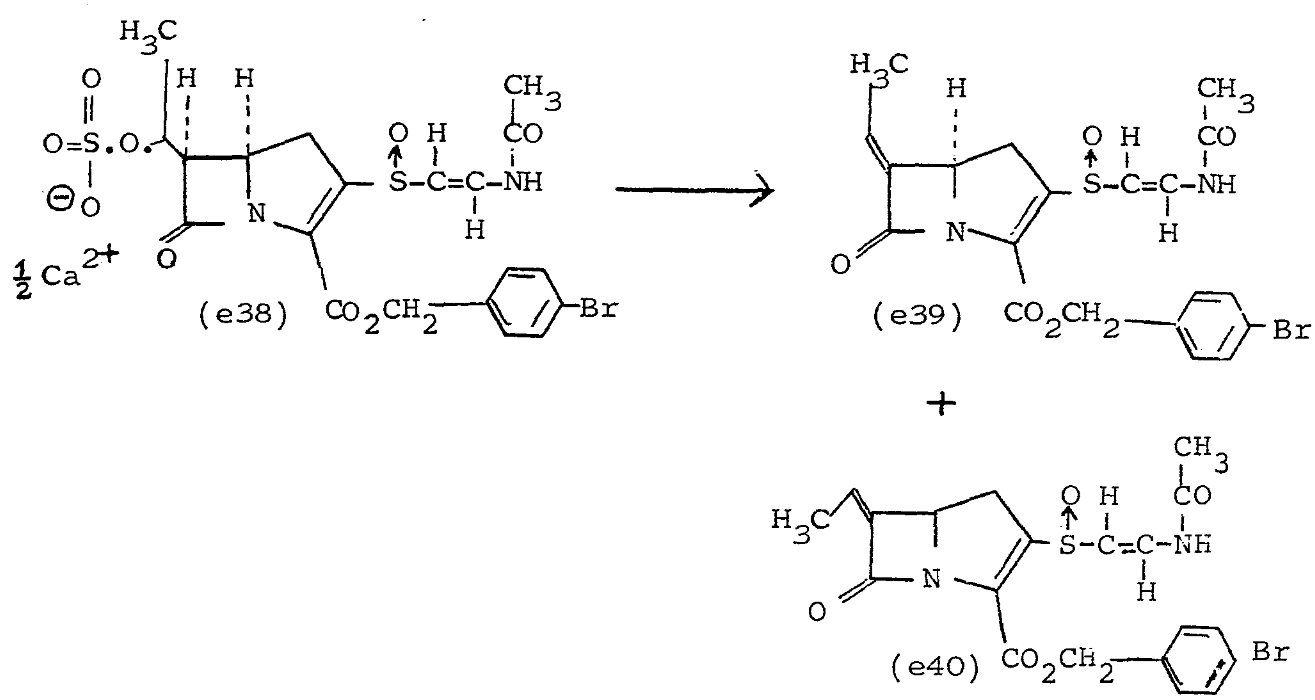

(e38) (e39) + (e40)

Calcium (5R,6R)-3-[(E)-2-acetamidoethenylsulphinyl]-6-[(S)-1-sulphonatoxyethyl]-7-oxo-2-p-bromobenzyloxycarbonyl-1-azabicyclo[3.2.0]hept-2-ene (e38) (180 mg) was heated under reflux in dioxan (20 ml) containing AMBERLITE IRC 50 (Na) (the sodium form of a carboxylate ion exchange resin) (3.0 g) for 1 3/4 hours. The solution was then cooled and filtered and the solvent evaporated to leave a gum (80 mg). This was chromatographed on silica gel (10 g), eluting with chloroform/ethanol mixtures to yield a mixture of p-bromobenzyl (5R,6E)-3-[(E)-2-acetamidoethenylsulphinyl-6-ethylidene-7-oxo-1-azabicyclo[3.2.0]hept-2-ene-2-carboxylate and

p-bromobenzyl (5R,6Z)-3-[(E)-2-acetamidoethenylsulphinyl-6-ethylidene-7-oxo-1-azabicyclo[3.2.0]hept-2-ene-2-carboxylate (e39 and e40) (5 mg); ν max ($CDCl_3$) 1785, 1710, 1630 $cm^{-1}$. ppm ($CDCl_3$) 1.85 (3H, d, J 8Hz), 2.08 (3H, s,), 2.9-3.8[2H, m(AB of an ABX system)], 4.8 (1H, broad t, J <u>ca</u>. 9Hz), 5.22 (2H, s), 6.2 (1H, d, J 15Hz), 6.48 (1H, broadened q, J <u>ca</u> 8Hz), 7.1-7.6 (5H, m), 8.35 (1H, d, J 9Hz).

A mass spectrum of the product showed an ion at m/e 363.01034 corresponding to $C_{16}H_{14}BrNO_4$ (requires m/e 363.01066), i.e. ($M^+$ - SCH:CHNHCOCH$_3$ + H).

The n.m.r. spectrum quoted is that of the (E)-isomer which is the major product of the reaction.

Example 24

Methyl (5R,6E)-3-[(E)-2-acetamidoethenylthio]-6-ethylidene-7-oxo-1-azabicyclo[3.2.0]hept-2-ene-2-carboxylate and methyl (5R,6Z)-3-[(E)-2-acetamido-ethenylthio] -6-ethylidene-7-oxo-1-azabicyclo[3.2.0] hept-2-ene-2-carboxylate

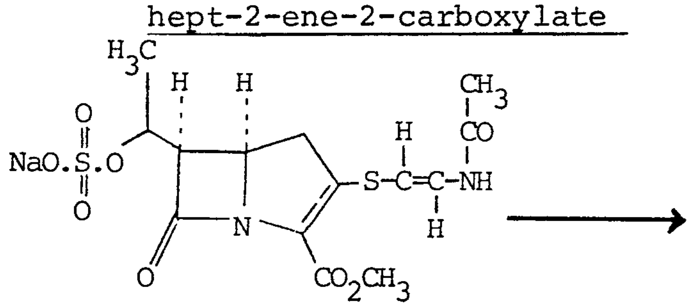

(e19)

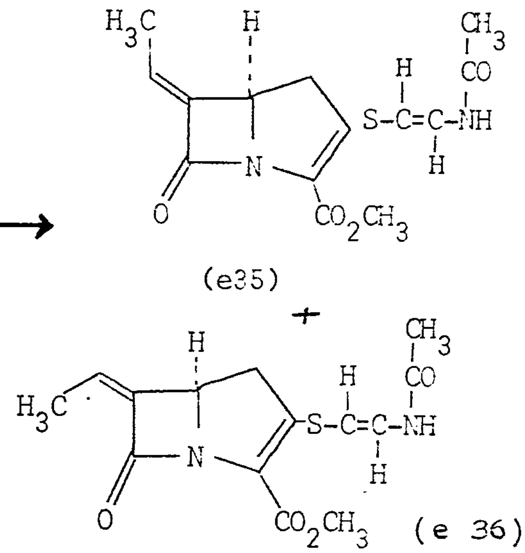

(e35)

+

(e 36)

Sodium (5R,6R)-3-[(E)-2-acetamidoethenylthio]-6-[(S)-1-sulphonatooxyethyl]-7-oxo-2-methoxycarbonyl-1-azabicyclo[3.2.0]hept-2-ene (e19) (370 mg) in dimethylformamide (6ml) was stirred with anhydrous potassium carbonate (560 mg) at room temperature for 20 hours. Most of the dimethylformamide was removed by evaporation and the product partitioned between chloroform (25 ml) and water (25 ml). The organic layer was washed with water (3 x ml) dried ($MgSO_4$) and evaporated. Chromatography of the product on silica gel (petrol - ethyl acetate as eluant) afforded a mixture of methyl (5R,6E)-3-[(E)-2-acetamidoethenyl-thio]-6-ethylidene-7-oxo-1-azabicyclo[3.2.0]hept-2-ene-2-carboxylate and methyl (5R,6Z)-3-[(E)-2-acetamidoethylthio]-6-ethylidene-7-oxo-1-azabicyclo [3.2.0.]hept-2-ene-2-carboxylate (e35 and e36) (160 mg) (*ca* 3:1) (e35 and e36). $\nu$ max ($CHCl_3$) 1770 (B-lactam CO), 1700 and 1625cm$^{-1}$, $\delta$ ($CDCl_3$) 1.79 (3H, d, *J* 7.5 $H_z$, $\underline{CH_3}CH$), 1.98 and 2.04 (3H, each s, $CH_3CO$ for Z- and *E*-

isomer, respectively), 3.00 and 3.05 (2H, each d, _J_ 9 and 10 Hz, 4-$CH_2$ for E- and Z-isomer, respectively), 3.80 ($C\underline{H}_3O_2C$), 4.65 (1H, M, 5-CH), 5.85 (1H, d, _J_ 14 Hz, SC_H_=), _ca_ 5.85 and 6.33 (1H, each qd, _J_ 7.5 and _ca_ 1 Hz, $CH_3$.C_H_ for 7 and E-isomer, respectively), 7.15 (1H, dd, _J_ 14 and 10 Hz, NH C_H_=) and 8.02 br (1H, d, _J_ 10 Hz, NH). [Found: $\underline{M}^+$, 308.08288. $C_{14}H_{16}N_2O_4S$ requires 308.08307].

Example 25

p-Bromobenzyl (5R,6E)-3-[(E)-2-acetamidoethenylthio]-6-ethylidene-7-oxo-1-azabicyclo[3.2.0]hept-2-ene-2-carboxylate and p-bromobenzyl (5R,6Z)-3-[(E)-2-acetamidoethenylthio]-6-ethylidene-7-oxo-1-azabicyclo[3.2.0]hept-2-ene-2-carboxylate

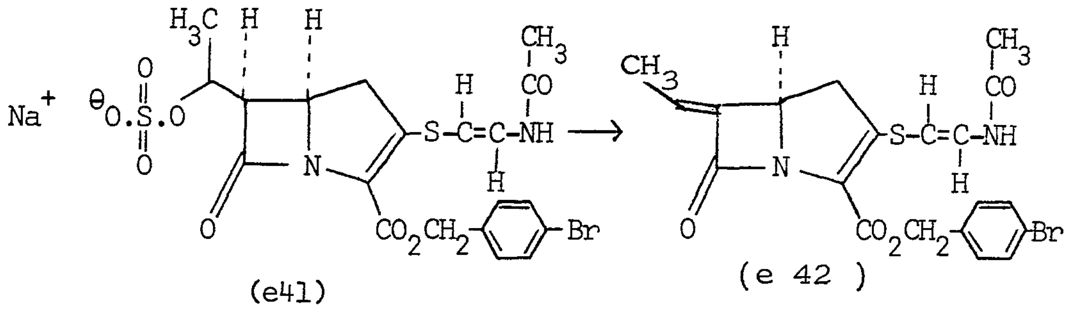

(e41) (e 42 )

Sodium (5R,6R)-3-[(E)-2-acetamidoethenylthio]-6-[(S)-1-sulphonatooxyethyl]-7-oxo-2-p-bromobenzyloxycarbonyl-1-azabicyclo[3.2.0]hept-2-ene (e41) (300 mg) in dimethylformamide (10 ml) was stirred at room temperature with anhydrous potassium carbonate (400 mg) for 20 hours. Most of the dimethylformamide was removed by evaporation and the product was diluted with ethyl acetate. The organic extract was washed with water (3 x 20 ml) and brine (20 ml), and then dried ($MgSO_4$). Evaporation gave a product which was fractionated on silica gel (petrol - ethyl acetate as eluant) to afford a mixture of p-bromobenzyl (5R,6E)-3-[(E)-2-acetamidoethenylthio]-6-ethylidene-7-oxo-1-azabicyclo[3.2.0]hept-2-ene-2-carboxylate and p-bromobenzyl (5R,6Z)-3-[(E)-2-acetamidoethenylthio]-6-ethylidene-7-oxo-1-azabicyclo[3.2.0]hept-2-ene-2-carboxylate (e42) (ca. 2:7) (104 mg) $\nu$ max. ($CHCl_3$) 1770, 1700 and

1625 $cm^{-1}$; ($CDCl_3$) 1.77 (3H, d, J 7.5 Hz, $CH_3CH$), 2.02 (3H, s, $CH_3CO$), 2.98 and 3.03 (2H, each d, J 8 and 9 Hz, 4-$CH_2$ for E and Z-isomer, respectively), 4.60 (1H, m, 5-CH), 5.08 and 5.17 (2H, ABq, J 13 Hz $CO_2CH_2$), 5.82 (1H, d, J 14 Hz, SCH=), 5.88 and 6.34 (1H, each qd, J 7.5 and ca. 1 Hz, for Z and E-isomer, respectively), 7.0-7.5 (5H, m, NHCH= and $C_6H_4Br$), and 7.85 br (1H, d, J 11 Hz, NH). [Found: $M^+$, 462.02042 $C_{20}H_{19}N_2O_4SBr$ requires 462.02493]

Example 26

Benzyl (5R,6E)-3-[(E)-2-acetamidoethenylthio]-6-ethylidene-7-oxo-1-azabicyclo[3.2.0]hept-2-ene-2-carboxylate and benzyl (5R,6Z)-3-[(E)-2-acetamido-ethenylthio]-6-ethylidene-7-oxo-1-azabicyclo[3.2.0]hept-2-ene-2-carboxylate

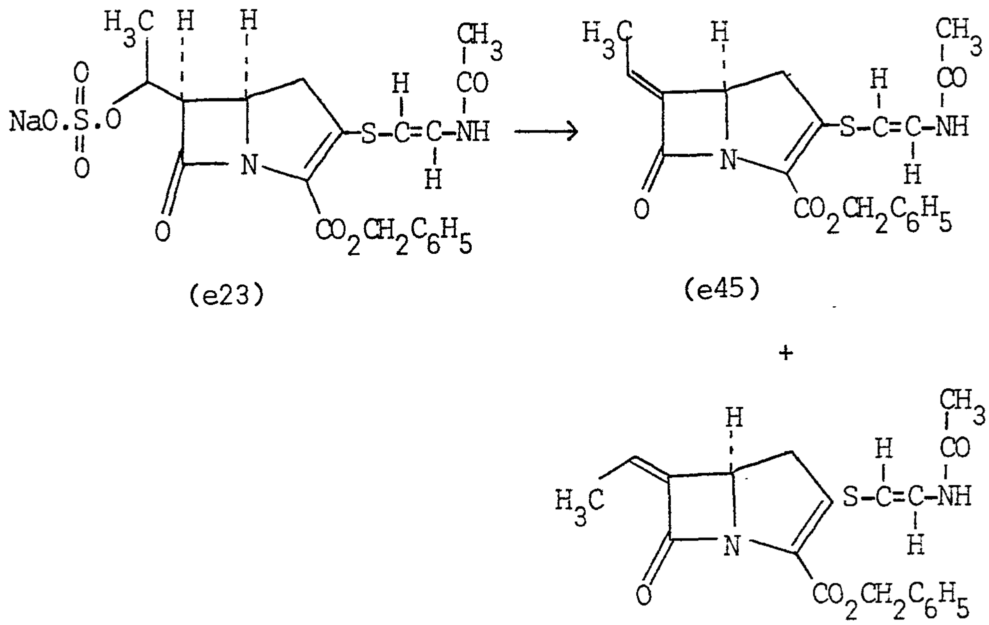

(e23)

(e45)

+

(e37)

Benzyl (5R,6R)-3-[(E)-2-acetamidoethenylthio]-6-[sodio (S)-1-sulphonatooxyethyl]-7-oxo-1-azabicyclo[3.2.0]hept-2-ene-2-carboxylate (e23) was stirred vigorously with potassium carbonate (750 mg) in dimethylformamide (10 ml) for 20 hours. The mixture was concentrated in vacuo, and the residue extracted with chloroform. The organic solution was washed with water (5 x 20 ml), dried ($MgSO_4$) and evaporated to give the crude product as a foam. The product was chromatographed on silica (ethyl acetate as eluant) to give benzyl (5R,)-3-[(E)-2-acetamidoethylthio]-6-ethylidene-7-oxo-1-azabicyclo[3.2.0]hept-2-

-ene-2-carboxylate (e45 and e37)(122 mg) in which the ratio of E- to Z- isomer was approximately 3.5:1. $\nu$ max. ($CHCl_3$) 3300 (br), 1770, 1700 and 1630cm$^{-1}$; $\lambda$ max. (EtOH) ca. 332 sh and 311 nm; $\delta$ ($CDCl_3$) E-isomer, 1.76 (3H, d, *J* 8 Hz, C$\underline{H}_3$CH), 2.00 (3H, s, $CH_3CO$), 2.98 (2H, d, *J* 9 Hz, 4-$CH_2$), 4.60 (1H, m, 5-CH), 5.24 (2H, centre of AA', wings at 5.09 and 5.39, C$\underline{H}_2$Ph), 5.81 (1H, d, *J* 14 Hz, SC$\underline{H}$=), 6.32 (1H dq, *J* 8 and 1Hz, C$\underline{H}_3$CH), 7.14 (1H, dd, *J* 14 and 10 Hz, NHC$\underline{H}$=), 7.30 (5H, m, $\underline{Ph}CH_2$) and 7.96 (1H, br, d, *J* 10 Hz, NH), Z-isomer as E-isomer except for 2.0 (3H, d, *J* 8 Hz, C$\underline{H}_3$CH), 302 (2H, d, *J* 9 Hz, 4-$CH_2$) and ca. 5.85 (1H, dq, *J* 8 and 1 Hz, $CH_3C\underline{H}$). Found: $M^+$, 384.1153, $C_{20}H_{20}N_2O_4S$ requires 384.1144.

Example 27

p-Nitrobenzyl (5R,6Z)-3-[(E)-2-acetamidoethenylthio]-6-ethylidene-7-oxo-1-azabicyclo[3.2.0]hept-2-ene-2-carboxylate and p-Nitrobenzyl (5R,6E)-3-[(E)-2-acetamidoethenylthio]-6-ethylidene-7-oxo-1-azabicyclo[3.2.0]hept-2-ene-2-carboxylate

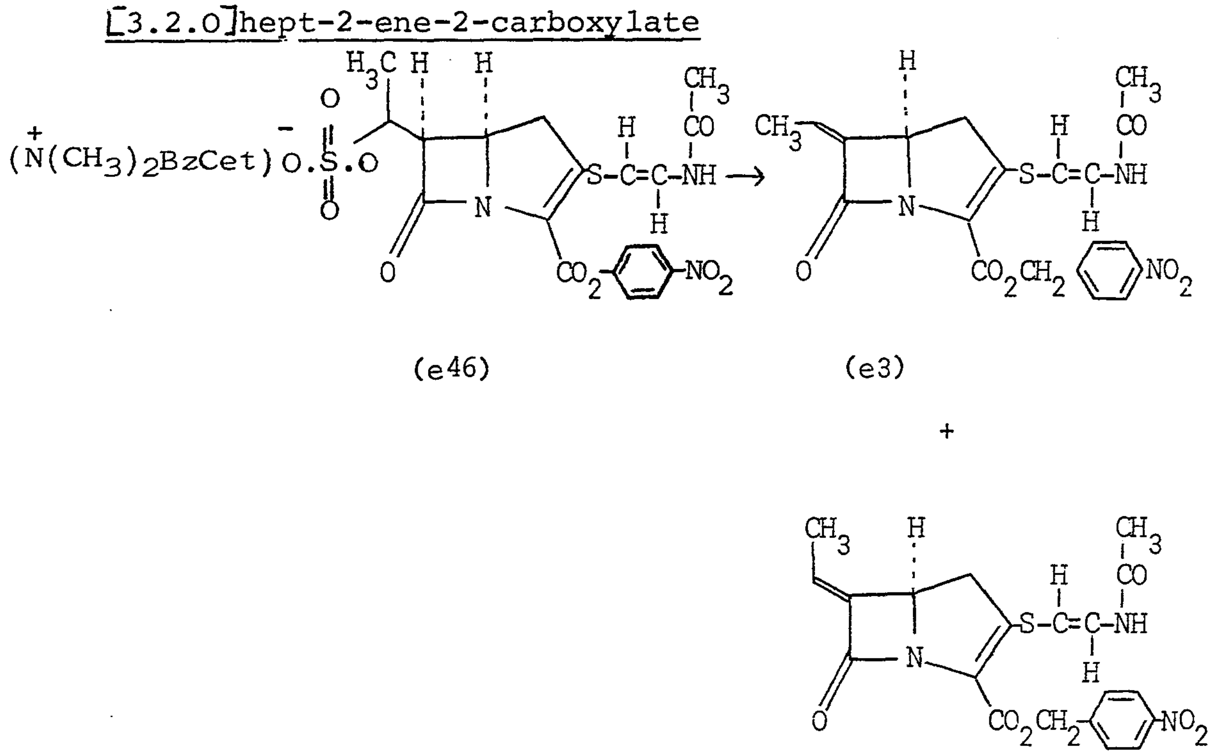

(e46) (e3)

\+

(e6)

Benzyldimethyl-n-hexadecylammonium (5R,6R)-3-[(E)-2-acetamidoethenylthio]-6-[(S)-1-sulphonatooxyethyl]-7-oxo-2-p-nitrobenzyloxycarbonyl-1-azabicyclo[3.2.0]hept-2-ene (e46) (1.85 mg) was dissolved in methylene chloride (40 ml) and the solution cooled to -15°. A solution of DBU (0.634 g) in methylene chloride (10 ml) was added, and stirring was continued at -10° for 3.5 hours. The organic solution was washed with 3 portions of brine (20 ml), then dried ($MgSO_4$) and evaporated. The residue was chromatographed on silica gel eluting first with petrol/ethyl acetate (1:4) and then with ethyl acetate

to yield p-nitrobenzyl (5R,6Z)-3-[(E)-2-acetamidoethenylthio] 6-ethylidene-7-oxo-1-azabicyclo[3.2.0]hept-2-ene-2-carboxylate and p-nitrobenzyl (5R,6E)-3-[(E)-2-acetamido-ethenylthio]-6-ethylidene-7-oxo-1-azabicyclo[3.2.0]hept-2-ene-2-carboxylate (e3 and e6) (0.46 g) in which the E- to Z- isomer ratio was about 1:1.2. Trituration of the mixed products under ethyl acetate gave a pale yellow solid; (Found; C, 55.8; H, 4.5, N, 9.7%, $\underline{M}^+$, 429.0999. $C_{20}H_{19}N_3O_6S$ requires C, 55.9; H, 4.5; N, 9.8%, $\underline{M}^+$ 429.0994). $\nu_{max}$ (KBr) 1760, 1690 and 1620 $cm^{-1}$; $\lambda_{max}$ (EtOH) 340sh (11,000), 310 (13,4 00) and 268 nm (17,400); $\delta$ (DMF-$d_7$) 1.82 and 2.00 (Total 3H, each d, $\underline{J}$ 7Hz, $C\underline{H}_3CH$ for E- and Z- isomer, respectively), ca. 1.98 (3H, s, $CH_3CO$), ca. 3.2 (2H, m, $C\underline{H}_2C.S$), 4.60-5.00 (1H, m, $C\underline{H}.CH_2$), 5.31 and 5.54 (each 1H, d, $\underline{J}$ 14, $C\underline{H}_2Ar$), 5.95 (1H, d, $\underline{J}$ 13.5, S.C$\underline{H}$=CH), 6.12 (dq, $\underline{J}$ 7 and 0.5Hz, $CH_3C\underline{H}$ for Z-isomer), 6.38 (dq, $\underline{J}$ 7 and 1Hz, $CH_3C\underline{H}$ for E-isomer), 7.15 (1H, dd, $\underline{J}$ 13.5 and 10.5Hz; NH.C$\underline{H}$=CH), 7.77 and 8.22 (each 2H, d, $\underline{J}$ 9Hz, aromatic protons).

## Example 28

**Methyl (5R,6E)-3-[(E)-2-acetamidoethenylthio]-6-ethylidene-7-oxo-1-azabicyclo[3.2.0]hept-2-ene-2-carboxylate and methyl (5R,6Z)-3-[(E)-2-acetamidoethenylthio]-6-ethylidene-7-oxo-1-azabicyclo[3.2.0]hept-2-ene-2-carboxylate**

**Separation of Isomers**

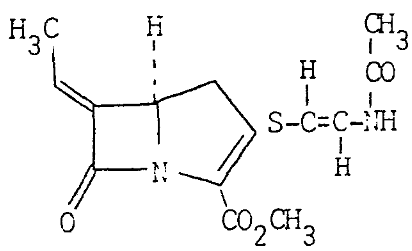

(e35)

+

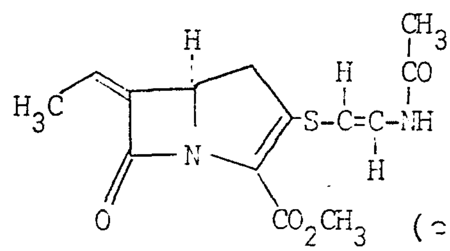

(e36)

A mixture of methyl (5R,6E)-3-[(E)-2-acetamidoethenylthio]-6-ethylidene-7-oxo-1-azabicyclo[3.2.0]hept-2-ene-2-carboxylate and methyl (5R,6Z)-3-[(E)-2-acetamidoethenylthio]-6-ethylidene-7-oxo-1-azabicyclo[3.2.0]hept-2-ene-2-carboxylate (e35 and e36) (150 mg) was chromatographed on silica gel (30 g) using a gradient elution starting with chloroform and finishing with chloroform/ethanol (3:2). The product was collected in 3 separate batches. The first batch (0.02g) consisting of the early fractions comprised of only the Z-isomer (e36) $\delta$ ($CDCl_3$) 2.03 (3H, d, $C\underline{H}_3CH$), 2.05 (3H, s, $CH_3CO$), 3.04 (2H, broad d, $\underline{J}$ 8Hz, $C\underline{H}_2CH$), 3.80 (3H, s, $CH_3O_2C$), 4.62 (1H, broad t, $\underline{J}$ 8Hz, $C\underline{H}.CH_2$), 5.90[2H, m, consisting of (d, $\underline{J}$ 14Hz, $CH{=}C\underline{H}.S$) and (q, $CH_3C\underline{H}{=}$)], 7.17 (1H, dd, $\underline{J}$ 14 and 10.5 Hz, $CH{=}C\underline{H}.NH$) and 8.25 (1H, broad d, $\underline{J}$ 10.5Hz, NH).

The second batch of product, obtained by combining the middle fractions, was a mixture of E- and Z-isomers (e35 and e36) (0.07 g) whilst the third batch, comprising the late fractions, consisted only of the E-isomer (e35), (0.028 g), $\delta$ ($CDCl_3$) 1.81 (3H, d, J 7.5Hz, $C\underline{H}_3CH$), 2.05 (3H, s, $CH_3CO$), 2.93 (1H, dd, J 17 and 8.5 Hz, $H_A$ of ABX), 3.17 (1H, dd, J 17 and 10Hz, $H_B$ of ABX), 3.80 (3H, s, $C\underline{H}_3O_2C$), 4.70 (1H, broad t, J 9Hz, $H_X$ of ABX), 5.87 (1H, d, J 14 Hz, S.C$\underline{H}$=CH), 6.36 (1H, dq, J 7.5 and 1Hz, $CH_3C\underline{H}$.NH) and 8.23 (1H, broad d, J 10Hz, NH).

Example 29

Methyl (5R,6E)-3-(2-acetamidoethylthio)-6-ethylidene-7-oxo-1-azabicyclo[3.2.0]hept-2-ene-2-carboxylate and methyl (5R,6Z)-3-(2-acetamidoethylthio)-6-ethylidene-7-oxo-1-azabicyclo[3.2.0]hept-2-ene-2-carboxylate

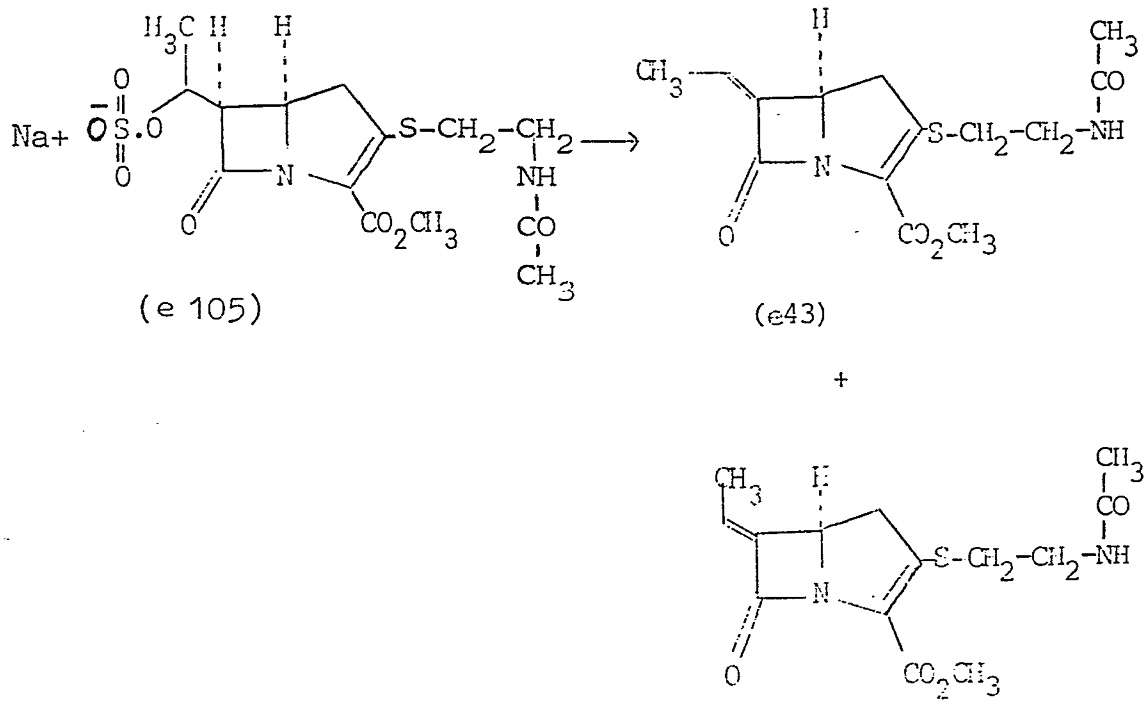

(e 105)

(e43)

+

(e44)

Methyl (5R,6R)-2-(2-acetamidoethylthio)-6-[sodium(S)-1-sulphonatooxyethyl]-7-oxo-1-azabicyclo[3.2.0]hept-2-ene-2-carboxylate (e105)(340 mg) was stirred at room temperature with potassium carbonate in dimethylformamide (10 ml) for 24 hours. The solvent was removed by evaporation, and the residue partitioned between ethyl acetate (40 ml) and water (40 ml). The organic layer was washed with water (3 x 20 ml) and brine (20 ml) and then dried ($MgSO_4$) and evaporated, to afford a gum (74 mg) which consisted largely of the ethylidene derivatives (e43 and e44). This material was chromatographed on silica gel using ethyl acetate to elute. Elution gave the ethylidene

derivatives (e43 and e44) (40 mg) (about 1:1); (Found: $M^+$, 310.0979; $C_{14}H_{18}N_2O_4S$ requires 310.0987), max ($CHCl_3$) 3450 br, 1770, 1705 and 1670 $cm^{-1}$; ($CDCl_3$) (E-isomer) 1.84 (3H, d, J 7.5Hz, $CH_3$.CH), 1.98 (3H, s, $CH_3CO$), 2.8-3.6 (6H, m, $CH_2$.C.S.$CH_2CH_2N$), 3.87 (3H, s, $CH_3O_2C$), 4.77 (1H, m, CH.$CH_2$.CS), 6.25 (1H, broad, NH) and 6.45 (1H, dq, J 7.5 and 1.5Hz, $CH_3CH$=), (Z-isomer) as for E-isomer except 2.07 (3H, dd, J 7.5 and 1Hz, $CH_3$.CH=) and 6.01 (1H, dq, J 7.5 and 0.5Hz, $CH_3CH$=).

<u>Example 30</u>

<u>Methyl (5R,6R)-3-[(E)-2-acetamidoethenylthio]-6-[(R)-1-phenylthioethyl]-7-oxo-1-azabicyclo[3.2.0]hept-2-ene carboxylate and methyl (5R,6R)-3-[(E)-2-acetamidoethenyl-thio]-6-[(S)-1-phenylthioethyl]-7-oxo-1-azabicyclo[3.2.0] hept-2-ene-carboxylate</u>

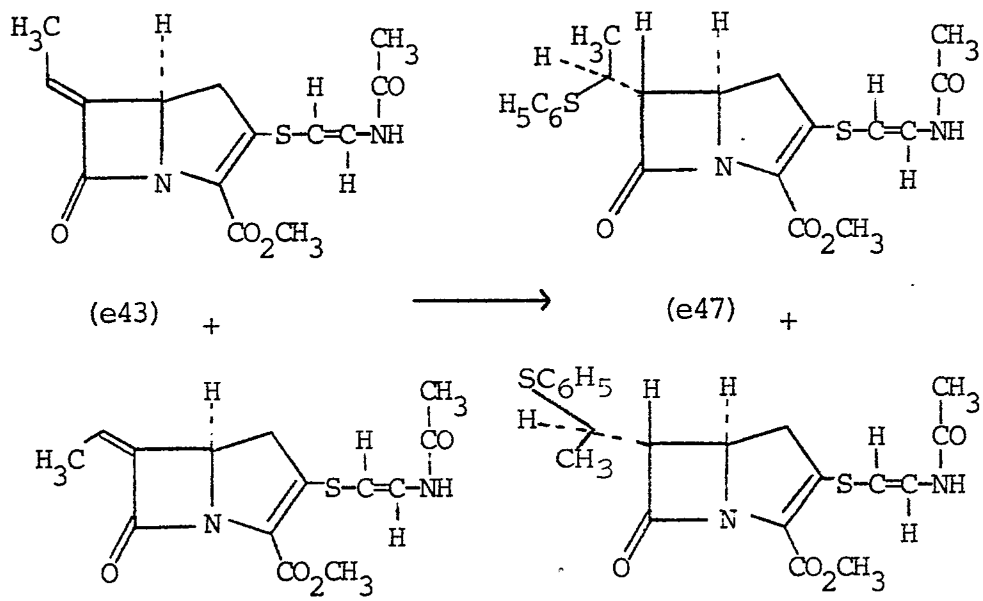

(e43) + (e47) + (e48)

A mixture of methyl (5R,6E)-3-[(E)-2-acetamidoethenylthio]-6-ethylidene-7-oxo-1-azabicyclo[3.2.0]hept-2-ene-2-carboxylate and methyl (5R,6Z) -3-[(E)-2-acetamidoethenylthio]-6-ethylidene-7-oxo-1-azabicyclo[3.2.0] hept-2-ene-2-carboxylate (e35 and e36) (0.1 g) was dissolved in dimethylformamide (3 ml) and the solution cooled to -10°. To the stirred solution was added anhydrous potassium carbonate (0.023 g) followed by thiophenol (0.036 g). After 40 minutes at -10°, ethyl acetate (15 mls) was added and the organic layer was washed with brine (15 ml), water (2 x 15 ml),

and again with brine (15 ml) before drying ($MgSO_4$) and evaporating to give the crude product. Chromatography on silica gel using ethylacetate/petrol mixtures (gradient elution from ethyl acetate/petrol 3:7 to neat ethyl acetate) yielded a mixture of methyl (5R,6R)-3-[(E)-2-acetamido-ethenylthio]-6-[(R)-1-phenylthioethyl]-7-oxo-1-azabicyclo [3.2.0]hept-2-ene-carboxylate and the corresponding 6-[(S)-1-phenylthioethyl] stereoisomer (e48 and e47) (0.02 g). Found: M$^+$, 418.1024; $C_{20}H_{22}N_2O_4S_2$ requires 418.1021, max. ($CHCl_3$) 1780, 1700 and 1625 $cm^{-1}$; max (EtOH) 321 and 221 nm.; ($CDCl_3$) 1.35 and 1.42 (total 3H, each d, J 6Hz, $CH_3CH$), 2.05 (3H, s, $CH_3CO$), 2.6 4.2 (5H, m, $CH_3.CH.CH.CH.CH_2$), 3.77 (3H, s, $CH_3O_2C$), (1H, d, J 13.5, CH=CH.S), 7.17 (1H, dd, J 13.5 and 10.5Hz, CH=CHNH), 7.28 (5H, br, m, PhS) and 8.27 (1H, broad d, J 10.5Hz, NH).

Example 31

Benzyl (5R,6R)-3-[(E)-2-acetamidoethenylthio]-6-[(R)-1-ethylthioethyl]-7-oxo-1-azabicyclo[3.2.0]hept-2-ene-2-carboxylate and benzyl (5R,6R)-3-[(E)-2-acetamidoethenylthio]-6-[(S)-1-ethylthioethyl]-7-oxo-1-azabicyclo[3.2.0]hept-2-ene carboxylate

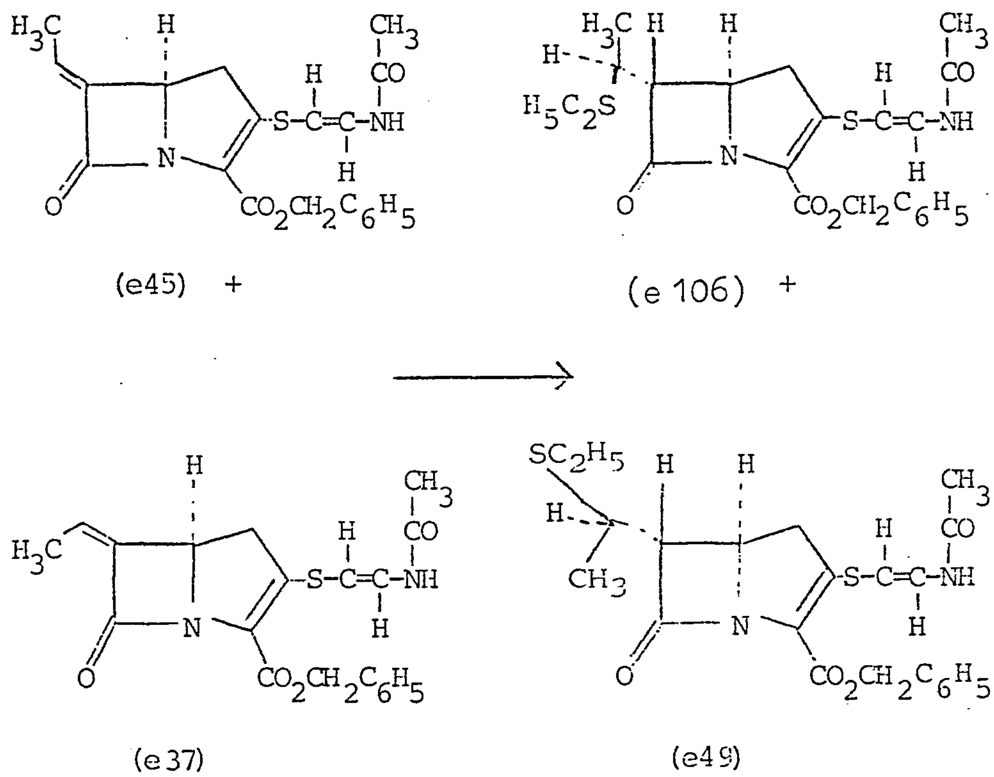

(e45) + (e 106) +

(e37) (e49)

A mixture of benzyl (5R,6E)-3-[(E)-2-acetamidoethenylthio]-6-ethylidene-7-oxo-1-azabicyclo[3.2.0]hept-2-ene-2-carboxylate and benzyl (5R,6Z)-3-[(E)-2-acetamidoethenylthio]-6-ethylidene-7-oxo-1-azabicyclo[3.2.0]hept-2-ene-2-carboxylate (e45 and e37) (0.30 g) was dissolved in dimethylformamide ((5 ml) and the solution cooled to -20$^{o}$. To the stirred solution was added anhydrous potassium carbonate (0.054 g) followed by ethanethiol (4 drops, estimated 1 equiv) After 25 minutes at -20$^{o}$, the solution was diluted with ethyl acetate (30 ml) and the organic layer was washed with aqueous potassium carbonate (10 ml), water (30 ml)

and brine (30 ml). Evaporation of the dried ($MgSO_4$) solution gave a residue which was chromatographed on silica gel using petrol/ethyl acetate as eluant. The major product (0.169 g) was an isomeric mixture of the ethylthio derivative (e 106) and (e49).

The 2 isomers were separated by a combination of further chromatography on silica gel (using a gradient elution beginning with ethyl acetate/petrol, 2:3 and finishing with ethyl acetate) and crystallisation from ethyl acetate/petrol. The least polar isomer was obtained as a white crystalline solid; m.p. 191-193. [Found: C, 58.9; H, 5.7; N, 6.1%. $C_{22}H_{26}N_2O_4S_2$ requires C, 59.2 H, 5.9; N, 6.3%] $\lambda_{max}$ (EtOH) 325 (15,400) and 229 (14,900) nm $\nu_{max}$ (KBr) 1780, 1710, 1680 and 1620 $cm^{-1}$; $\delta$ (DMF-$d_7$) 1.19 (3H, t, J 7Hz, $C\underline{H}_3CH_2$), 1.37 (3H, d, J 6.5Hz, $C\underline{H}_3CH$), 1.98 (3H, s, $CH_3CO$), 2.60 (2H, q, J 7Hz, $C\underline{H}_2CH_3$), 3.20 (2H, d, J 9Hz, $CHC\underline{H}_2$). ca. 3.5 (2H, m, $CH_3C\underline{H}.C\underline{H}.C\underline{H}$), 4.07 (1H, dt, J 3 and 9Hz, $CHC\underline{H}CH_2$), 5.24 (2H, centre of AB, wings at 5.09 and 5.38, $PhC\underline{H}_2$), 5.92 (1H, d, J 14Hz, S.C$\underline{H}$=ch), 7.13 (1H, dd, J 10 and 14Hz, NH.C$\underline{H}$=CH), 7.25-7.55 (5H, m, $\underline{Ph}CH_2$).

The more polar isomer was isolated as a gum; $\nu_{max}$ ($CHCl_3$), 1780, 1700 and 1625 $cm^{-1}$, $\delta$ ($CDCl_3$) 1.22 (3H, t, J 7Hz, $C\underline{H}_3C$) 1.33 (3H, d, J 6.5Hz, $C\underline{H}_3CH$), 2.00 (3H, s, $CH_3CO$), 2.54 (2H, q, J 7Hz, $C\underline{H}_2CH_3$), 2.7-3.4 (3H, m, $C\underline{H}_2CH$ and C$\underline{H}$.SEt), 3.38 (1H, dd, J 3 and 6Hz, CH.C$\underline{H}$CH), 4.03 (1H, m, $C\underline{H}CH_2$), 5.23 (2H, centre of AB with wings at 5.08 and 5.38, $C\underline{H}_2Ph$), 5.80 (1H, d, J 13.5Hz, CH=C$\underline{H}$S) 7.0-7.5 (6H, m, $\underline{Ph}CH_2$ + CH=C$\underline{H}$.NH) and 7.92 (1H, d, J 10Hz, NH).

Example 32

p-Nitrobenzyl (5R,6R)-3-[(E)-2-acetamidoethenylthio]-6-[(R)-1-ethylthioethyl]-7-oxo-1-azabicyclo[3.2.0]hept-2-ene-2-carboxylate and p-nitrobenzyl (5R,6R)-3-[(E)-2-acetamidoethenylthio]-6-[(S)-1-ethylthioethyl]-7-oxo-1-azabicyclo[3.2.0]hept-2-ene-carboxylate

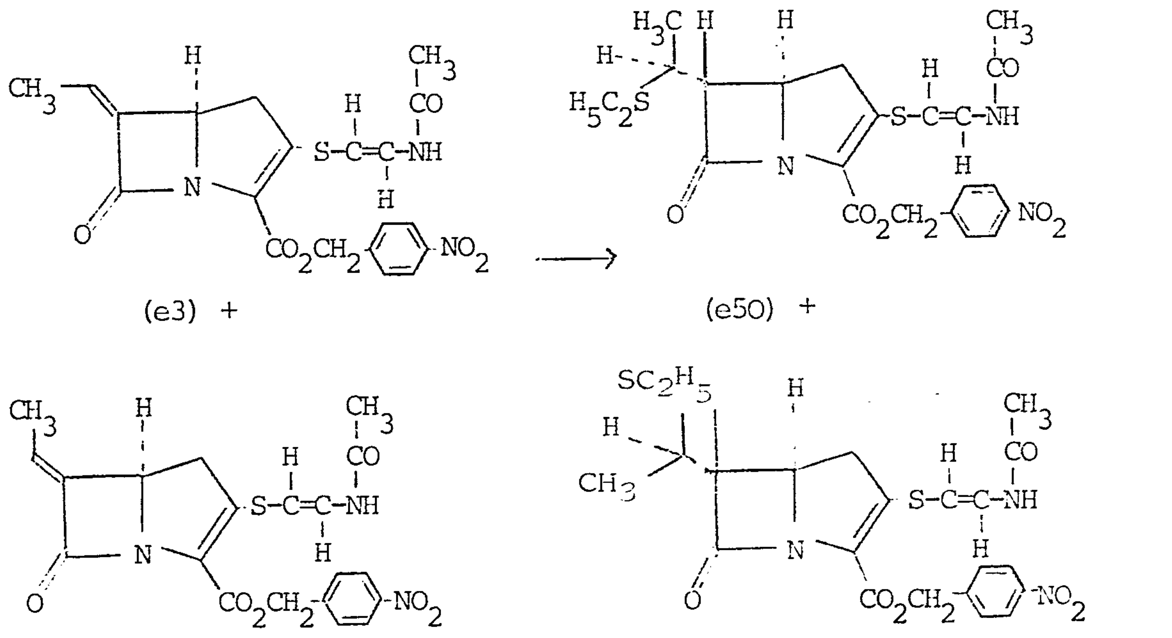

(e3) + (e50) +

(e6) (e51)

A mixture of p-nitrobenzyl (5R,6Z)-3-[(E)-2-acetamidoethenylthio]-6-ethylidene-7-oxo-1-azabicyclo[3.2.0]hept-2-ene-2-carboxylate and p-nitrobenzyl (5R,6E)-3-[(E)-2-acetamidoethenylthio]-6-ethylidene-7-oxo-1-azabicyclo[3.2.0]hept-2-ene-2-carboxylate (e3 and e6) (0.420 g) were dissolved in dimethylformamide (5 ml) and the solution cooled to -20°. Anhydrous potassium carbonate (0.068 g) followed by ethanethiol (0.058 g) were added to the solution which was then stirred at -20° for 20 minutes. The solution was diluted with ethyl acetate, and the organic layer was washed with aqueous potassium carbonate (10 ml), water (3 x 30 ml) and brine (30 ml).

Evaporation of the dried ($MgSO_4$) solution gave a crude product which was chromatographed on silica gel using petrol/ethyl acetate 1:1 ⟶ 1:4 to elute.

The first eluted component from the column was the least polar isomer of the ethylthio-derivative which was obtained as a pale-yellow crystalline solid from ethyl acetate/petrol (0.099 g) m.p. 195-198°. [Found: C, 53.6; H, 5.1; N, 8.6%. $C_{22}H_{25}N_3O_6S_2$ requires C, 53.8; H, 5.1; N, 8.6%]. $\lambda_{max}$ (EtOH) 327 (13,800), 266 (14,100) and 220 (14,00); $\nu_{max}$ (KBr) 1775, 1690 broad and 1620 $cm^{-1}$; $\delta$ (DMF-$d_7$) 1.20 (3H, t, *J* 7Hz, $CH_3CH_2$), 1.39 (3H, d, *J* 6.5 $CH_3CH$), 1.99 (3H, s, $CH_3CO$), 2.62 (2H, q, $CH_2CH_3$), 3.25 (2H, d, *J* 9Hz, $CH_2CH$), *ca.* 3.4 (1H, m, CH.SEt), 3.53 (1H, dd, *J* 8.5 and 3Hz, CHCH.CH), 4.12 (1H, dt, *J* 3 and 9Hz, $CH.CH_2$), 5.30 and 5.53 (each 1H, d, *J* 14Hz $ArCH_2$), 5.95 (1H, d, *J* 13.5Hz, CH=CH.S), 7.14 (1H, dd, *J* 10 and 13.5Hz, CH=CH.NH), 7.75 and 8.21 (each 2H, d, *J* 9Hz, aromatic protons.

Further elution gave a product consisting of both p-nitrobenzyl (5R,6R)-3-[(E)-2-acetamidoethenylthio]-6-[(R)-1- ethylthioethyl]-7-oxo-1-azabicyclo[3.2.0]hept-2-ene-2-carboxylate and p-nitrobenzyl (5R,6R)-3-[(E)-2-acetamido-ethenylthio]-6-[(S)-ethylthioethyl]-7-oxo-1-azabicyclo [3.2.0] hept-2-ene-carboxylate isomers (e51 and e50) (0.174 g). This was further chromatographed on silica gel to afford a pure sample of the more polar isomer as a pale-yellow foam; $\nu_{max}$ ($CHCl_3$) 1780, 1700 and 1620 $cm^{-1}$. $\delta$ (DMF-$d_7$) 1.19 (3H, t, *J* 7Hz, $CH_3CH_2$), 1.31 (3H, d, *J* 6.5Hz, $CH_3CH$), 1.98 (3H, s, $CH_3CO$), 2.61 (1H, q, *J* 7Hz, $CH_2CH_3$), 3.24 (2H, d, *J* 9Hz, $CH_2CH$), *ca.* 3.4 (1H, m, CH.SEt), 3.75 (1H, dd, *J* 3 and 6Hz, CHCHCH), 4.15 (1H, dt, *J* 3 and 9Hz, $CHCH_2$), 5.29 and 5.53 (each 1H, d, *J* 14Hz, $CH_2Ar$), 5.95 (1H, d, *J* 13.5Hz, CH=CH.S), 7.14 (1H, dd, *J* 13.5 and 10.5Hz, NHCH=CH), 7.75 and 8.20 (each 2H, d, *J* 9Hz, aromatic protons) and 10.29 (1H, broad d, *J* 10.5Hz, NH.

Example 33

Sodium (5R,6R)-3-[(E)-2-acetamidoethenylthio]-6-(-1-ethylthioethyl)-7-oxo-1-azabicyclo[3.2.0]hept-2-ene carboxylate

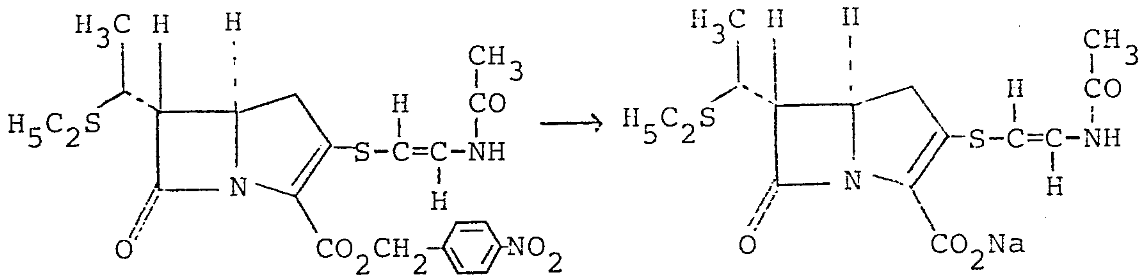

(e51) (e52)

The ethylthio-derivative of Example 32 (least polar isomer) (98 mg) was added to 5% 'unreduced' Pd on C (0.150 g) which had been prehydrogenated for 0.5 hours in a mixture of dioxan (7.0 ml) and water (3.5 ml). The mixture was hydrogenated for a further 3.25 hours before adding a solution of sodium bicarbonate (17 mg) in water (1 ml). The mixture was filtered through celite washing well with water (5 ml) and dioxan (5 ml). The solution was concentrated by evaporation to a volume of about 10 ml and then was extracted with ethyl acetate (3 x 20 ml). The aqueous layer was evaporated in vacuo, and then evaporated down from ethanol (10 ml) and finally toluene (10 ml). Sodium (5R,6R)-3-[(E)-2-acetamidoethenylthio]-6-(-1-ethylthio-ethyl)-7-oxo-1-azabicyclo[3.2.0]hept 2-ene-2-carboxylate (e52) was obtained as a yellow solid; $\nu_{max}$ (EtOH) 308 and 228 nm. $\lambda_{max}$ (KBr) 1750 (β-lactam, 1670 (amide) and 1600cm$^{-1}$ (broad - $CO_2$).

The sodium salt showed the following anti-bacterial spectrum when tested in a standard MIC test in DST agar + 5% horse blood:

| | |
|---|---|
| Citrobacter freundii E8 | 3.1 |
| Enterobacter cloacae M 1 | 12.5 |
| E.coli 0111 | 3.1 |
| Klebsiella aerogenes A | 0.8 |
| Proteus mirabilis 0977 | 12.5 |
| Proteus morganii I 580 | 12.5 |
| Proteus rettgeri WM 16 | 6.25 |
| Proteus vulgaris WO 91 | 6.25 |
| Ps aeruginosa A | 100 |
| Salmonella typhimurium CT10 | 6.25 |
| Serratia marcescens US20 | 6.25 |
| Shigella sonnei MB 11967 | 3.1 |
| Staph. aureus Oxford | 0.8 |
| Staph. aureus Russell | 0.8 |
| Staph. aureus 1517 | 6.25 |
| Strep. faecalis I | 50 |

Example 34

Benzyl (5R,6R)-3-[(E)-2-acetamidoethenylthio]-6-ethyl-7-oxo-1-azabicyclo[3.2.0]hept-2-ene-2-carboxylate

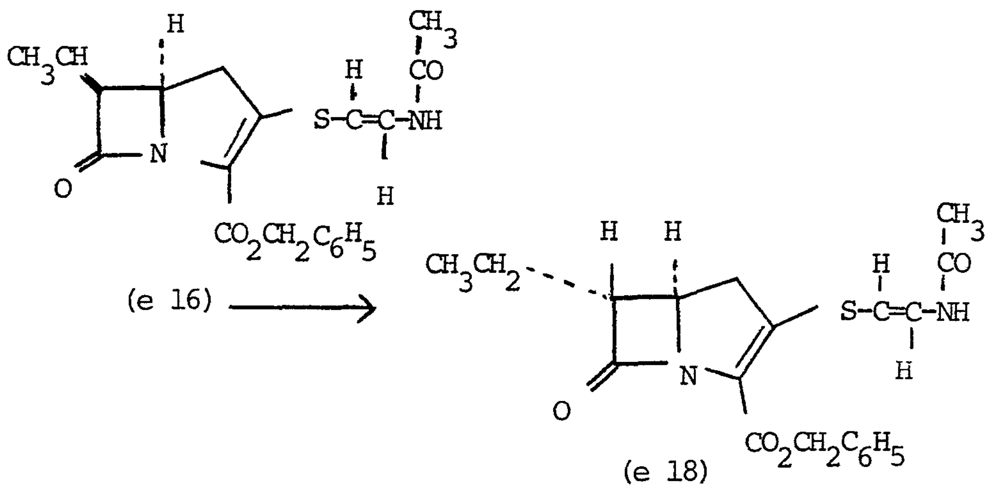

(e 16) (e 18)

Benzyl (5R)-3-[(E)-2-acetamidoethenylthio]-6-ethylidene-7-oxo-1-azabicyclo[3.2.0]hept-2-ene-2-carboxylate (e16) (100 mg) was dissolved in a mixture of ethanol (2 ml) and pyridine (1ml), and the solution was cooled to $-5^{o}$. Sodium borohydride (9.9 mg) was added to the solution which was then stirred at $-5^{o}$ for 1.5 h. Ethyl acetate (20 ml) was added and the organic solution was successively washed with water (10 ml), pH3 phosphate buffer (2 x 10 ml), dilute aqueous sodium bicarbonate solution (10 ml) and brine (10 ml). The dried ($MgSO_4$) organic solution was concentrated in vacuo and the residue was chromatographed on silica gel, employing a gradient elution of petroleum ether: ethyl acetate mixtures (3:7→1:9). The requisite fractions were combined and evaporated in vacuo to afford benzyl (5R, 6R)-3-[(E)-2-acetamidoethenylthio]-6-ethyl-7-oxo-1-azabicyclo [3.2.0]hept-2-ene-2-carboxylate (e 18) (17 mg); $\lambda$max. (EtOH) 325 and 228 nm., $\nu$max. ($CHCl_3$) 1775, 1700 and 1625 $cm^{-1}$; $\delta$($CDCl_3$) 1.02 (3H, t, J7 Hz, $C\underline{H}_3CH_2$) 1.6-1.95 (2H, m, $C\underline{H}_2CH_3$) 2.8 - 3.3 (3H, m, 4-$C\underline{H}_2$ and 6-C$\underline{H}$) 3.87 (1H, dt, J3 and 9Hz, 5-C$\underline{H}$) 5.27 (2H, centre of AB with wings at 5.12 and 5.43 $C\underline{H}_2$Ph) 5.85 (1H, d, J 13 Hz, = C$\underline{H}$.S), 6.95 - 7.55 (6H, m, = C$\underline{H}$.N and aromatic protons) and 7.80 (1H, br d, $\underline{J}$ 10 Hz, N$\underline{H}$).

Example 35

p-Nitrobenzyl (5R,6R)-3-[(E)-2-acetamidoethenylthio]-6-ethyl-7-oxo-1-azabicyclo[3.2.0]hept-2-ene-2-carboxylate

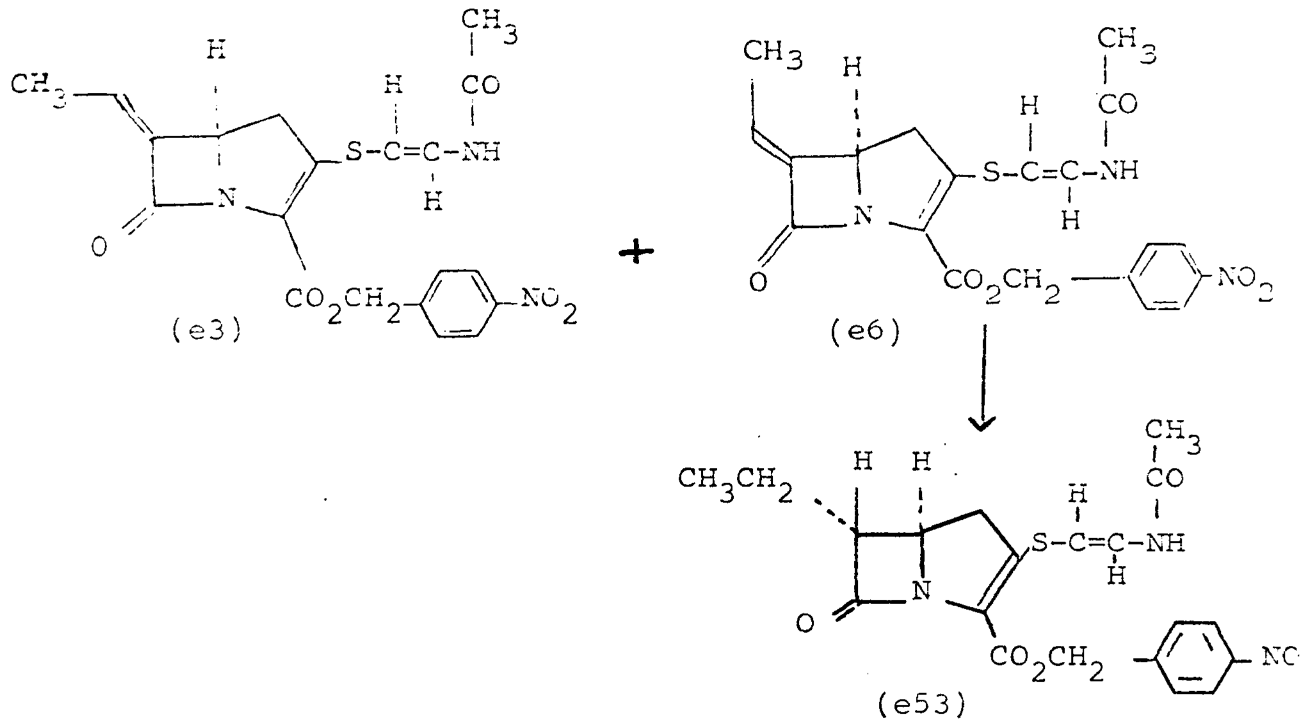

(e3) + (e6) → (e53)

p-Nitrobenzyl (5R)-3-[(E)-2-acetamidoethenylthio]-6-ethylidene-7-oxo-1-azabicyclo[3.2.0]hept-2-ene-2-carboxylate (e 3 and e 6) (155 mg) was dissolved in tetrahydrofuran (10 ml). The solution was cooled to -10$^{o}$ and to it was added a solution of sodium borohydride (55 mg) in pH 7 buffer solution (0.5 ml). The solution was warmed to 5$^{o}$ and maintained at that temperature for 1.5 h. Ethyl acetate (30 ml) was added and the organic solution was washed with water (30 ml) and brine (20 ml). The solution was dried ($MgSO_4$) and evaporated in vacuo. The product was chromatographed on silica gel using petrol/ethylacetate mixtures (2:3 → 1:9) to elute.

The appropriate fractions were combined and evaporated in vacuo to afford p-nitrobenzyl (5R,6R)-3-[(E)-2-acetamidoethenylthio]-6-ethyl-7-oxo-1-azabicyclo [3.2.0] hept-2-ene-2-carboxylate (e53) (40 mg) as an oil; $\lambda$max. (EtOH) 325 , 263 and 231 nm., $\nu$max. ($CHCl_3$) 1775, 1700, and 1625 cm$^{-1}$., $\delta$ ($CDCl_3$) 1.03 (3H, t, J 7 Hz, $CH_3CH_2$), ca. 1.6 - 1.95 (2H, m, $CH_2CH_3$) 2.06 (3H, s, $CH_3CO$) 2.75 -

3.4 (3H, m, 4-$CH_2$ and 6-CH), 3.91 (1H, dt, *J* 3 and 9Hz, 5-CH) 5.22 and 5.48 (each 1H, d, *J* 9 Hz, $CH_2$Ar) 5.88 (1H, d, *J* 14 Hz, = CH S) 7.20 (1H, dd, *J* 11 and 14 Hz, = CH — N) 7.63 and 8.19 (each 2H, d, *J* 8.5 Hz, aromatic protons) and 8.05 (1H, br d, *J* 11 Hz, NH) [Found: $M^+$ 431.1179, $C_{20}H_{21}N_3O_6S$ requires $M^+$, 431.1150]

Example 36

Sodium (5R,6R)-3-[(E)-2-acetamidoethenylthio]-6-ethyl-7-oxo-1-azabicyclo[3.2.0]hept-2-ene-2-carboxylate

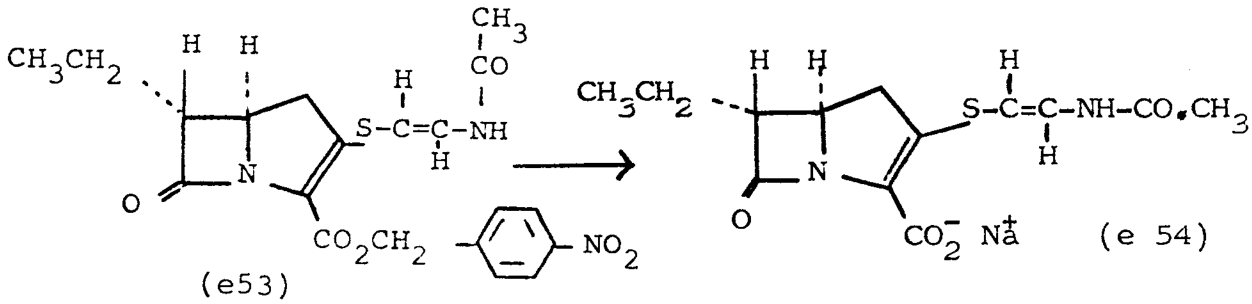

(e53) (e 54)

A solution of the ester from Example 35 (e 53) (40mg) in 20% aqueous dioxan (3ml) was added to a mixture of 5% Pd on C and 20% aqueous dioxan (6ml) which had been prehydrogenated at 20° and atmospheric pressure for 0.5h. Hydrogenation was continued for 4h and sodium bicarbonate (8mg) was then added. The mixture was filtered over Hiflo, washing well with water, and the aqueous solution was concentrated in vacuo to a volume of 10 ml. The aqueous solution was washed with ethyl acetate (3x 25ml) and was then further concentrated to a volume of 3 ml. The solution was then chromatographed on a column (15 x 2.5 cm) of Biogel P2, eluting with deionised water. Fractions were monitored by UV and those having a chromophore at $\lambda$max. ($H_2O$) 307 and 226 nm contained sodium (5R,6R)-3-[(E)-2-acetamidoethenylthio]-6-ethyl-7-oxo-1-azabicyclo[3.2.0]hept-2-ene-2-carboxylate (e 54) .

The minimum inhibitory concentrations (MIC) of the compound (e54) was determined using a standard DST agar + 10% horse blood substrate. The tests used an undiluted inoculum of bacteria. The results are shown below.

| ORGANISM | MIC (μg/ml) |
|---|---|
| *Citrobacter freundii* E8 | 12.5 |
| *Enterobacter cloacae* N1 | 25 |
| *Escherichia coli* O111 | 3.1 |
| *Escherichia coli* JT 39 | 3.1 |
| *Klebsiella aerogenes* A | 3.1 |
| *Proteus mirabilis* C977 | 25 |
| *Proteus morganii* I580 | 25 |
| *Proteus rettgeri* WM16 | 25 |
| *Proteus vulgaris* WO91 | 25 |
| *Pseudomonas aeruginosa* A | >50 |
| *Salmonella typhimurium* CT10 | 3.1 |
| *Serratia marcescens* US20 | 12.5 |
| *Shigella sonnei* MB 11967 | 3.1 |
| *Bacillus subtilis* A | 0.8 |
| *Staphylococcus aureus* Oxford | 0.4 |
| *Staphylococcus aureus* Russell | 0.4 |
| *Staphylococcus aureus* 1517 | 12.5 |
| *Streptococcus faecalis* I | 25 |
| *Streptococcus pneumoniae* CN33 | 0.1 |
| *Streptococcus pyogenes* CN10 | 0.2 |
| *E. coli* ESS | 0.4 |

Example 37

Sodium (5R,6R)-3-(2-acetamidoethylthio)-6-[(S)-1-sulphonatooxyethyl]-7-oxo-2-p-methoxycarbonylbenzyloxycarbonyl-1-azabicyclo-[3.2.0]hept-2-ene

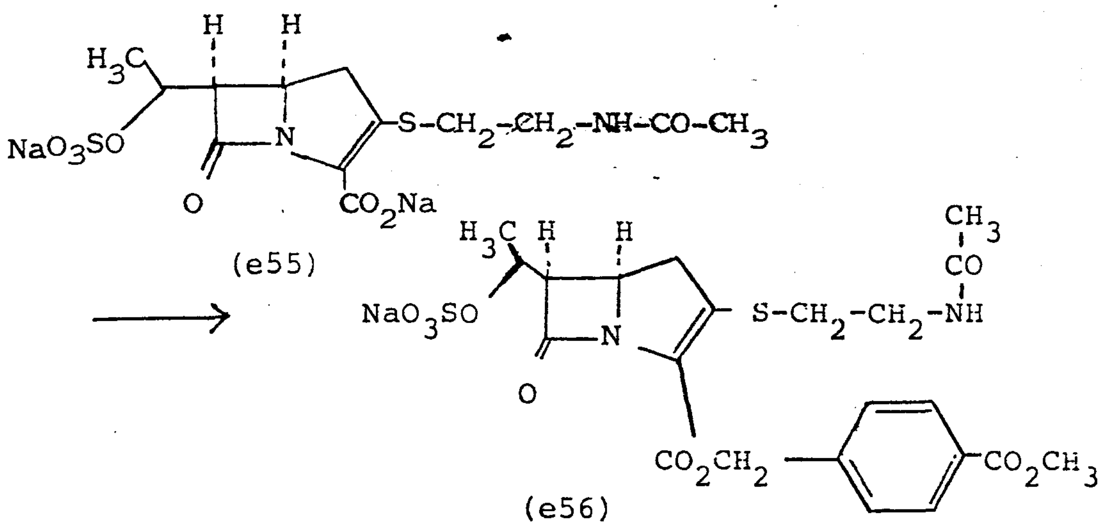

(e55)

(e56)

The di-salt (e55) (2.93g) was suspended in DMF (50ml) and treated with methyl 4-bromomethylbenzoate (4.5g). After 3h., the solvent was removed in vacuo and the residual oil was suspended in $CHCl_3$ (70ml) and silica gel (20g; 230-400 mesh) was added. The chloroform was removed in vacuo, and the residue was re-suspended in chloroform (30ml) and loaded on a silica gel column (100g; 2 : 1 mixture of 230-400 mesh and <230 mesh). The column was eluted with chloroform (200ml), ethanol/chloroform (3 : 7, 100ml), ethanol/chloroform (4 : 6, 500ml) and ethanol/chloroform (1 : 1), to yield Sodium (5R,6R)-3-(2-acetamidoethylthio)-6-[(S)-1-sulphonatoxy-ethyl]-7-oxo-2-p-methoxycarbonylbenzyloxycarbonyl-1-azabicyclo[3.2.0]hept-2-ene (e56) (2.06g). $\lambda$max ($H_2O$): 317 ($\epsilon$ 10100) and 234 ($\epsilon$ 15900) nm. $\nu$max (KBr): 1790, 1750, 1718, 1700, 1650 $cm^{-1}$. $\delta$(DMSO - $d_6$): inter alia 1.38 (3H,d,J 6Hz, $C\underline{H}_3CH$),1.80 (3H,s,$C\underline{H}_3CO$), 3.83 (3H, s,$CO_2C\underline{H}_3$), 4.0 - 4.6 (2H,m,$CH_3C\underline{H}$ and C5-$\underline{H}$), 5.20 and 5.37 (2H, ABq, J 15Hz, $C\underline{H}_2Ar$), 7.56 and 7.96 (each 2H, d,J 8Hz,$C_6\underline{H}_4$), 8.1 (broad, N$\underline{H}$).

Example 38

p-Methoxycarbonylbenzyl (5R)-3-(2-acetamidoethylthio)-6-[(E, Z)-ethylidene]-7-oxo-1-azabicyclo [3.2.0]hept-2-ene-2-carboxylate

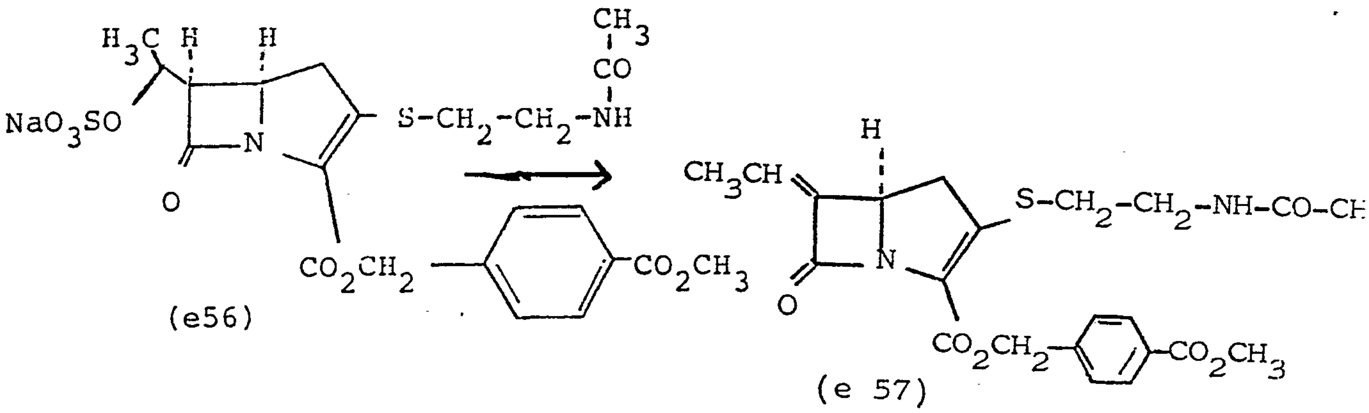

(e56) (e 57)

The mono-ester (e56) (164mg) in water (10ml) was shaken with benzyldimethyl-n-hexadecylammonium chloride (115mg) in $CH_2Cl_2$ (10ml). The organic layer was separated, dried ($MgSO_4$) and evaporated in vacuo to leave the quaternary ammonium salt. This was redissolved in $CH_2Cl_2$ (4ml), cooled in an ice bath and treated with 1,5-diazabicyclo [5.4.0]undec-5-ene (DBU) (90mg) in $CH_2Cl_2$ (1ml). The reaction mixture was stirred for 3h., then washed with water (10ml) and dilute brine (10ml). The organic layer was dried ($MgSO_4$) and evaporated to leave a gum, which was chromatographed on silica gel (5g, 230 - 400 mesh) eluting with $CHCl_3$ followed by $CHCl_3$/EtOH (95:5) to give p-methoxycarbonylbenzyl 3-(2-acetamidoethylthio)-6-[(E, Z)-ethylidene]-7-oxo-1-azabicyclo [3.2.0]hept-2-ene-2-carboxylate (e57) (22mg). νmax ($CH_2Cl_2$) 3450, 1767, 1720, 1687 $cm^{-1}$. δ($CDCl_3$) 1.82 and 2.05 (total 3H, each d, J ca.7.5Hz, $CH_3CH$, E and Z, ratio ca. 2:1), 1.94 (3H,s,$CH_3CO$), 2.6 - 3.6

($6H,m,SCH_2$, $CH_2NH$, 4-$CH_2$), 3.88 ($3H,s,CO_2CH_3$), 4.5 - 4.9 (1H,m,C5-H) 5.23 and 5.45 (2H, $AB_q$, J 15Hz, $CH_2Ar$), 5.95 and 6.41 (total 1H, each broad q, J ca 7.5Hz, $CH_3CH$, Z and E), 6.1 (1H, broad, NH), 7.55 and 8.01 (each 2H, d, J 8Hz, $C_6H_4$).

Example 39

p-Methoxycarbonylbenzyl (5R, 6S)-3-(2-acetamidoethylthio)-6-ethyl-7-oxo-1-azabicyclo[3.2.0]hept-2-ene-2-carboxylate

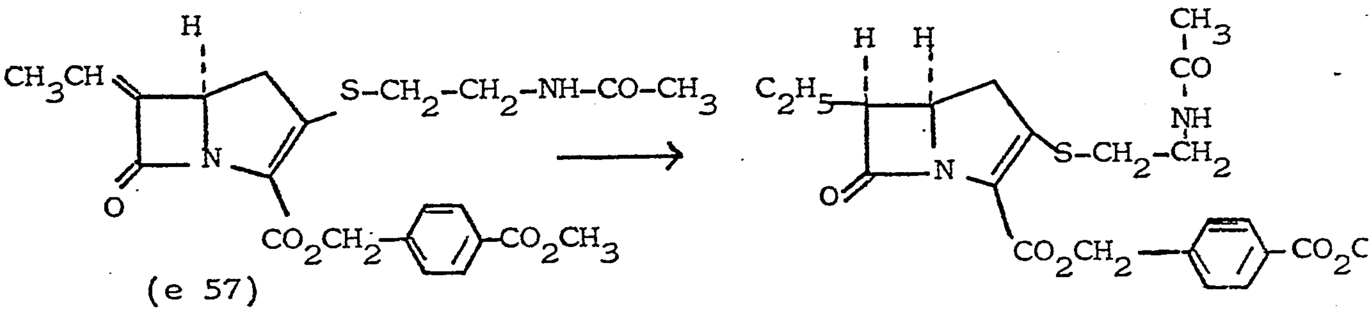

(e 57) (e 58)

p-Methoxycarbonylbenzyl-3-(2-acetamidoethylthio)-6-[(E,Z)-ethylidene]-7-oxo-1-azabicyclo[3.2.0]hept-2-ene-2-carboxylate (e57)(20 mg) in ethyl acetate (10 ml) was hydrogenated over $PtO_2$ (25 mg) for 18 h. at atmospheric pressure. The catalyst was filtered off and the filtrate was chromatographed on silica gel (5 g, 230 - 400 mesh), eluting with ethyl acetate/2% ethanol, and collecting 2 ml fractions. Fractions 31-39 were combined and evaporated in vacuo to yield p-methoxycarbonylbenzyl (5R,6S)-3-(2-acetamidoethylthio)-6-ethyl-7-oxo-1-azabicyclo [3.2.0]hept-2-ene-2-carboxylate (e58) (6.6 mg).

$\lambda$max (EtOH) 317 and 233 nm

$\nu$max ($CH_2Cl_2$) 3450, 1780, 1720, 1680 $cm^{-1}$

$\delta$ ($CDCl_3$) 1.05 (3H, t, J ca. 7 Hz, $CH_3CH_2$), 1.5 - 1.8 (2H, m, $CH_3CH_2$), 1.96 (3H, s, $CH_3CO$), 2.8 - 3.7 (7H, m, $SCH_2$, $CH_2NH$, 4-$CH_2$, C6-H) 3.90 (3H, s, $CO_2CH_3$), 4.0-4.5 (1H, m, C5-H), 5.16 and 5.42 (2H, $AB_q$, J ca. 14 Hz, $CH_2Ar$), 5.8 (1H, broad s, NH), 7.50 and 8.00 (each 2H, d, J 8 Hz, $C_6H_4$). Found: $M^+$ 446.1539; $C_{22}H_{26}N_2O_6S$ requires $M^+$ 446.1509.

Example 40

p-Methoxycarbonylbenzyl (5R,6R)-3-(2-acetamidoethylthio)-6-[(S)-1-ethoxysulphonyloxyethyl]-7-oxo-1-azabicyclo-[3.2.0]hept-2-ene-2-carboxylate

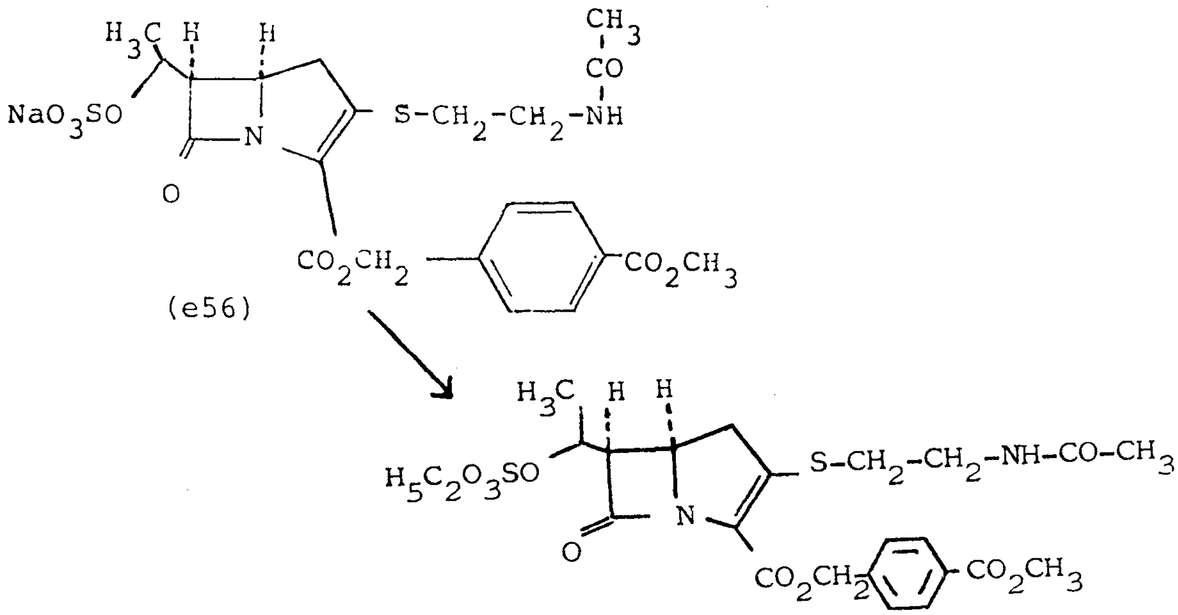

(e56)

(e 59)

Method I

Sodium (5R,6R)-3-(2-acetamidoethylthio)-6-[(S)-1-sulphonatooxyethyl]-7-oxo-2-p-methoxycarbonylbenzyloxycarbonyl-1-azabicyclo[3.2.0]hept-2-ene (e56) (50 mg) was suspended in dry $CH_2Cl_2$ (2 ml) and treated with $Et_3OBF_4$ in $CH_2Cl_2$ (100 mg/ml; 0.19 ml). The mixture was stirred for 0.75 h. then washed with water, dried ($MgSO_4$) and evaporated to yield p-methoxycarbonylbenzyl (5R,6R)-3-(2-acetamidoethylthio)-6-[(S)-1-ethoxysulphonyloxyethyl]-7-oxo-1-azabicyclo[3.2.0]hept-2-ene-2-carboxylate(e59).

Method II

Sodium (5R,6R)-3-(2-acetamidoethylthio)-6-[(S)-1-sulphonatooxyethyl]-7-oxo-2-p-methoxycarbonylbenzyloxycarbonyl-1-azabicyclo[3.2.0]hept-2-ene (e56) (50 mg) in $H_2O$ (5 ml) was shaken with $(n\text{-}C_{16}H_{33})(C_6H_5CH_2)(CH_3)_2N^+Cl^-$ (40 mg) in $CH_2Cl_2$ (10 ml). The organic layer was separated, dried ($MgSO_4$) and evaporated to yield the quaternary ammonium salt. This was taken up in dry $CH_2Cl_2$ and treated with $Et_3OBF_4$ in $CH_2Cl_2$ (100 mg/ml, 0.19 ml). After 0.5 h., a further 0.05 ml of $Et_3OBF_4$ solution was added. After 5 min., the solution was washed with water dried ($MgSO_4$) and evaporated to leave p-methoxycarbonylbenzyl (5R,6R)-3-(2-acetamidoethylthio)-6-[(S)-1-ethoxysulphonyloxyethyl]-7-oxo-1-azabicyclo[3.2.0]hept-2-ene-2-carboxylate (e 59), contaminated with the quaternary ammonium tetrafluoroborate.

Example 41

p-Methoxycarbonylbenzyl (5R)-3-(2-acetamidoethylthio)-6-[(E)-ethylidene)-7-oxo-1-azabicyclo[3.2.0]hept-2-ene-2-carboxylate

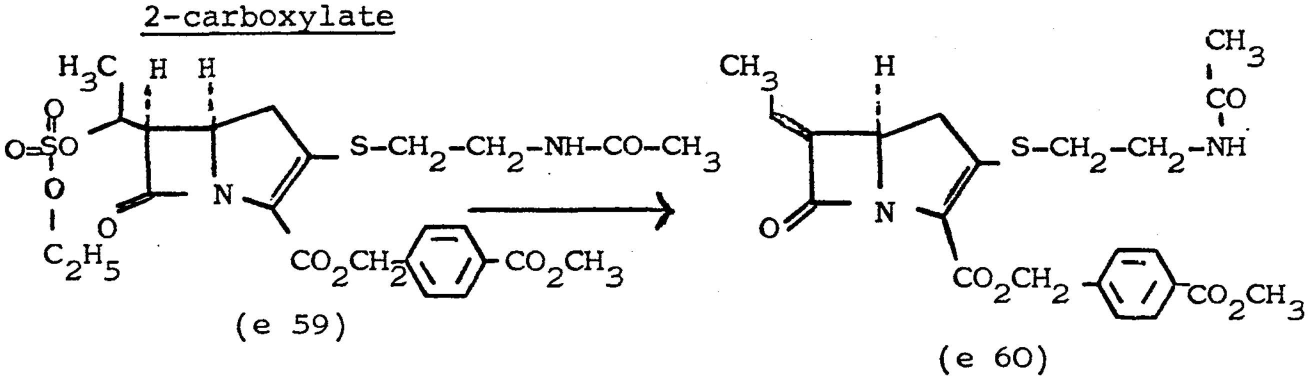

(e 59) (e 60)

p-Methoxycarbonylbenzyl (5R,6R)-3-(2-acetamidoethylthio)-6-[(S)-1-ethoxysulphonyloxyethyl]-7-oxo-1-azabicyclo[3.2.0]hept-2-ene-2-carboxylate from Example 40, Method II, was taken up in DMF (2 ml) and treated with powdered $K_2CO_3$ (50 mg). The mixture was stirred for 1.5 h, then the solvent was evaporated in vacuo and the residue was dissolved in ethyl acetate (10 ml) and water (10 ml) and shaken. The ethyl acetate layer was separated, washed with water (3 x 10 ml) then brine, dried ($MgSO_4$) and evaporated in vacuo. The residue was chromatographed on silica gel (3g, 230 - 400 mesh), eluting with ethyl acetate/4% ethanol, to give, after evaporation of the solvent, p-methoxycarbonylbenzyl (5R)-3-(2-acetamidoethylthio)-6-[(E)-ethylidene]-7-oxo-1-azabicyclo[3.2.0]hept-2-ene-2-carboxylate (e60) (8.2 mg). $\delta$ ($CDCl_3$) 1.89 (3H, d, J 7Hz, $C\underline{H}_3CH$), 1.95 (3H, s, $C\underline{H}_3CO$), 2.7 - 3.7 (6H, m, $SC\underline{H}_2$, $C\underline{H}_2NH$, 4-$C\underline{H}_2$ ),3.90 (3H, s, $CO_2C\underline{H}_3$), 4.75 (1H, broad t, J ca. 9Hz, 5-$C\underline{H}$), 5.22 and 5.43 (2H, ABq, J ca. 14 Hz, $C\underline{H}_2Ar$), 5.8 (1H, broad, N$\underline{H}$), 6.40 (1H, dq, J ca. 1 and 7 Hz, $CH_3C\underline{H}$) 7.52 and 8.00 (each 2H, d, J 8Hz, $C_6\underline{H}_4$).

Example 42

Benzyl (5R)-3-[(Z)-2-acetamidoethenylthio]-6-[(EZ)-ethylidene]-7-oxo-1-azabicyclo[3.2.0]hept-2-ene-2-carboxylate

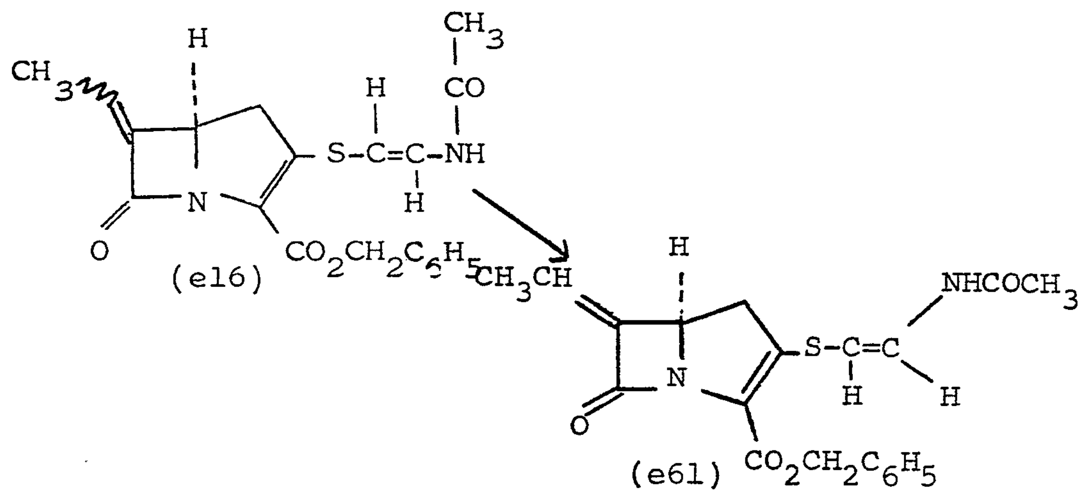

(e16) (e61)

A solution of benzyl (5R)-3-[(E)-2-acetamidoethenylthio -6-ethylidene-7-oxo-1-azabicyclo[3.2.0]hept-2-ene-2-carboxylate [40 mg of an approximately 1:1 mixture of (E)- and (Z)-ethylidene isomers] in 20% aqueous acetonitrile (0.5 ml) was stirred with mercuric chloride (27 mg) at room temperature for 5 min. The solution was diluted with ethyl acetate and the organic phase was washed twice with aqueous sodium bicarbonate solution and brine. The solution was dried ($MgSO_4$) and concentrated in vacuo. The residue was chromatographed on silica gel, eluting with petroleum ether (60-80$^o$) / ethyl acetate (1:4) to afford benzyl (5R)-3-[(Z)-2-acetamidoethenylthio]-6-[(EZ)-ethylidene]-7-oxo-1-azabicyclo[3.2.0]hept-2-ene-2-carboxylate (e61) (approximately 1:1 mixture of isomers) (15 mg) as a gum; $\lambda$max. (EtOH) 312 and 223 nm; $\nu$max ($CHCl_3$) 3410, 1770, 1700 and 1630 $cm^{-1}$; $\delta$ ($CDCl_3$) 1.78 and 2.03 [3H, each d, J 7 Hz, C$\underline{H}_3$CH for (6E) and (6Z)-isomers, respectively], 2.07 (3H, s, C$\underline{H}_3$CO) 2.75 - 3.30 (2H, m, 4-$CH_2$), 4.50 -

4.80 (1H, m, 5-CH), 5.05 - 5.47 (3H, m, $CH_2$ Ph and SCH=), 5.91 and 6.38 [1H, each dq, *J* *ca* 1 and 7 Hz, CH=$CH_3$ for (6Z) - and (6E) - isomers, respectively], 7.15 - 7.6 (6H, m, $C_6H_5$ and =CHN) and 7.94 (1H, br, d, NH).

Example 43

Benzyl (5R,6R)-3-[(Z)-2-acetamidoethenylthio]-6-ethyl-7-oxo-1-azabicyclo[3.2.0]hept-2-ene-2-carboxylate

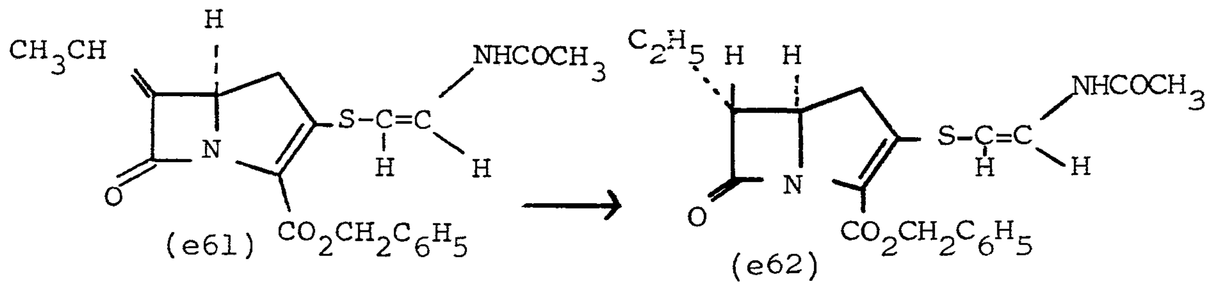

(e61) (e62)

To a solution of benzyl (5R)-3-[(Z)-2-acetamidoethenylthio]-6-[(EZ)-ethylidene]-7-oxo-1-azabicyclo [3.2.0]hept-2-ene-2-carboxylate (e 61) (25mg) in THF (1ml) at $-5^{\circ}$ was added a solution of sodium borohydride (9 mg) in aqueous pH 7 buffer solution (0.1 ml). The mixture was allowed to warm to $5^{\circ}C$ and then was left to stand for 0.5 h. Ethyl acetate (20 ml) was added and the organic solution was washed with water and brine. Evaporation of the dried ($MgSO_4$) solution gave a residue which was chromatographed on silica gel using petroleum ether ($60 - 80^{\circ}$) / ethyl acetate (2:3) to elute. The first eluted component was benzyl (5R,6R)-3-[(Z)-2-acetamido-ethenylthio]-6-ethyl-7-oxo- 1-azabicyclo[3.2.0]hept-2-ene-2-carboxylate (e62) (3mg); λmax. (EtOH) 324 nm; ν max. ($CHCl_3$) 3410, 1775, 1700 and 1630 cm.

Example 44

Sodium (5R,6S)-3-(2-acetamidoethylthio)-6-ethyl-7-oxo-1-azabicyclo[3.2.0]hept-2-ene-2-carboxylate

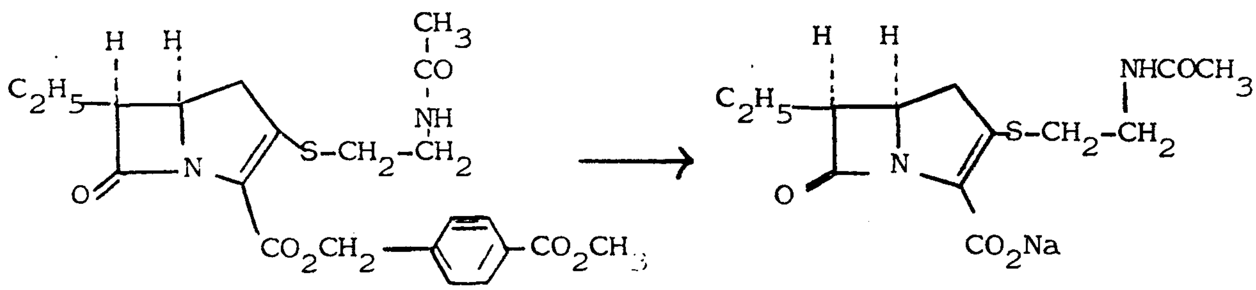

(e 58) (e63)

p-Methoxycarbonylbenzyl (5R,6S)-3-(2-acetamidoethylthio)-6-ethyl-7-oxo-1-azabicyclo[3.2.0]hept-2-ene-2-carboxylate (e58) (18mg) was added to the cathodic compartment of an H-type divided two-compartment electrochemical cell, with a mercury cathode and a platinum anode. The cell contained de-gassed 0.1M n-$Bu_4NI$ in dimethylformamide as electrolyte. The volume of the catholyte was about 15 ml [glacial acetic acid (about 10 mg) added] and the volume of the anolyte was about 8 ml. A potentiostat was employed to fix the potential of the cathode at -1.9V with respect to the standard calomel electrode, and the initial current, about 56 mA, fell to about 15 mA after about 15 min. The catholyte was removed from the cell and the solvent was evaporated in vacuo. The residue was taken up in $CH_2Cl_2$ (10 ml) and washed with water (10 ml). The aqueous phase was separated and washed with $CH_2Cl_2$. The aqueous phase, containing the tetrabutylammonium salt corresponding to (e63) was passed through an Amberlite IR 120 (Na form) column (17 x 1 cm) and the eluate was reduced to low volume by

evaporation in vacuo. The residue was passed through a Biogel P-2 column, to yield fractions containing sodium (5R,6S)-3-(2-acetamidoethylthio)-6-ethyl-7-oxo-1-azabicyclo[3.2.0]hept-2-ene-2-carboxylate (e63) having λ max 298 nm.

The minimum inhibitory concentrations (MIC) of this compound was determined using a standard DST agar + 10% horse blood substrate.

The results are shown below.

| ORGANISM | MIC (μg/ml) |
|---|---|
| *Citrobacter freundii* E8 | 2.5 |
| *Enterobactor cloacae* N1 | 20 |
| *Escherichia coli* 0111 | 2.5 |
| *Escherichia coli* JT 39 | 10 |
| *Klebsiella aerogenes* A | 1.2 |
| *Proteus mirabilis* C977 | 10 |
| *Proteus morganii* I580 | 40 |
| *Proteus rettgeri* WM16 | 10 |
| *Proteus vulgaris* W091 | 5.0 |
| *Pseudomonas aeruginosa* A | >40 |
| *Salmonella typhimurium* CT10 | 2.5 |
| *Serratia marcescens* US20 | 10 |
| *Shigella sonnei* MB 11967 | 2.5 |
| *Bacillus subtilis* A | - |
| *Staphylococcus aureus* Oxford | 0.6 |
| *Staphylococcus aureus* Russell | 0.6 |
| *Staphylococcus aureus* 1517 | 10 |
| *Streptococcus faecalis* I | 40 |
| *Streptococcus pneumoniae* CN33 | NG |
| *Streptococcus pyogenes* CN10 | 0.1 |
| *E. coli* ESS | 1.2 |

Example 45

tert-Butyldiphenylsilyl (5R,6S)-3-[(E)-2-acetamidoethenyl thio]-6-[(1S)-1-hydroxyethyl]-7-oxo-1-azabicyclo[3.2.0] hept-2-ene-2-carboxylate

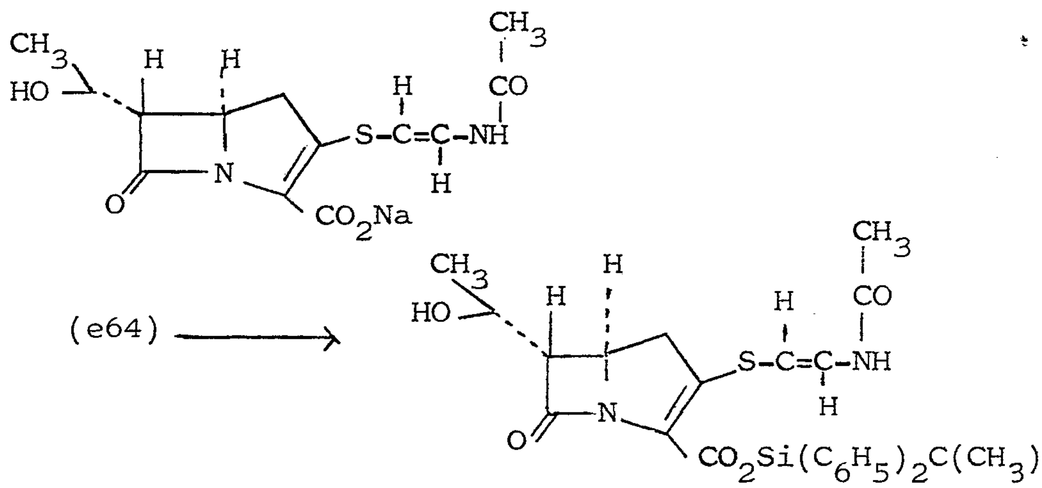

(e64)

(e65)

Method I

Sodium (5R,6S)-3-[(E)-2-acetamidoethenylthio]-6-[( S)-1-hydroxyethyl]-7-oxo-1-azabicyclo[3.2.0]hept-2-ene-2-carboxylate (e64) (50mg, 50% pure material) in dry tetrahydrofuran (THF) (5ml) containing 15-crown-5 crown ether (2 drops) and 3Å molecular sieves was treated with tert-butylchlorodiphenylsilane (80mg). After stirring at r.t. for 20hr., the THF was evaporated in vacuo and the mixture loaded onto a silica gel column (10g) and the column eluted with chloroform (10ml) followed by a chloroform/ethanol mixture (4:1). The fractions containing the ester were combined and evaporated to give tert-Butyldiphenylsilyl (5R,6S)-3-[(E)-2-acetamidoethenylthio]-6-[( S)-1-hydroxyethyl]-7-oxo-1-azabicyclo[3.2.0]hept-2-ene-2-carboxylate (e65) as an oil (20mg), $\nu_{max}$ ($CH_2Cl_2$) 1785, 1700, 1685, 1625 $cm^{-1}$. $\delta$($CDCl_3$) 1.13 (9H,s,$C(C\underline{H}_3)_3$), 1.27 (3H,d,J 6Hz, $C\underline{H}_3CH$), 1.77 (3H,s,$C\underline{H}_3CO$), 2.7 - 3.2 (2H,m, 4-$C\underline{H}_2$), 3.22 (1H,

m, sharpens to dd on $D_2O$ exchange, $\underline{J}_1$ approx. 5Hz, $J_2$ approx. 3Hz, 6-C$\underline{H}$), 3.6 (1H, broad, exchanges $D_2O$, O$\underline{H}$), 3.9 - 4.3 (2H,m, 5-C$\underline{H}$, 8-C$\underline{H}$), 5.60 (1H,d,$\underline{J}$ 14Hz, S C$\underline{H}$=), 7.02 (1H,dd,$\underline{J}_1$ 14Hz, $\underline{J}_2$ 10Hz,d,$\underline{J}$ 14Hz on $D_2O$ exchange, CH=C$\underline{H}$ NH), 7.3 - 7.8 (10H, Ar-$\underline{H}$), 8.34 (1H, broad d, exchange $D_2O$, N$\underline{H}$) p.p.m. The UV spectrum showed a maximum at about 328nm.

Method II

The sodium salt (e64) (50mg; 50% pure) in N,N-dimethylformamide (DMF) (2ml) was stirred with 3Å molecular sieves (200mg) for 3/4 hr. tert-Butyl chlorodiphenylsilane (50mg) was then added and the mixture was stirred for 15 min. Most of the DMF was then removed by evaporation in vacuo. Ethyl acetate was added, the mixture was filtered through celite and the resultant solution was washed with dilute aqueous $NaHCO_3$ followed by water. The ethyl acetate was dried ($MgSO_4$), evaporated in vacuo to give the crude ester. Chromatography on silica gel yielded pure tert-Butyldiphenylsilyl (5R,6S)-3-[(E)-2-acetamidoethenylthio]-6-[(1S)-1-hydroxyethyl]-7-oxo-1-azabicyclo[3.2.0]hept-2-ene-2-carboxylate (e65).

Method III

The sodium salt (e64) (50mg, 50% pure) in acetonitrile(3ml) was stirred with 3Å molecular sieves (150mg) and 15-crown-5 crown ether (2 drops) for 1hr. tert-Butylchorodiphenylsilane (about 40 mg) was added and stirring was continued for 1hr. The reaction mixture was worked up as in method I to give tert-Butyldiphenylsilyl (5R,6S )-3-[(E)-2-acetamidoethenylthio]-6-[(1S)-1-hydroxyethyl]-7-oxo-1-azabicyclo[3.2.0]hept-2-ene-2-carboxylate (e65) (13mg).

Example 46

tert-Butyldiphenylsilyl (5R,6S)-3-[(E)-2-acetamidoethenyl thio]-6-[(1S)-1-methylsulphonyloxyethyl]-7-oxo-1-azabicyclo[3.2.0]hept-2-ene-2-carboxylate

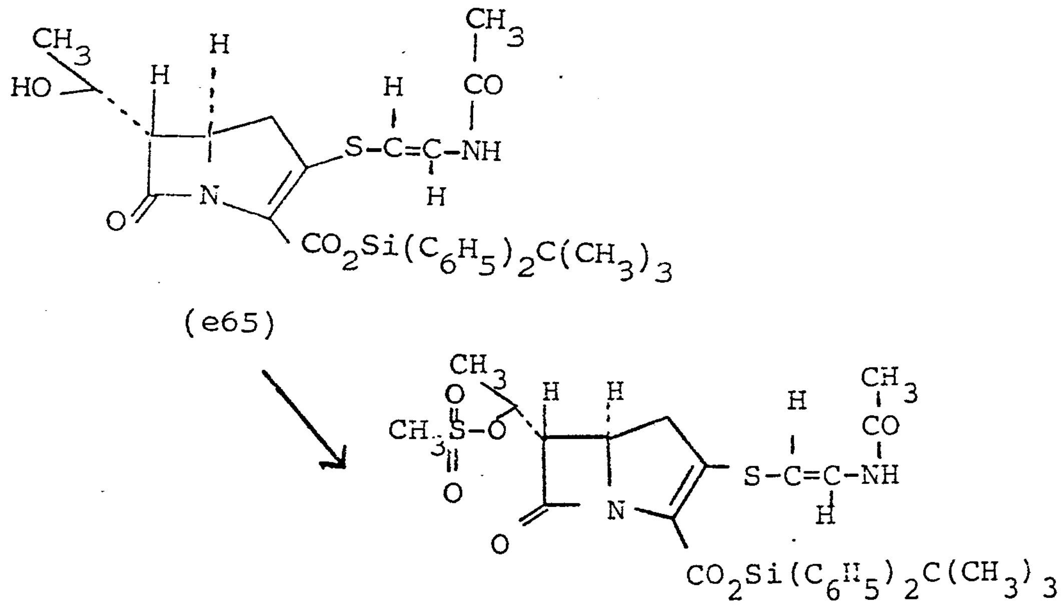

(e65)

(e66)

tert-Butyldiphenylsilyl (5R,6S)-3-[(E)-2-acetamidoethenyl-thio]-6-[(1S)-1-hydroxyethyl]-7-oxo-1-azabicyclo[3.2.0] hept-2-ene-2-carboxylate (e65) (45mg) in $CH_2Cl_2$ (1ml) was cooled to -20°C and treated with a solution of triethylamine in $CH_2Cl_2$ (0.13ml, 100mg/ml) followed by a solution of methane sulphonyl chloride in $CH_2Cl_2$ (0.11ml, 100mg/ml). The mixture was stirred whilst maintaining the temperature between -20°C and 0°C for 6 hr. The reaction mixture was then diluted with $CH_2Cl_2$, washed with water and then with brine, dried ($MgSO_4$) and evaporated in vacuo. The residue was chromatographed on a silica gel column (3-4g, 230 - 400 mesh ASTM) to give tert- Butyldiphenylsilyl (5R,6S)-3-[(E)-2-acetamidoethenylthio]-6-[(1S)-1-methylsulphonyloxy-ethyl]-7-oxo-1-azabicyclo[3.2.0]hept-2-ene-2-carboxylate

(e66) (20mg), νmax 3440, 1785, 1700, 1685(sh), 1625 $cm^{-1}$. δ($CDCl_3$) 1.12 [9H,s,$C(CH_3)_3$], 1.54 (3H,d,J Ca 6Hz, $CH_3CH$), 1.84 (3H,s,$COCH_3$), 3.00 (5H,s superposed on m, $CH_3SO_3$, 4-$CH_2$), 3.4 - 3.6 (1H,m,dd,J 3 and 5Hz on $D_2O$ exchange, 6-CH), 4.0 - 4.3 (1H,m,5-CH), 4.9 - 5.3 (1H,m,8-CH), 5.64 (1H,d,J 14Hz, SCH=CH), 7.08 (1H,dd,J 14 and 10Hz, d, J 14Hz on $D_2O$ exchange, CH NH), 7.2 - 7.8 (10H,m, $(C_6H_5)_2$), 7.90 (1H,d,J 10Hz, exchanges $D_2O$, NH)ppm.

Example 47

tert-Butyldiphenylsilyl (5R)-3-[(E)-2-acetamidoethenylthio]-6-[(Z)-ethylidene]-7-oxo-1-azabicyclo[3.2.0]hept-2-ene-2-carboxylate

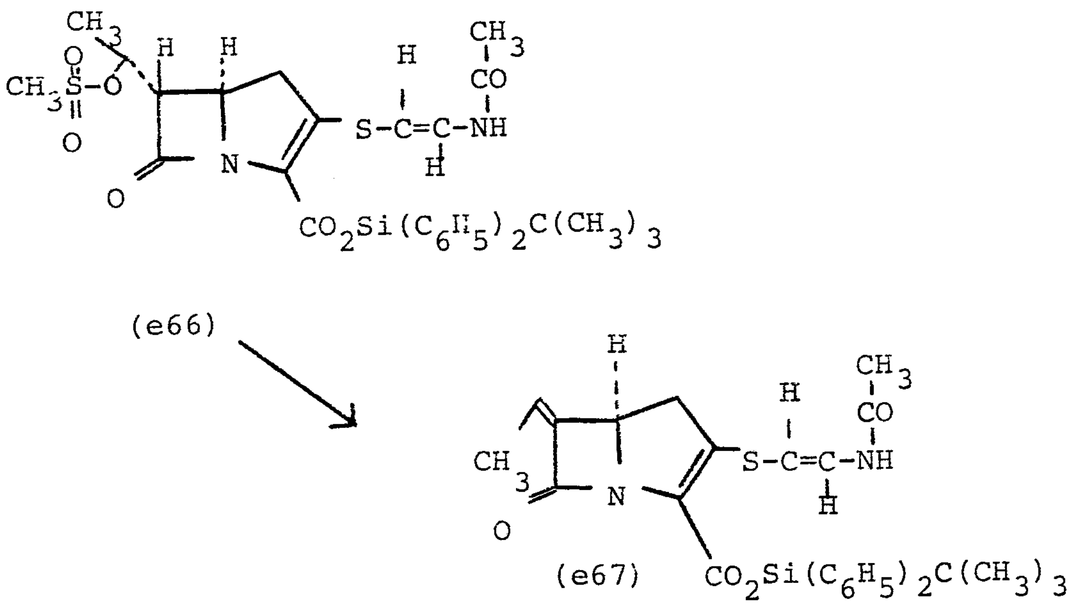

(e66)

(e67)

Method I

tert-Butyldiphenylsilyl (5R,6S)-3-[(E)-2-acetamidoethenylthio]-6-[(1S)-1-methylsulphonyloxyethyl]-7-oxo-1-azabicyclo[3.2.0]hept-2-ene-2-carboxylate (20 mg) in dichloromethane (1 ml) was treated with 1,5-diazabicyclo [5.4.0]undec-5-ene (0.05 ml of a 100 mg/ml solution in $CH_2Cl_2$) at room temperature for 20 min. The dichloromethane solution was diluted, washed with water, dried ($MgSO_4$) and evaporated in vacuo to give tert-Butyldiphenylsilyl (5R)-3-[(E)-2-acetamidoethenylthio]-6-[(Z)-ethylidene]-7-oxo-1-azabicyclo[3.2.0]hept-2-ene-2-carboxylate (e67) (6.8 mg). νmax 1770, 1700, 1675, 1640, 1620 $cm^{-1}$. δ($CDCl_3$+$D_2O$) 1.11[9H,s,C($C\underline{H}_3$)$_3$], 1.85 (3H,s,$C\underline{H}_3CO$), 2.05 (3H,d,J approx. 7Hz,$C\underline{H}_3CH$), 3.7 - 3.3 (2H,m, 4-$C\underline{H}_2$), 4.4 - 4.7 (1H,m, 5-C$\underline{H}$), 5.65 (1H,d,J 14Hz, SC$\underline{H}$=CH), 5.83 (1H, broadened q, J ca 7Hz, $CH_3C\underline{H}$), 7.00 (1H,d,J 14Hz C$\underline{H}$NH), 7.2 - 7.8 (10H,m,Ar-H).

Method 2

The mesylate (e66) (150 mg) was prepared from the hydroxy compound (290 mg) as described in Example 46 and was converted to the ethylidene compound as in Method I. Chromatography on silica gel (8 g 230 - 400 mesh ASTM) using gradient elution from 50% ethyl acetate/cyclohexane to neat ethyl acetate gave the ethylidene compound (e67) (21 mg).

Example 48

Tert-Butyldiphenylsilyl (5R,6R)-3-[(E)-2-acetamidoethenylthio]-6-[sodium (S)-1-sulphonatooxyethyl]-7-oxo-1-azabicyclo[3.2.0]-hept-2-ene-2-carboxylate

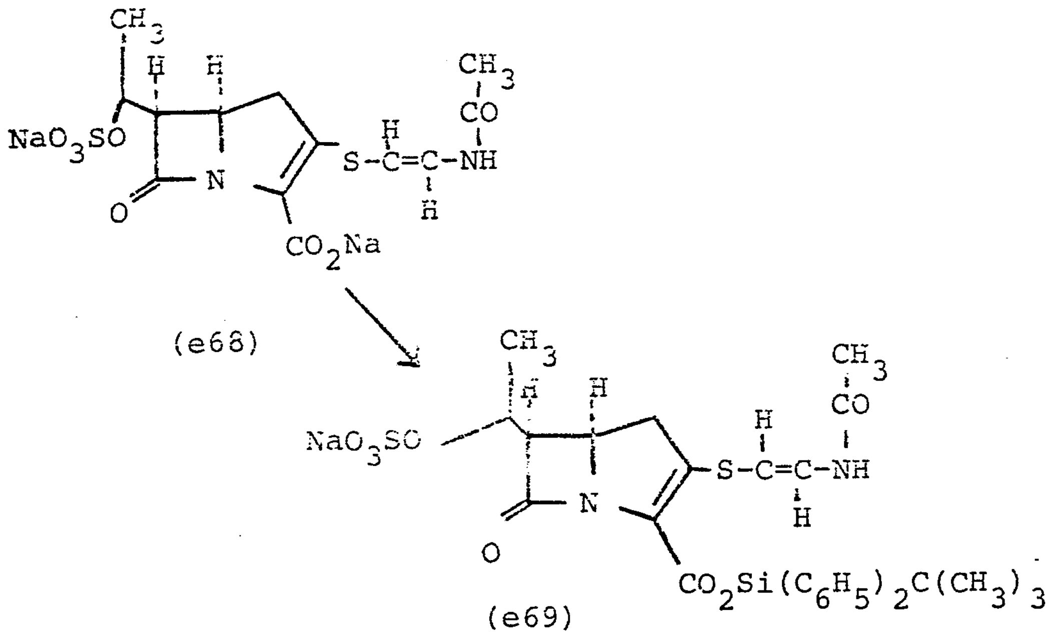

(e68)

(e69)

The disodium salt (e68) (200 mg) in N,N-dimethylformamide (DMF) (2 ml) was treated with tert-butylchlorodiphenylsilane (133 mg) in DMF (1 ml). After 30 min, the DMF was evaporated in vacuo and the residue was loaded onto a silica gel column (18 g) and eluted with $CHCl_3$/$C_2H_5OH$ (3:2). Relevant fractions were combined and evaporate in vacuo, toluene was added to the residue and evaporated to leave tert- butyldiphenylsilyl (5R, 6R)-3-[(E)-2-acetamidoethenylthio]-6-[sodium (S)-1-sulphonatoxyethyl]-7-oxo-1-azabicyclo[3.2.0]-hept-2-ene-2-carboxylate (e69) as a solid (40 mg). νmax (KBr) 1770, 1670, 1520 $cm^{-1}$. λmax (EtOH) 328 (13,200), 218 (26,000)nm.

Example 49

(5R,6R)-3-[(E)-2-acetamidoethenylthio]-6-[(1S)-1-hydroxysulphonyloxyethyl]-2-p-methoxycarbonylbenzyloxycarbonyl-7-oxo-1-azabicyclo-[3.2.0]hept-2-ene sodium salt

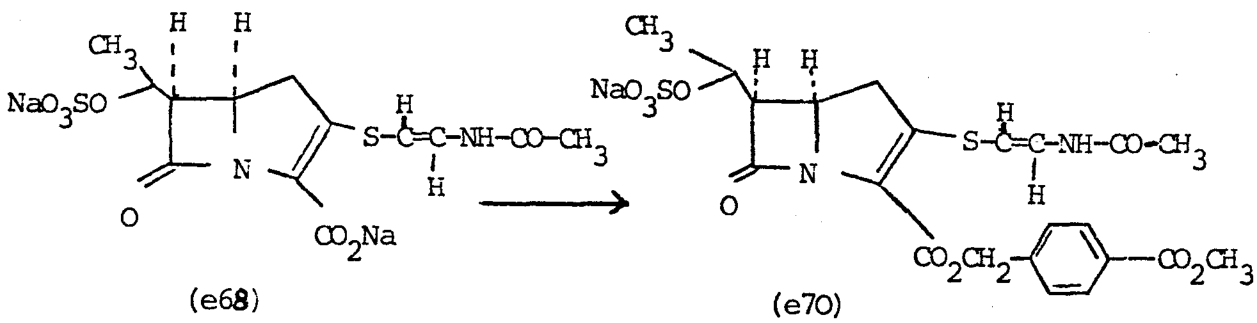

(e68) (e70)

The disodium salt (e68) (3.50 g) in N,N-dimethylformamide (DMF) (30 ml) was treated with methyl 4-bromomethylbenzoate (5.5 g; 3 eq.). After 2½ hr the DMF was removed in vacuo and the residue suspended in chloroform (100 ml) and loaded onto a silica gel column (90 g). The column was then eluted with chloroform/ethanol mixtures ; - 65:35 (400 ml); 60:40 (400 ml) and finally 40:60. Fractions containing the ester were combined and the solvents removed in vacuo to give (5R, 6R)-3-[(E)-2-acetamidoethenylthio]-6-[(1S)-1-hydroxysulphonyloxyethyl]-2-p-methoxycarbonylbenzyloxycarbonyl-7-oxo-1-azabicyclo-[3.2.0]hept-2-ene sodium salt (e70) (2.5 g); νmax (KBr) 1770, 1715-1690, 1620, 1280 $cm^{-1}$. The ultraviolet absorption spectrum showed maxima at 323 and 233 nm.

Example 50

p-Methoxycarbonylbenzyl-3-[(E)-2-acetamidoethenylthio]-6-(EZ)ethylidene-7-oxo-1-azabicyclo[3.2.0]hept-2-ene-2-carboxylate

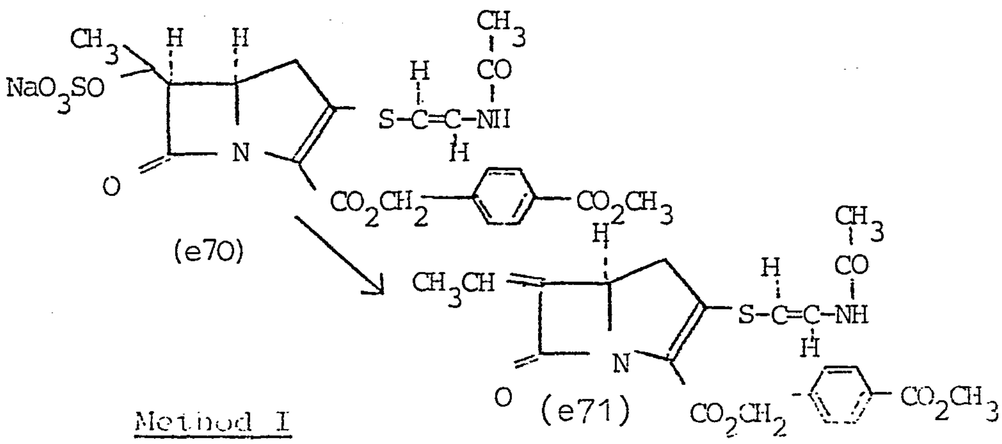

(e70)

(e71)

Method I

The sodium salt of (5R,6R)-3-[(E)-2-acetamidoethenylthio]-6-[(1S)-1-hydroxysulphonyloxyethyl]-2-p-methoxycarbonylbenzyloxycarbonyl-7-oxo-1-azabicyclo[3.2.0]hept-2-ene (e70) (340 mg) in DMF (6ml) and THF (6ml) was cooled to about 4° (ice bath), 3Å molecular sieves (about 300 mg) were added and the mixture was stirred for 15 min. Potassium hydride 24% dispersion in oil, 109 mg) in suspension in THF (2ml) was added and the mixture stirred for a further 12 mins. Ethyl acetate and dilute brine were added and the layers were separated. The ethyl acetate layer was washed with water (3 x) and then with brine, and then dried ($MgSO_4$) and evaporated in vacuo. The residue was chromatographed on silica gel (18 g) eluting with dichloromethane (10 ml) then with ethyl acetate (100 ml) and then with ethyl acetate containing 4% of ethanol to give p-methoxycarbonylbenzyl 3-[(E)-2-acetamidoethenylthio]-6-(E,Z)ethylidene-7-oxo-1-azabicyclo[3.2.0]hept-2-ene-2-carboxylate (e71) (17 mg), $\lambda$max (EtOH) 310 (12,900) 231 (36,800) nm. $\nu$max($CH_2Cl_2$) 1765, 1715, 1700, 1620 $cm^{-1}$. ($M^+$ m/e 442.1185; $C_{22}H_{22}N_2O_6S$ requires m/e 442.1196). The n.m.r. spectrum indicated that the product was a mixture of E and Z ethylidene isomers.

Method 2

The ester (e70) (560 mg) in dry DMF (10 ml) and dry THF (10 ml) was cooled (icebath) and stirred with 3A molecular sieves. The cooled mixture was then treated with sodium hydride (38 mg, 50% dispersion in oil) suspended in dry THF (2 ml). The mixture was stirred in the cold for 1 hr. and then at room temperature for 2.75 hr. Ethyl acetate (about 150 ml) and saturated brine (50 ml) and water (50 ml) were added and the mixture was worked up by a similar route to that in method I to give the ethylidene derivative (e71) (71 mg).

Method 3

The ester (e70) (100 mg) in water (10 ml) was treated with one equivalent of benzyldimethyl-n-hexadecylammonium chloride in dichloromethane (10 ml). After shaking, the $CH_2Cl_2$ layer was separated and dried ($MgSO_4$) and evaporated to dryness to give the monoquaternary ammonium salt of the ester. This was taken up in dichloromethane (4 ml) and treated with tetramethylguanidine in $CH_2Cl_2$ (0.2 ml, 100mg/ml solution). The mixture was stirred in the cold (icebath) for 15 min and then at r.t. After 2.5 hr, a further aliquot of the tetramethylguanidine solution (0.2 ml) was added and stirring was continued for a further 1 hr. The reaction mixture was worked up by washing with water, drying the $CH_2Cl_2$ solution ($MgSO_4$), evaporation and chromatography as in method I to give the ethylidene compound (e 71) (18 mg).

Method 4

The ester (e70) (500 mg) in DMF (7 ml) was warmed to 70°C in the presence of powdered $K_2CO_3$ (750 mg). After 4 hr., t.l.c. indicated that the reaction had proceeded almost to completion. Ethyl acetate (50 ml) and water (20 ml) were added, and after separation, the aqueous layer was re-extracted with ethyl acetate. The ethyl acetate layers were combined, washed with water (3 x 20 ml) brine (10 ml), dried ($MgSO_4$) and evaporated. The residue was chromatographed on silica gel in a similar way to that described in Method I to give the ethylidene compound (e71) (67 mg).

Example 51

p-Methoxycarbonylbenzyl (5R,6S)-3-[(E)-2-acetamidoethenylthio]-6-ethyl-7-oxo-1-azabicyclo[3.2.0]hept-2-ene-2-carboxylate

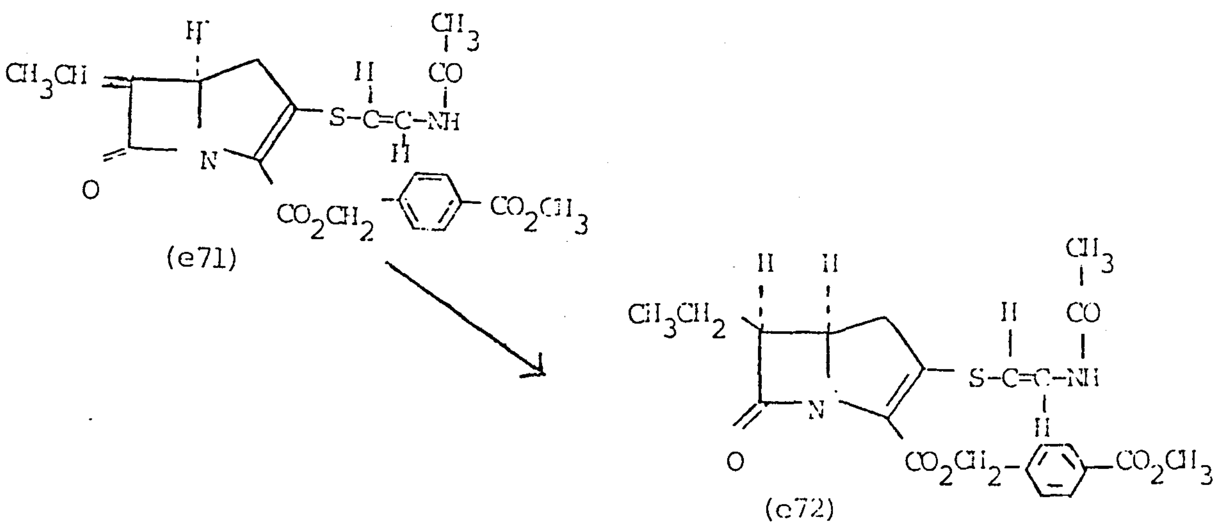

(e71) (c72)

p-Methoxycarbonylbenzyl (5R)-3-[(E)-2-actamidoethenylthio]-6-(EZ)ethylidene-7-oxo-1-azabicyclo[3.2.0]hept-2-ene carboxylate (e71) (107 mg) in ethyl acetate (25 ml) was hydrogenated at atmospheric pressure over $PtO_2$(150 mg) for 3 hr. More $PtO_2$(60 mg) was then added and hydrogenation continued for a further 18 hr. The catalyst was filtered off, the ethyl acetate was evaporated and the residue was chromatographed on silica gel (6 g) eluting with ethyl acetate to give p-Methoxycarbonylbenzyl (5R,6S)-3-[(E)-2-acetamidoethenylthio]-6-ethyl-7-oxo-1-azabicyclo[3.2.0]hept-2-ene-2-carboxylate (e72) (13.8 mg). νmax ($CH_2Cl_2$)1787, 1720, 1710(sh), 1625 $cm^{-1}$. δ($CDCl_3$) 0.98 (3H,t,J 7Hz, $C\underline{H}_3CH_2$), 1.5 - 1.9 (2H,m,$CH_3C\underline{H}_2CH$), 2.03 (3H,s,$C\underline{H}_3CO$), 2.90 (2H,d,J 9Hz, 4-$CH_2$), 3.4 - 3.7 (1H,m,6-C$\underline{H}$), 3.87 (3H,s,$C\underline{H}_3O$), 4.0 - 4.4 (1H,d t,J 6 and 9Hz, 5-C$\underline{H}$), 5.20 and 5.40 (2H, ABq., J 16Hz, $CO_2C\underline{H}_2$), 5.88 (1H,d,J 15Hz, S.C$\underline{H}$=CH), 7.21 (1H,dd,J 15 and 10Hz, CH=C$\underline{H}$.NH), 7.49 and 8.00 (each 2H,d, J 8Hz, $C_6\underline{H}_4$), 7.86 (1H, broad d, J 10Hz, CH.N$\underline{H}$). The U.V. spectrum showed

peaks at 325 and 236 nm. The n.m.r spectrum indicated that a trace of the 5R,6R isomer was also present.

Example 52

Sodium (5R,6S)-3-[(E)-2-acetamidoethenylthio]-6-ethyl-7-oxo-1-azabicyclo[3.2.0]hept-2-ene-2-carboxylate

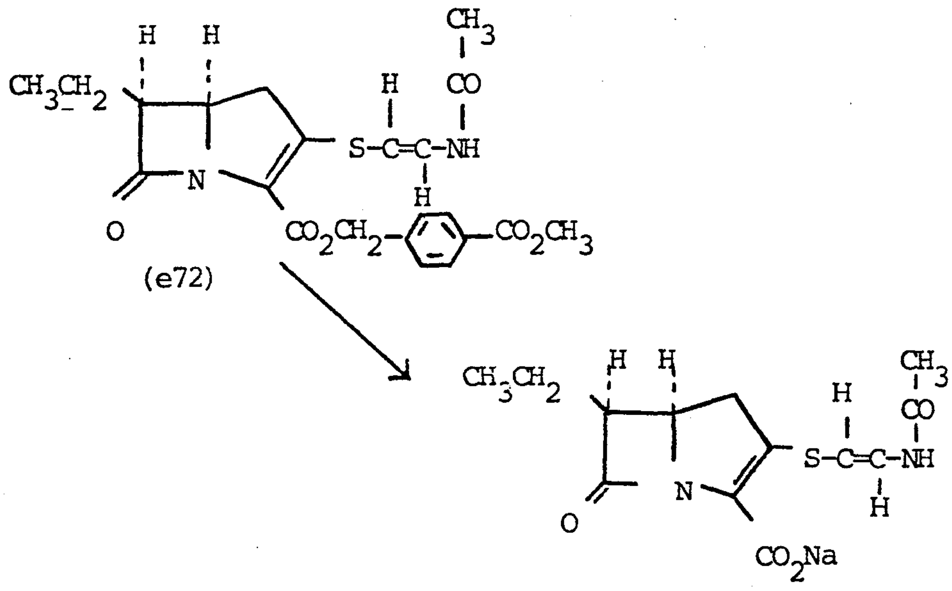
(e72)

(e73)

p-Methoxycarbonylbenzyl (5R,6S)-3-[(E)-2-acetamidoethenyl-thio]-6-ethyl-7-oxo-1-azabicyclo[3.2.0]hept-2-ene-2-carboxylate (e 72) (64 mg) in solution in 0.1M $(n\text{-}C_4H_9)_4NBF_4$ in dry DMF was added to the cathodic compartment of a divided cylindrical electrochemical cell, with a mercury cathode and platinum anode. The volume of electrolyte in the cathodic compartment was about 25 ml, and in the anodic compartment about 10 ml. Acetic acid (12 mg) was added to the cathodic compartment and ethanol (250 mg) was added to the anodic compartment. The cell was degassed by passing argon for 30 min. The potentiostat was engaged to maintain the potential of the cathode at -1.9V w.r.t. standard calomel electrode. The initial current (40 mA) fell to about 5 mA after 25 min. The solvent was removed from the catholyte by evaporation in vacuo and the residual oil was taken up in $CH_2Cl_2$ (20 ml) and extracted with water (15 ml) containing sodium tetrafluoroborate (100 mg). The aqueous extract

was washed with $CH_2Cl_2$ and then passed down an Amberlite 1R·120 column (1 x 20 cm) in the sodium form. The volume of the eluate was reduced to about 5 ml by evaporation in vacuo and loaded onto a Biogel P-2 column (18 x 3 cm). Elution with water yielded fractions containing sodium (5R,6S)-3-[(E)-2-acetamidoethenylthio]-6-ethyl-7-oxo-1-azabicyclo[3.2.0]hept-2-ene-2-carboxylate (e 73) λmax 308 nm.

The minimum inhibitory concentrations (MIC) of this compound was determined using a standard DST agar + 10% horse blood substrate.

The results are shown below.

| ORGANISM | MIC (μg/ml) |
|---|---|
| *Citrobacter freundii* E8 | 2.5 |
| *Enterobactor cloacae* N1 | 2.5 |
| *Escherichia coli* 0111 | 2.5 |
| *Escherichia coli* JT 39 | 10 |
| *Klebsiella aerogenes* A | 2.5 |
| *Proteus mirabilis* C977 | 10 |
| *Proteus morganii* I580 | 5.0 |
| *Proteus rettgeri* WM16 | 10 |
| *Proteus vulgaris* W091 | 10 |
| *Pseudomonas aeruginosa* A | >40 |
| *Salmonella typhimurium* CT10 | 1.2 |
| *Serratia marcescens* US20 | 5.0 |
| *Shigella sonnei* MB 11967 | 2.5 |
| *Bacillus subtilis* A | 1.2 |
| *Staphylococcus aureus* Oxford | 0.6 |
| *Staphylococcus aureus* Russell | 2.5 |
| *Staphylococcus aureus* 1517 | >40 |
| *Streptococcus faecalis* I | 40 |
| *Streptococcus pneumoniae* CN33 | ≤ 0.1 |
| *Streptococcus pyogenes* CN10 | NG |
| *E. coli* ESS | 0.3 |

Example 53

Cleavage of the tert-Butyldiphenylsilyl Ester Grouping

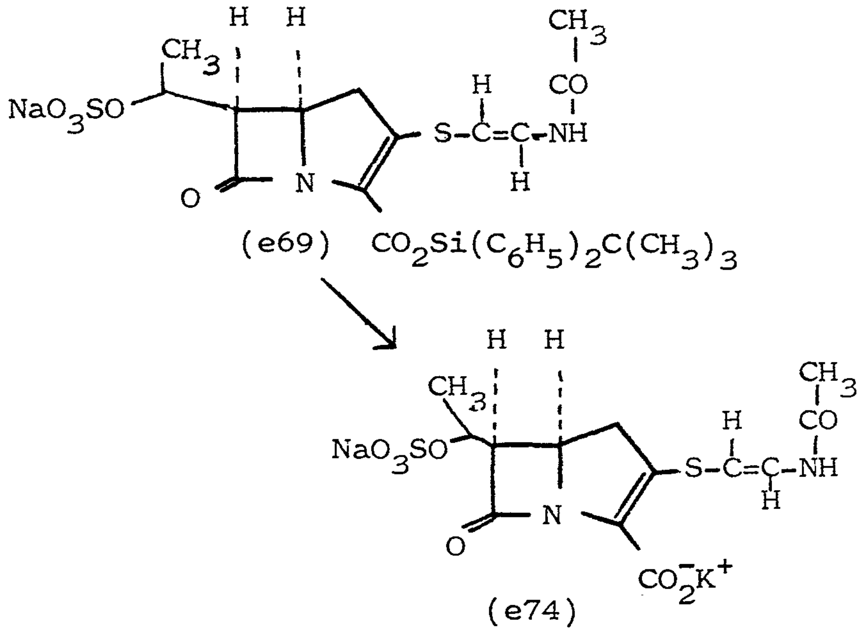

(e69)

(e74)

Method I

tert-Butyldiphenylsilyl (5R,6R)-3-[(E)-2-acetamidoethenylthio]-6-[sodium (S)-1-sulphonatooxyethyl]-7-oxo-1-azabicyclo[3.2.0]-hept-2-ene-2-carboxylate (e 69) (17.8 mg) in N,N-dimethylformamide (0.5 ml) was treated with a few crystals of 18-crown-6 crown ether, followed by anhydrous potassium fluoride (3 mg). After 5 hr. the solvent was removed in vacuo and ethyl acetate (2 ml) and water (2 ml) were added to the residue. After partitioning, the aqueous layer was loaded onto Biogel P-2 (13 x 2.3 cm column) and eluted with deionised water, the pH of which had been adjusted to 7 by addition of very dilute $NaHCO_3$ solution. Fractions containing the salt were combined and evaporated in vacuo to low volume, ethanol was added and evaporated in vacuo (3x), then toluene was added and evaporated in vacuo to leave the di-salt (e 74) as a solid (3 mg).

Method 2

The ester (e 69) was dissolved in water. H.p.l.c.

of the solution [Waters $C_{18}$ Microbondapack column eluting with aqueous $NH_4H_2PO_4$ (5.75 g/l) with 5% $CH_3CN$, pH adjusted to 6.8 with $NH_4OH$] demonstrated that hydrolysis of the ester had occurred.

Example 54

p-Nitrobenzyl (5R,6E)-3-(2-acetamidoethylthio)-6-ethylidene-7-oxo-1-azabicyclo[3.2.0]hept-2-ene-2-carboxylate and p-Nitrobenzyl (5R,6Z)-3-(2-acetamidoethylthio)-6-ethylidene-7-oxo-1-azabicyclo[3.2.0]hept-2-ene-2-carboxylate

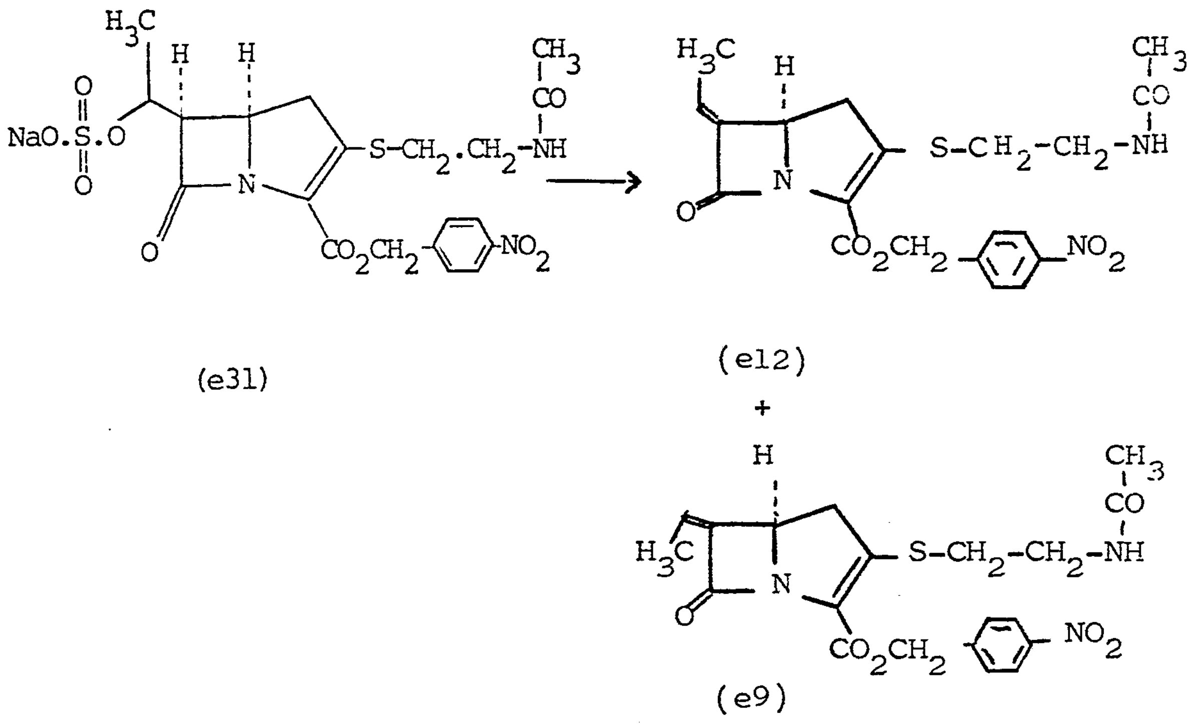

(e31)

(e12)

+

(e9)

A solution of the mono-ester, mono-salt (e 31) (0.8 g of 80% pure material) in water (50 ml) was shaken with a solution of cetylbenzyldimethyl-ammonium chloride (0.46 g) in chloroform (50 ml). The aqueous layer was re-extracted with chloroform (10 ml) and the combined organic phases were dried ($MgSO_4$) and evaporated in vacuo.

The resulting quaternary ammonium salt was dissolved in dichloromethane (50 ml) and the solution was cooled to $-30^{\circ}$. 1,5-Diazabicyclo [5.4.0] undec-5-ene (DBU) (0.25 g) was added to the solution which was

then kept at 0° for 4.5 hr. The solution was then washed with brine and dried ($MgSO_4$). Evaporation of the solution in vacuo afforded a gum which was chromatographed on silica gel, using ethyl acetate followed by 20% ethanol in ethyl acetate to elute, to afford a mixture of E- and Z-ethylidene derivatives (e 12) and (e 9) (0.103 g) (about 1:1); νmax (KBr) 1765, 1700, 1655 and 1635 $cm^{-1}$.

Example 55

p-Bromobenzyl (5R,6Z)-3-(2-acetamidoethylthio)-6-ethylidene-7-oxo-1-azabicyclo[3.2.0]hept-2-ene-2-carboxylate and p-bromobenzyl (5R,6E)-3-(2-acetamidoethylthio)-6-ethylidene-7-oxo-1-azabicyclo[3.2.0]hept-2-ene-2-carboxylate

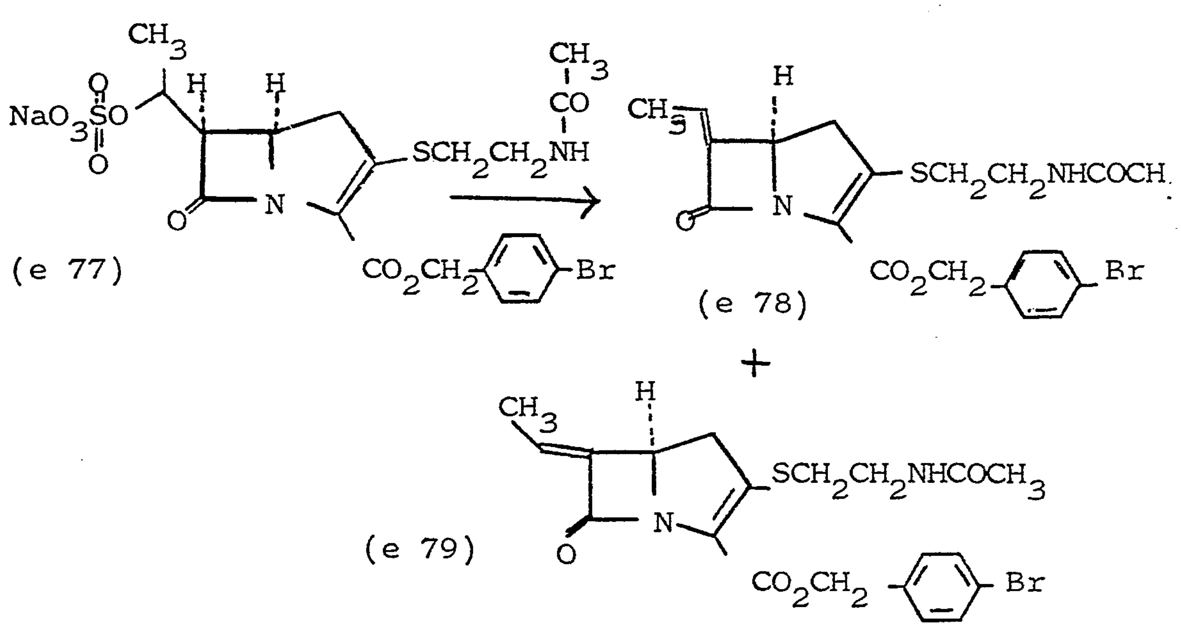

(e 77) (e 78) (e 79)

A solution of the mono-ester (e77) (1.15g, about 60% pure) in water (30 ml) was shaken with a solution of cetylbenzyldimethylammonium chloride (0.39 g) in dichloromethane (30 ml). The organic layer was dried ($MgSO_4$) and evaporated in vacuo to afford a foam (881 mg).

The product was dissolved in dichloromethane (25 ml) and to the cooled ($0^o$) solution was added DBU (0.202 g). After standing at $0^o$ for 4 h the solution was washed with water and brine, dried ($MgSO_4$) and concentrated in vacuo. Chromatography of the residue on silica gel using neat $CHCl_3$ grading to 10% ethanol in $CHCl_3$ to elute afforded the title esters (e78 and e79) (81 mg) as a mixture of Z- and E-isomers (ca. 1:1); $\nu$max ($CHCl_3$) 3460, 1770, 1700, and 1670 $cm^{-1}$; $\delta$($CDCl_3$) 1.81 and 2.04 (total 3H, each d, J 7Hz, $CH_3CH$ for E- and Z-isomers respectively), 1.94

(3H, s, $C\underline{H}_3CO$), 2.80 - 3.55 (6H, m, $NC\underline{H}_2C\underline{H}_2S$ and 4-$C\underline{H}_2$ ), 4.70 (1H, m, 5-C$\underline{H}$), 5.12 and 5.33 (each 1H, d, *J* 13 Hz, $CO_2C\underline{H}_2$), 5.95 (approx. 0.5H, br q, *J* 7 Hz, $CH_3C\underline{H}$ for Z-isomer), *ca.* 6.25 (1H, br, N$\underline{H}$), 6.40 (approx. 0.5H, q d, *J* 7 and 1 Hz, $CH_3C\underline{H}$ for E-isomer) and 7.25 - 7.55 (4H, m, $C_6\underline{H}_4$).

Example 56

Phthalidyl (5R)-3-[(E)-2-acetamidoethenylthio]-6-(Z)-ethylidene-7-oxo-1-azabicyclo[3.2.0]hept-2-ene-2-carboxyla

(e 80)

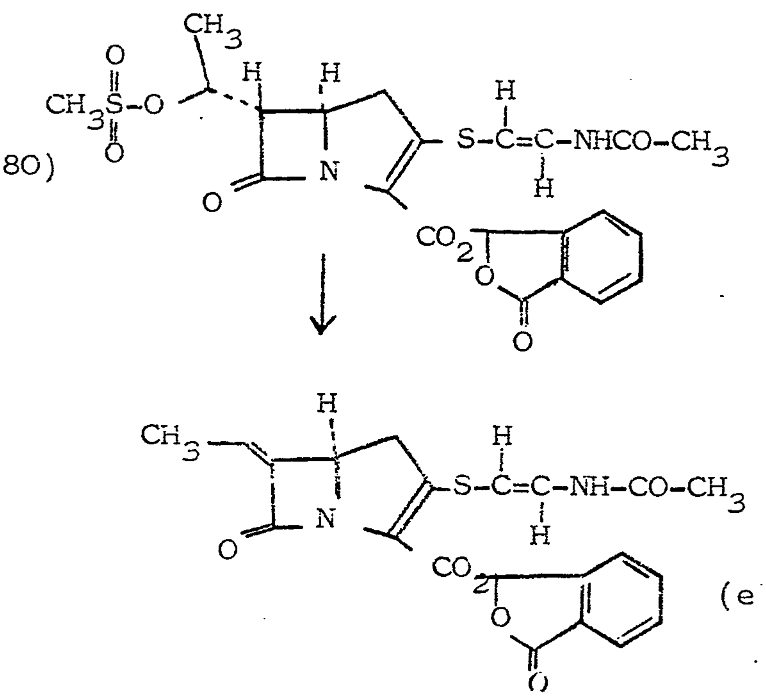

(e 81)

A solution of the mesylate (e80) (350 mg) in DMF (5 ml) was stirred vigorously with anhydrous $K_2CO_3$ (277 mg) for 1 h. The mixture was filtered and the filtrate was evaporated in vacuo. The residue was chromatographed on silica gel using a gradient elution of $CHCl_3$ to 10% ethanol in $CHCl_3$ to elute. The ethylidene phthalidyl ester (e81) was obtained as a pale yellow solid (110 mg); $\lambda$ max. (EtOH) 336, 272 and 229 nm; $\nu$max. (KBr) 1760 - 1785 (br), 1700 and 1620 $cm^{-1}$; $\delta$[$(CD_3)_2SO$] approx. 1.95 (6H, br, C$\underline{H}_3$CH and C$\underline{H}_3$CO), approx. 3.1 (2H, m, 4-C$\underline{H}_2$), 4.66 (1H, br t, $\underline{J}$ 9 Hz, 5-C$\underline{H}$), 5.86 (1H, d, $\underline{J}$ 14 Hz, =C$\underline{H}$S), 6.10 (1H, br q, $\underline{J}$ 7 Hz, $CH_3$C$\underline{H}$:), approx. 6.85 - 7.25 (1H, m, =C$\underline{H}$N), approx. 7.57 - 8.00 (5H, m, phthalidyl protons).

Example 57

Benzyl (5R)-3-[(E)-2-acetamidoethenylthio]-6-(Z)-ethylidene-7-oxo-1-azabicyclo[3.2.0]hept-2-ene-2-carboxylate

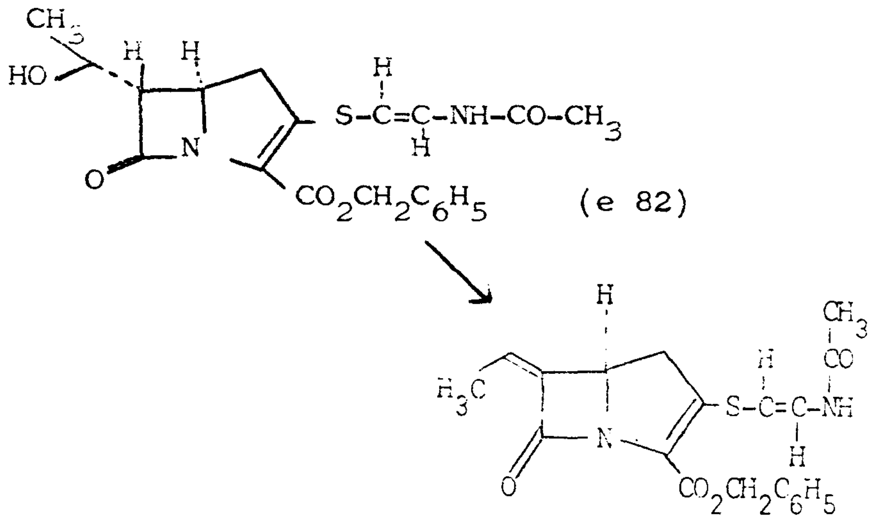

(e 82)

(e37)

The ester (e82) (50 mg) was dissolved in THF (3 ml) and triphenylphosphine ($Ph_3P$) (49 mg) was added. The solution was cooled to 0° and diethylazodicarboxylate (DEAD) (32 mg) was added with stirring. The solution was allowed to warm to room temperature and stirring was continued for 1 h after which more $(C_6H_5)_3P$ (50 mg) and DEAD (32 mg) were added. After a further 15 min the solution was evaporated and the residue was partitioned between ethyl acetate and water. The organic layer was washed with brine, then dried ($MgSO_4$) and evaporated in vacuo, and the residue chromatographed on silica gel using a gradient elution of $CHCl_3$ to 10% ethanol in $CHCl_3$.

The (Z)-isomer (e37) was obtained as a foam (28 mg); $\nu_{max}$ ($CHCl_3$) 1765, 1700 and 1625 $cm^{-1}$.

Example 58

Benzyl (5R)-3-[(E)-2-acetamidoethenylthio]-6-ethylidene-7-oxo-1-azabicyclo[3.2.0]hept-2-ene-2-carboxylate

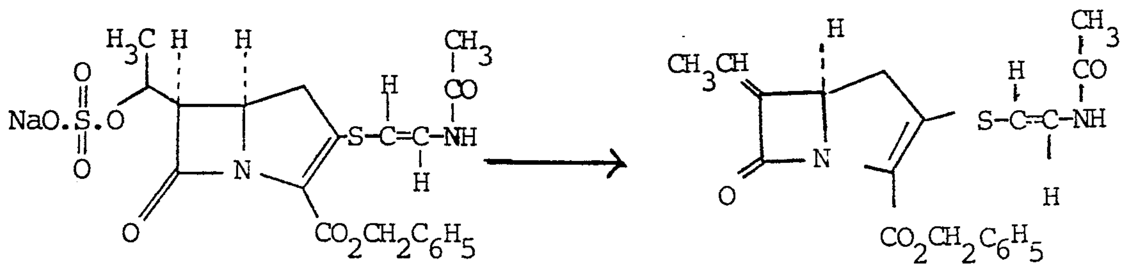

(e23) (e 16)

A solution of the benzyl ester (e23) (1.17 g) in water (50 ml) was shaken with a solution of cetylbenzyldimethylammonium chloride (0.9 g) in dichloromethane (50 ml). The organic layer was dried ($MgSO_4$) and evaporated in vacuo to afford the quaternary ammonium salt as a foam (1.8 g).

The salt was dissolved in dichloromethane (40 ml) and the solution was cooled to $0^{o}$ before adding DBU (0.6 g). After 4 h at $0^{o}$ the solution was washed with brine. The organic layer was dried ($MgSO_4$) and evaporated in vacuo. Chromatography on silica gel, using a gradient elution from ethylacetate/petroleum ether (4:1) to ethyl acetate, afforded the ethylidene derivative (e16) as a mixture of E- and Z-isomers (0.345 g).

Example 59

p-Nitrobenzyl (5R)-3-[(E)-2-acetamidoethenylthio]-6-ethylidene-7-oxo-1-azabicyclo[3.2.0]hept-2-ene-2-carboxylate

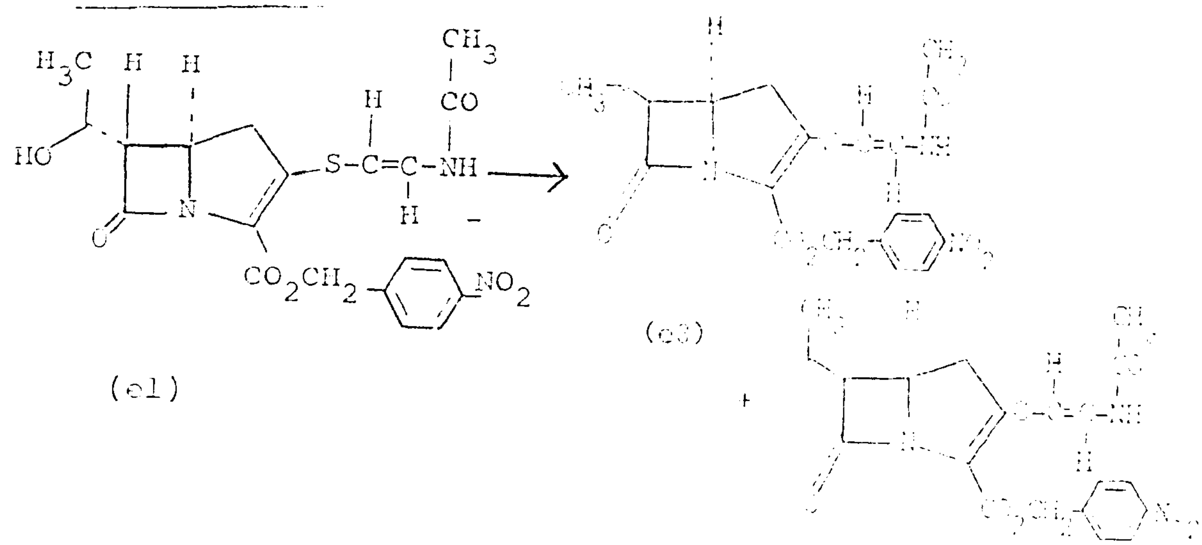

(e1) (e3) + (e6)

To a suspension of the ester (e1) (50 mg) in THF (2 ml) was added tri-n-butylphosphine (45 mg). Diethylazodicarboxylate (39 mg) was added with stirring, and stirring was continued until all the material had gone into solution (5 min). The solution was diluted with ethyl acetate and water and the organic layer was washed with water (x 2) and brine. Evaporation of the dried ($MgSO_4$) organic solution afforded a product which was chromatographed on silica gel using a gradient elution of 20% petrol in ethyl acetate to ethyl acetate. The ethylidene derivative was obtained as a mixture of (E)- and (Z)- isomers (e6 and e3) (about 2:3) (31 mg).

## Example 60

p-Nitrobenzyl (5R)-3-[(E)-2-acetamidoethenylthio]-6-(Z)-ethylidene-7-oxo-1-azabicyclo[3.2.0]hept-2-ene-2-carboxylate

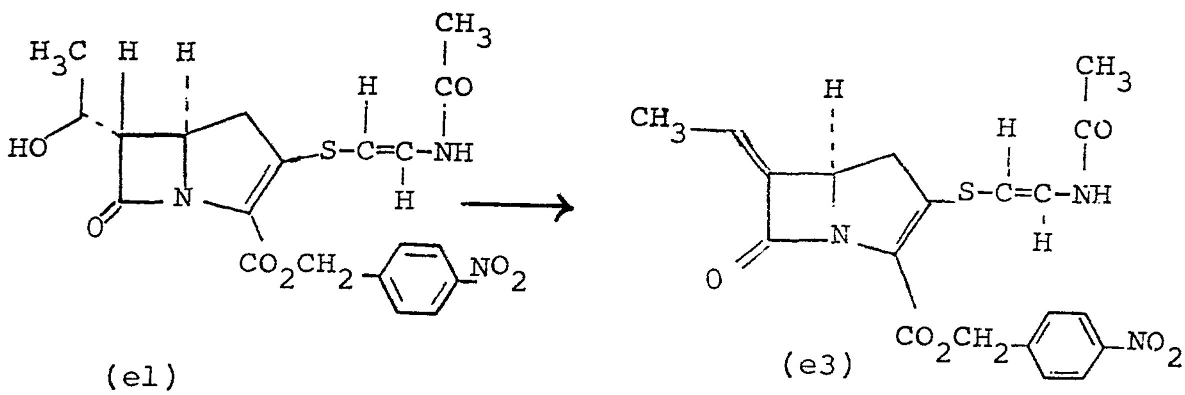

(e1) (e3)

To a suspension of the ester (e1) (1 g) in THF (30 ml) was added triphenylphosphine (1.17 g). The mixture was cooled to 0° and to it was added dropwise with stirring a solution of diethylazodicarboxylate (DEAD) (0.78 g) in THF (5 ml). The solution was allowed to warm to room temperature for 30 min, after which it was again cooled to 0° and further quantities of $(C_6H_5)_3P$ (0.3 g) and DEAD (0.2 g) were added. After another 10 min at room temperature the solution was evaporated in vacuo and the residue was partitioned between ethyl acetate (70 ml) and water (50 ml). The organic phase was washed with water (50 ml) and brine (30 ml), then dried ($MgSO_4$) and evaporated in vacuo. The product was chromatographed on silica gel using a gradient elution of 20% petroleum ether in ethyl acetate to 5% ethanol in ethyl acetate. Fractions containing the product were combined and concentrated in vacuo. Crystallisation from ethyl acetate-ether afforded the (Z)-isomer (e3) as a pale yellow solid (0.81 g).

Example 61

Sodium (5R,6S)-3-[(E)-2-acetamidoethenylthio]-6-[(S)-1-methyl-sulphonyloxyethyl]-7-oxo-1-azabicyclo[3.2.0]hept-2-carboxylate

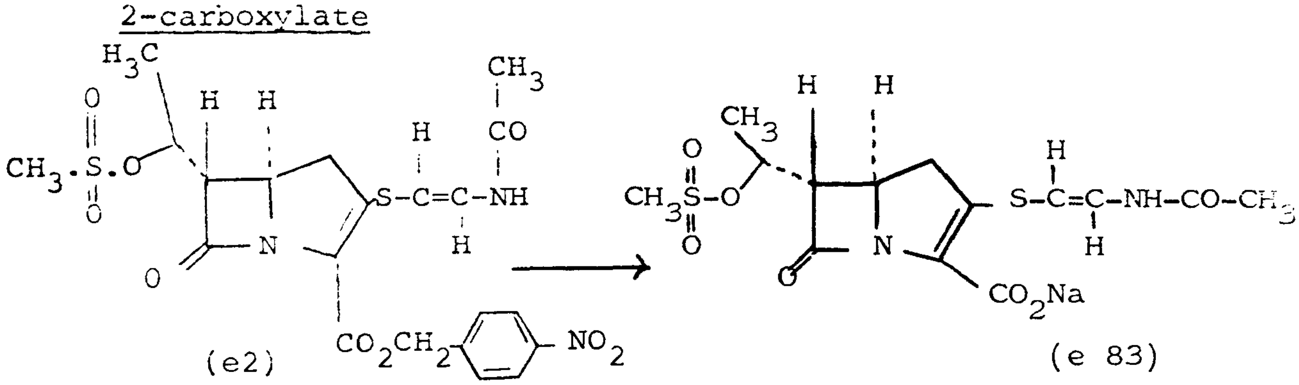

(e2) (e 83)

A solution of the mesylate (e2) (50 mg) in 40% aqueous dioxan (2 ml) was added to a mixture of 5% Pd on C (75 mg) and 40% aqueous dioxan (5 ml) which had previously been pre-hydrogenated for 0.5 h. Hydrogenation of the mixture was then continued for 3 h at room temperature and atmospheric pressure. The mixture was filtered through Celite and $NaHCO_3$ (9 mg) was added. The solution was concentrated in vacuo, and the aqueous layer washed with ethyl acetate (3 x 25 ml). The aqueous solution was chromatographed on Biogel P2 using water to elute. The fractions contining a u.v. chromophore at $\lambda$max. 305 nm were combined and evaporated down, using ethanol to azeotrope out water and toluene to azeotrope out ethanol, to afford the title salt (e83) as a cream-coloured solid (31 mg) ; $\lambda$max ($H_2O$) 305 and 228 nm; $\nu$max (KBr) 1765, 1670 and 1620 $cm^{-1}$.

Example 62

Sodium (5R,6R)-3-[(E)-2-acetamidoethenylthio]-6-[(S)-1-methylsulphonyloxyethyl]-7-oxo-1-azabicyclo[3.2.0]hept-2-ene-2-carboxylate

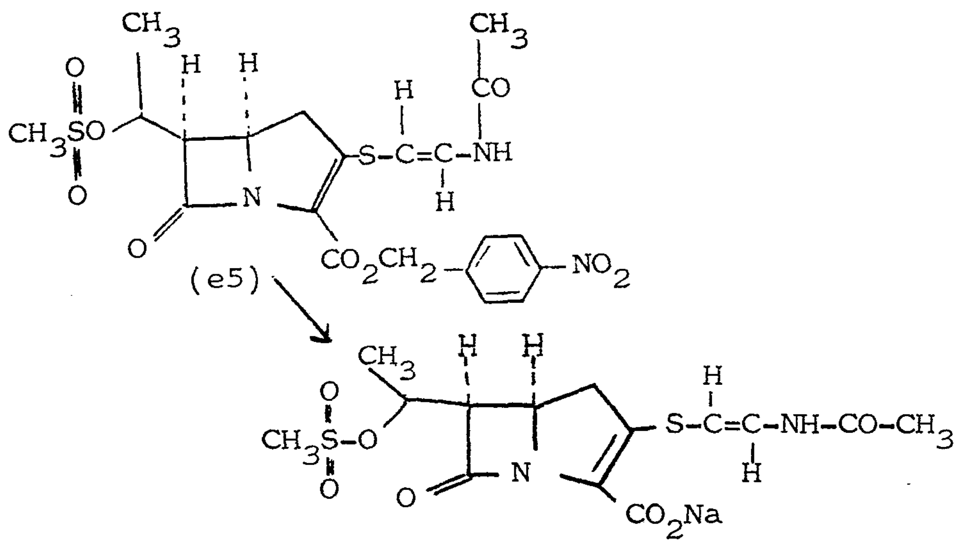

(e5)

(e 84)

The ester (e5) (90 mg) was hydrogenolysed as described in Example 61, using 5% Pd on C (100 mg) and 40% aqueous dioxan (10 ml).

The title salt (e 84 ) was obtained in aqueous solution after chromatography on Biogel P2. This solution was divided into two equal parts; one of these portions was evaporated in vacuo as in Example 61 to afford the salt (e84) as an amorphous solid (14 mg); $\lambda$max ($H_2O$) 306 and 228 nm; $\nu$max (KBr) 1750, 1670 and 1615 nm.

Example 63

Phthalidyl (5R,6S)-3-[(E)-2-acetamidoethenylthio]-6-[(S)-1-methylsulphonyloxyethyl]-7-oxo-1-azabicyclo[3.2.0]hept-2-ene-2-carboxylate

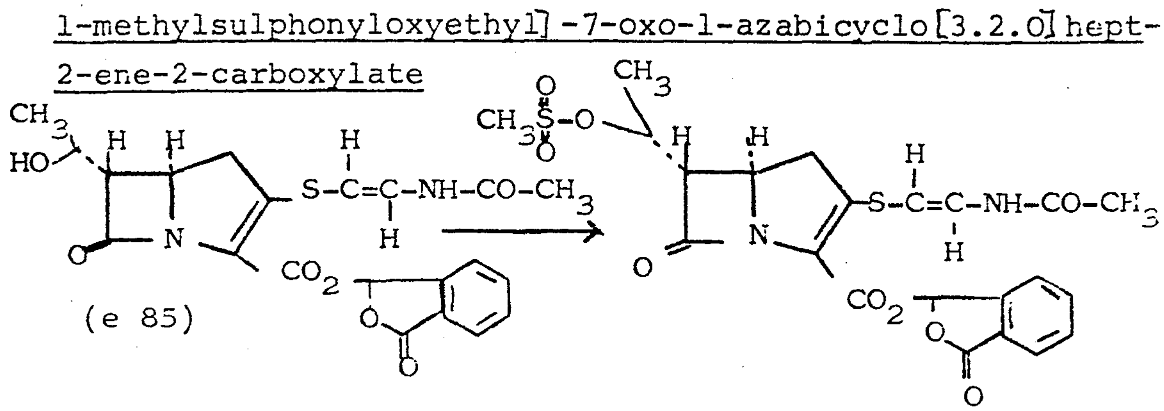

(e 85)

(e 80)

Triethylamine (34 mg) was added to a suspension of the ester (e85) (50 mg) in methylene chloride (2 ml). The mixture was cooled to -5$^{o}$ before adding a solution of mesyl chloride (25.7 mg) in methylene chloride (0.87 ml) dropwise over 2 min. Stirring was continued at -5$^{o}$ until the starting ester had completely dissolved (10 min) and a further quantity (15 ml) of methylene chloride was then added. The organic solution was washed with cold water (20 ml), cold pH3 phosphate buffer (2 x 15 ml) and cold aqueous $NaHCO_3$ solution (20 ml). It was then dried ($MgSO_4$) and evaporated in vacuo. Trituration of the residue with ether afforded the mesylate (e 80) as a yellow solid (32 mg); $\lambda$max ($C_2H_5OH$) 332 (12,450) and 230 nm (20,800); $\nu$max ($CHCl_3$) 1785, 1705 and 1625 $cm^{-1}$.

Example 64

p-Bromobenzyl (5R,6S)-3-(2-acetamidoethylthio)-6-ethyl-7-oxo-1-azabicyclo[3.2.0]hept-2-ene-2-carboxylate

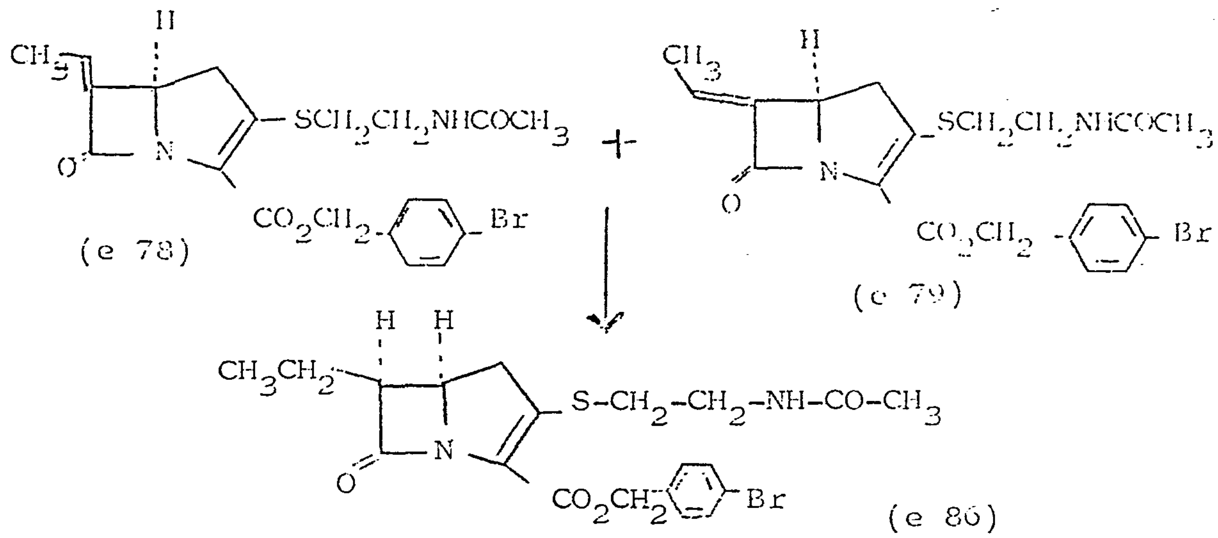

(e 78) (e 79) (e 80)

A solution of the Z- and E- ethylidene derivatives (e78 and e79) (80 mg) in ethyl acetate (10 ml) was hydrogenated at atmospheric pressure and room temperature in the presenc of platinum oxide catalyst (80 mg) for 23 h. The mixture was filtered through Celite and the solution was concentrated in vacuo. The product was chromatographed on a short silica column using ethyl acetate as eluant. The (5R,6S) derivative (e86) was obtained as a gum (17 mg); $\lambda$max (EtOH) 316 nm; $\nu$max ($CHCl_3$) 3460, 1780 and 1700 - 1670 (br) $cm^{-1}$ ; $\delta$ ($CDCl_3$) 1.03 (3H, t, J 7 Hz, $C\underline{H}_3CH_2$), approximately 1.67 (2H, m, $C\underline{H}_2CH_3$), 1.97 (3H, s, $C\underline{H}_3CO$), approx. 2.8 - 3.7 (7H, m, $SC\underline{H}_2C\underline{H}_2N$, 4-$C\underline{H}_2$ and 6-C$\underline{H}$), 4.25 (1H, dt, J 6 and 9 Hz, 5-C$\underline{H}$), 5.09 and 5.30 (each 1H, d, J 14 Hz, $CO_2C\underline{H}_2$), 5.90 (1H, br, N$\underline{H}$), 7.28 and 7.47 (each 2H, d, J 9 Hz, $C_6\underline{H}_4$). [$M^+$, 466(+468)]

The product contained a trace of the corresponding (5R,6R)-isomer.

Example 65

p-Nitrobenzyl (5R,6R)-3-(2-acetamidoethylthio)-6-ethyl-7-oxo-1-azabicyclo[3.2.0]hept-2-ene-2-carboxylate

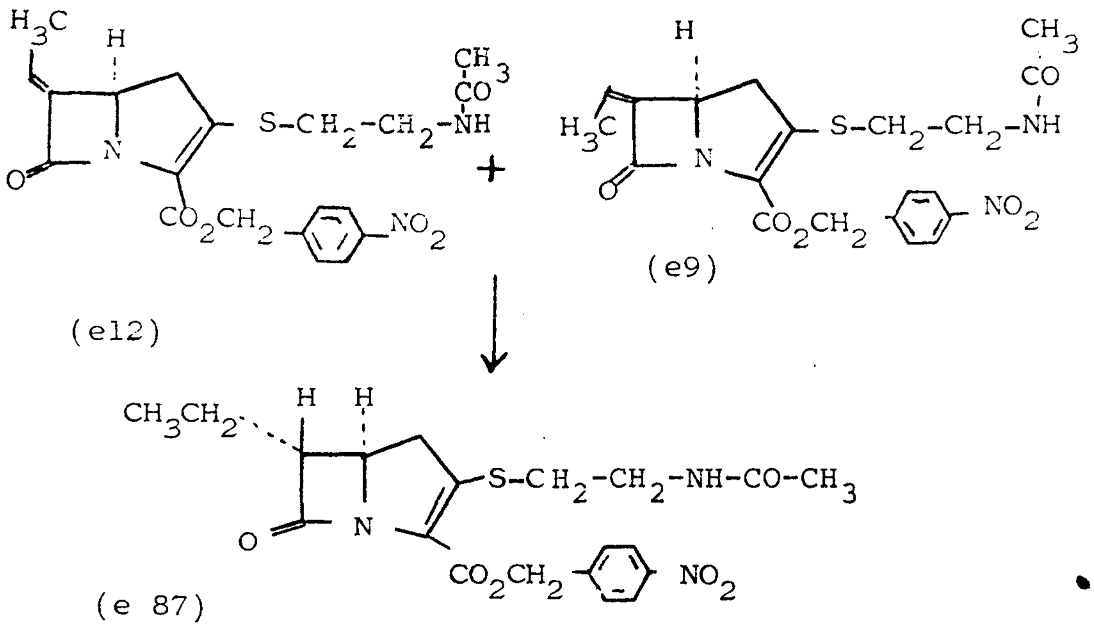

(e12) (e9) (e 87)

A mixture of E- and Z- ethylidene derivatives (e12 and e 9) (200 mg) were suspended in THF (10 ml) and water (ca.1 ml) was added until a solution was formed. The solution was cooled in an ice-salt bath and solid $NaBH_4$ (70 mg) was added with stirring. After 30 min, ethyl acetate (50 ml) was added and the solution was washed with water and brine, before drying ($MgSO_4$) and evaporating the solvent in vacuo.

The product was chromatographed on silica gel, using a gradient elution from ethyl acetate to 10% ethanol in ethyl acetate, to afford the title compound(e87) as a yellow solid (60 mg); $\lambda$max (EtOH) 317 (12,300) and 266 nm (11,200); $\nu$max (KBr) 1790 sh, 1780, 1700 sh, 1695 and 1630cm$^{-1}$; ($CHCl_3$) 1775, 1700 and 1670 cm$^{-1}$; $\delta$ [$(CD_3)_2$ NCDO] 1.02 (3H, t, J 7 Hz, $C\underline{H}_3CH_2$) ca. 1.63 - 2.00 (2H, m, $C\underline{H}_2CH_3$), 1.88 (3H, s, $C\underline{H}_3CO$), ca. 3.0-3.6 (7H, m, $SC\underline{H}_2C\underline{H}_2N$, 4-$C\underline{H}_2$ and 6-C$\underline{H}$), 4.03 (1H, dt, J 2.5 and 8.5 Hz, 5-C$\underline{H}$), 5.32 and 5.57 (each 1H, d, J 14 Hz, $CO_2C\underline{H}_2$), 7.82 and 8.27

(each 2H, d, J 9 Hz, $C_6H_4$) 8.10 (1H, br, NH)
[$M^+$, 433.1312. $C_{20}H_{23}N_3O_6S$ requires M, 433.1305]

The product contained a trace of the corresponding (5R,6S)-isomer.

Example 66

Sodium (5R,6R)-3-(2-acetamidoethylthio)-6-ethyl-7-oxo-1-azabicyclo[3.2.0]hept-2-ene-2-carboxylate

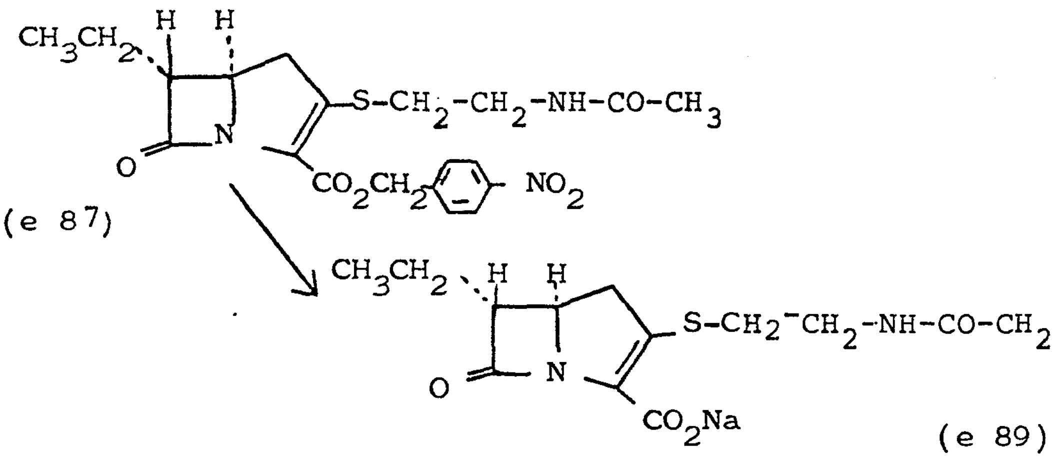

(e 87)

(e 89)

The ester (e87) (65 mg) was dissolved in 20% aqueous dioxan (2ml) and the solution was added to a mixture of 5% Pd on C (80 mg) in aqueous dioxan (8 ml) which had been prehydrogenated for 0.5 h. Hydrogenation was continued for 2 h at atmospheric pressure and room temperature before adding a further quantity of catalyst (30 mg) and hydrogenating for 1 h more. A solution of $NaHCO_3$ ( 5 mg) in water (2 ml) was then added and the mixture was filtered through Celite, washing well with water. The solution was concentrated in vacuo to about 10 ml and was then washed with ethyl acetate (3 x 50 ml). The aqueous layer was concentrated in vacuo to about 5 ml and then loaded onto a column (15 x 2.5 cm) of Biogel P2. Elution with deionised water gave several fractions containing the title salt (e89) detected by u.v.; $\lambda$max ($H_2O$) 299 nm.

Example 67

p-Nitrobenzyl (5R,6R)-3-[(E)-2-acetamidoethenylthio]-6-(1-methoxycarbonylmethylthioethyl)-7-oxo-1-azabicyclo [3.2.0]hept-2-ene-2-carboxylate

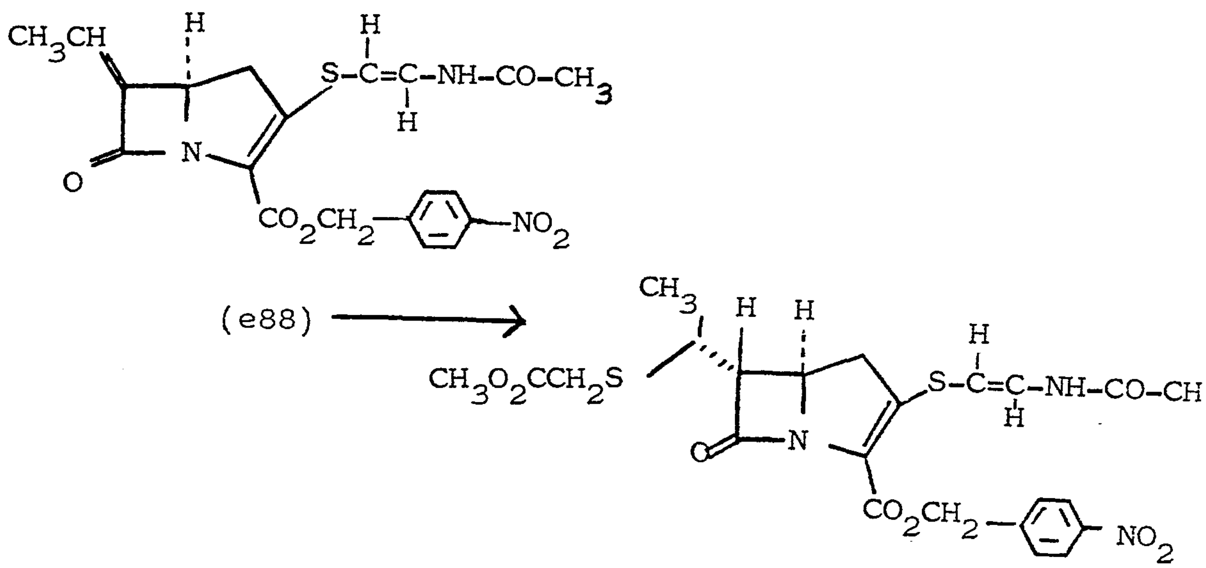

(e88)

(e 90)

A solution of the ethylidene derivative (e88) (180 mg) in DMF (5 ml) was cooled to -30°C. Anhydrous $K_2CO_3$ (29 mg) was added with vigorous stirring followed by methyl 2-mercaptoacetate (42 mg). After stirring for 15 min at -30°C the solution was diluted with ethyl acetate (30 ml) and the organic solution was washed with water (x3) and brine. Evaporation of the dried ($MgSO_4$) organic solution afforded a gum which was chromatographed on a column of silica gel, employing a gradient elution of petroleum ether/ethyl acetate (2:3) to ethyl acetate. After discarding several fractions containing non-β-lactam products, other fractions containing impure thioether (e 90) were combined and concentrated in vacuo.

the crude product was re-chromatographed using the same solvent system to afford substantially pure thioether (e 90) (19 mg) as a mixture of diastereoisomers; λmax (EtOH) 325 nm; νmax ($CHCl_3$) 1780, 1730, 1700 and 1625 $cm^{-1}$ δ($CDCl_3$) approx 1.30 - 1.60 (3H, m, $C\underline{H}_3CH$), 2.09 (3H, s, $C\underline{H}_3CO$), approx 2.85 - 3.55 (6H, m, 4-$C\underline{H}_2$, 6-C<u>H</u>, $C\underline{H}SC\underline{H}_2$), 3.75 (3H, s, $C\underline{H}_3O_2C$), 4.12 (1H, m, 5-C<u>H</u>), 5.35 (2H, centre of AB, $CO_2C\underline{H}_2$), 5.86 (1H, d, *J* 13.5 Hz, =CHS), 7.22 (1H, m, =C<u>H</u>N), approx 7.5 (1H, N<u>H</u>), 7.63 and 8.22 (each 2H, d, *J* 8.5 Hz, aromatic protons).

Example 68

Benzyl (5R,6R)-3-[(E)-2-acetamidoethenylthio]-6-[(R)-1-phenylthioethyl]-7-oxo-1-azabicyclo[3.2.0]hept-2-ene-2-carboxylate

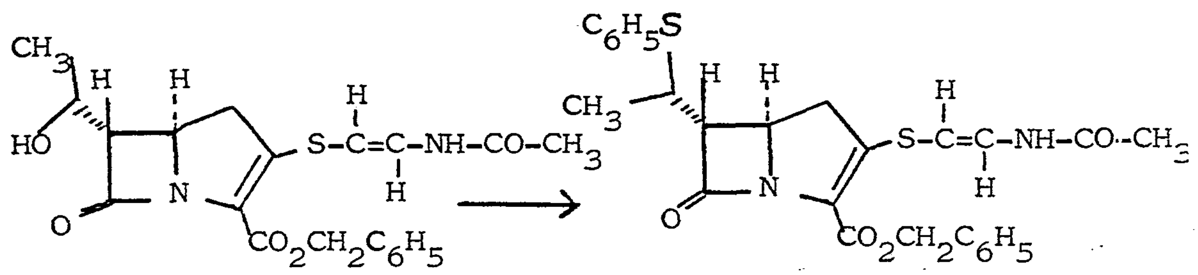

(e 82) (e 91)

Phenylthiosuccinimide (31 mg) and tri-n-butylphosphine (30 mg) were dissolved in THF (3 ml). After stirring for 5 min at r.t. the alcohol (e 82) (50 mg) was added in one portion. After stirring for 4 hr at room temperature the solution was diluted with ethyl acetate (25 ml) and washed with water and brine. Evaporation of the dried ($MgSO_4$) solution gave a residue which was chromatographed on silica gel using a gradient elution of petroleum ether/ethyl acetate (1:1 to 3:7). The title compound (e 91) was obtained as a gum (8 mg); λmax (EtOH) 325 nm; νmax ($CHCl_3$) 1775, 1700 and 1620 $cm^{-1}$. δ($CDCl_3$) 1.44 (3H, d, $\underline{J}$ 6.5 Hz, $C\underline{H}_3CH$), 2.06 (3H, s, $C\underline{H}_3CO$), 2.85 - 3.20 (3H, m, 4-$C\underline{H}_2$ and 6-$C\underline{H}$), approx. 3.4 (1H, m, $CH_3C\underline{H}$), 3.93 (1H, dt, $\underline{J}$ 3 and 9 Hz, 5-$C\underline{H}$), 5.24 (2H, centre of AB, wings at 5.07 and 5.40, $CO_2C\underline{H}_2$), 5.82 (1H, d, $\underline{J}$ 13.5 Hz, $SC\underline{H}$=) and 7.0 - 7.5 (12H, m, $C_6\underline{H}_5CH_2$, $C_6\underline{H}_5S$, =$C\underline{H}$.$N\underline{H}$).

Example 69

p-Nitrobenzyl (5R,6R)-3-(2-acetamidoethylthio)-6-(1-ethylthioethyl)-7-oxo-1-azabicyclo[3.2.0]hept-2-ene-2-carboxylate

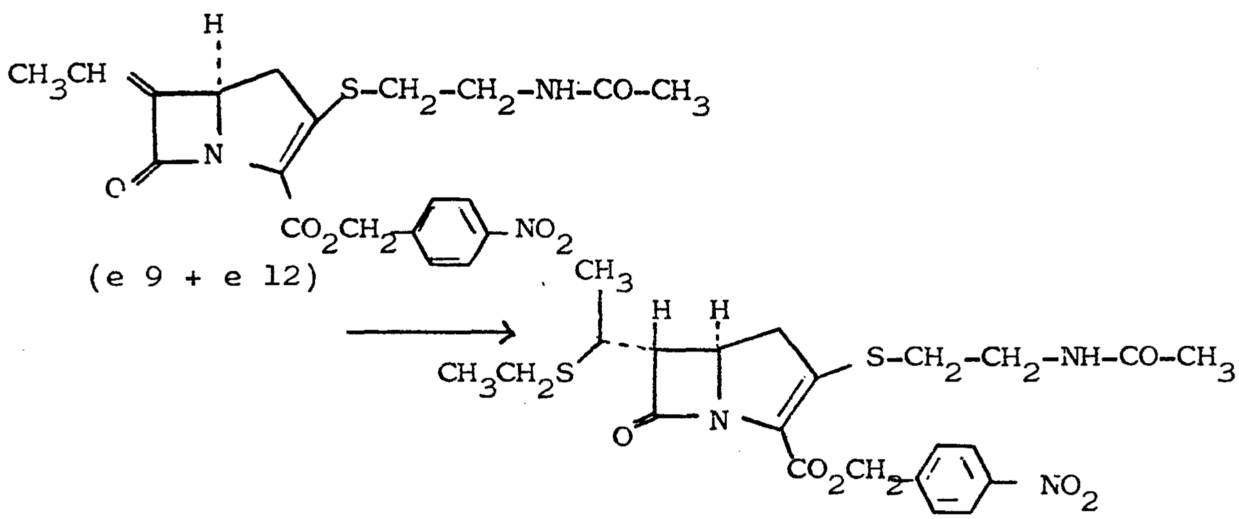

(e 9 + e 12)

(e 92)

A solution of the ethylidene derivatives(e 9 + e 12) (200 mg) in DMF (4 ml) was cooled to -30°C and anhydrous $K_2CO_3$ (32 mg) was added. To the vigorously stirred mixture was added ethanethiol (26 mg), and stirring at -30°C was continued for 1 hr. Ethyl acetate (30 ml) was added and the organic solution was washed with water (x4) and brine, then dried ($MgSO_4$) and evaporated in vacuo. The residue was chromatographed on silica gel using a gradient elution of ethyl acetate/petroleum ether (7:3) to ethanol/ethyl acetate (5:95). The title compound (e 92) was obtained as a foam (58 mg); λmax (EtOH) 320 and 267 nm; νmax ($CHCl_3$) 1775, 1700 and 1670cm$^{-1}$; δ($CDCl_3$) 1.26 (3H, t, J 7.5 Hz, $CH_3CH_2$), ca 1.25 - 1.50 (3H, m, $CH_3CH$), 1.97 (3H, s, $CH_3CO$), 2.59 (2H, q, J 7.5 Hz, $SCH_2CH_3$), 2.85 - 3.6 (8H, m, 4-$CH_2$, $NCH_2CH_2S$, 6-CH, and $CH_3CH$ ), 4.15 (br t, J approx 8 Hz, 5-CH), 5.20 and 5.49 (each 1H, d, J 14 Hz, $CO_2CH_2$), 6.05 (1H, br, NH), 7.63 and 8.15 (each 2H, d, J 9 Hz, $C_6H_4$).

Example 70

p-Nitrobenzyl (5R,6R) -3-[(E)-2-actamidoethenylthio]-6-(1-benzylthioethyl)-7-oxo-1-azabicyclo[3.2.0]hept-2-ene-2-carboxylate

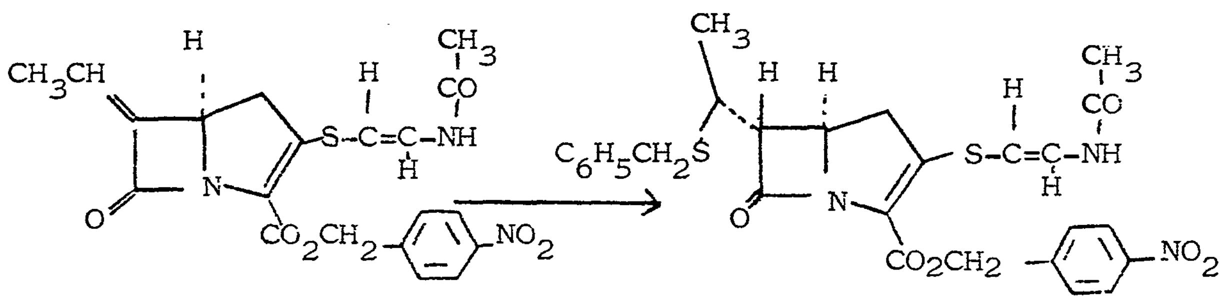

(e88) (e93)

The ethylidene derivative (e88) (320 mg) was dissolved in DMF and the solution was cooled to -25°. Anhydrous $K_2CO_3$ (52 mg) was added followed by benzyl mercaptan (83 mg) with vigorous stirring. Stirring was continued at -20°for 25 min and the solution was then diluted with ethyl acetate (35 ml) and washed with water, aqueous $NaHCO_3$ solution, more water (x 2) and brine. Evaporation of the dried ($MgSO_4$) organic solution gave a residue which was chromatographed on silica gel using a gradient elution of petroleum ether/ethyl acetate (3:2) to ethyl acetate. Early fractions containing the title thioether (e93) were evaporated in vacuo and the residue was crystallised from ethyl acetate/ether to afford a single isomer of the thioether (e93) (45 mg) ; $\lambda$max (EtOH) 327 (13,200) and 261 nm (13,800); $\nu$max (KBr) 1775, 1700, 1685 and 1620cm$^{-1}$; $\delta$ [$(CD_3)_2NCDO$] 1.40 (3H, d, J 7 Hz, C$\underline{H}_3$CH), 3.15 (2H, d, J 9 Hz, 4-C$\underline{H}_2$), approx. 3.15 (1H, m, $CH_3C\underline{H}S$), 3.52 (1H, dd, J 3 and 9 Hz, 6-C$\underline{H}$), 3.89 (2H, s, C$\underline{H}_2$S), 4.05 (1H, dt, J 3 and 9 Hz, 5-C$\underline{H}$), 5.32 and 5.56 [each 1H, d, J 14 Hz, $CO_2C\underline{H}_2$], 5.97 (1H, d, J 13.5 Hz, =C$\underline{H}$.S), 7.05 - 7.55 (6H, m, $C_6\underline{H}_5$ and =C$\underline{H}$.N), 7.79 and 8.24

[each 2H, d, J 9Hz, $C_6H_4$] and 11.2 (1H, brd, J 11 Hz, NH). [$M^+$, 553.1297. $C_{27}H_{27}N_3O_6S_2$ requires M, 553.1339].

Intermediate fractions containing the product were combined and evaporated to afford a mixture of the two diastereoisomers of compound (e93) (approx. 1:1) (42 mg) and later fractions afforded a mixture (approx. 2:3) (37 mg) that was enriched in the more polar isomer of the title compound; νmax. ($CHCl_3$) 1778, 1700 and 1625$cm^{-1}$; δ[$(CD_3)_2NCDO$] as described for the least polar isomer except for extra signals at 1.29 ( d, J7 Hz, $CH_3CH$), 3.76 (dd,J3 and 6 Hz, 6-CH) and approx 4.15 (m, 5-CH).

Example 71

Sodium (5R,6R)-3-[(E)-2-acetamidoethenylthio]-6-(1-benzylthioethyl)-7-oxo-1-azabicyclo[3.2.0]hept-2-ene-2-carboxylate

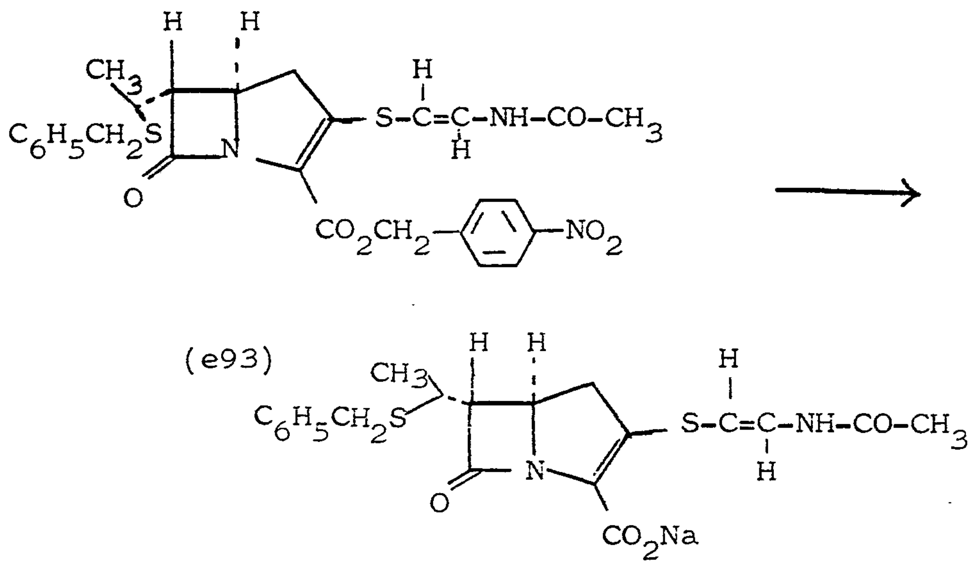

(e93)

(e94)

The least polar crystalline isomer of the ester (e93) (40 mg) obtained in Example 70 was added to a mixture of 5% Pd on C (60 mg) in 20% aqueous dioxan (5 ml) which had been prehydrogenated for 0.5 h. Hydrogenation was continued for 4 h and the mixture was filtered through Celite after adding $NaHCO_3$ (7 mg) and water (10 ml). The solution was concentrated to a volume of about 10 ml and was then washed with ethyl acetate (3 x 30 ml). The aqueous solution was concentrated to about 5 ml and then loaded onto a column (10 x 2.5 cm) of Biogel P2. Elution with water afforded fractions containing the title sodium salt (e94) as identified by the characteristic u.v. chromophore; λmax. ($H_2O$) 309 nm.

The minimum inhibitory concentrations (MIC) of this compound was determined using a standard DST agar + 10% horse blood substrate. The results are shown below.

| ORGANISM | MIC (μg/ml) |
|---|---|
| *Citrobacter freundii* E8 | 6.2 |
| *Enterobactor cloacae* N1 | 3.1 |
| *Escherichia coli* 0111 | 3.1 |
| *Escherichia coli* JT 39 | 3.1 |
| *Klebsiella aerogenes* A | 3.1 |
| *Proteus mirabilis* C977 | 12.5 |
| *Proteus morganii* I580 | 6.2 |
| *Proteus rettgeri* WM16 | 6.2 |
| *Proteus vulgaris* W091 | 6.2 |
| *Pseudomonas aeruginosa* A | 50 |
| *Salmonella typhimurium* CT10 | 1.6 |
| *Serratia marcescens* US20 | 6.2 |
| *Shigella sonnei* MB 11967 | 1.6 |
| *Bacillus subtilis* A | 1.6 |
| *Staphylococcus aureus* Oxford | 0.4 |
| *Staphylococcus aureus* Russell | 0.8 |
| *Staphylococcus aureus* 1517 | 50 |
| *Streptococcus faecalis* I | 25 |
| *Streptococcus pneumoniae* CN33 | 0.2 |
| *Streptococcus pyogenes* CN10 | 0.4 |
| *E. coli* ESS | 0.4 |

Example 72

p-Nitrobenzyl (5R,6R)-3-[(E)-2-acetamidoethenylthio]-6-(1-allylthioethyl)-7-oxo-1-azabicyclo[3.2.0]hept-2-ene-2-carboxylate

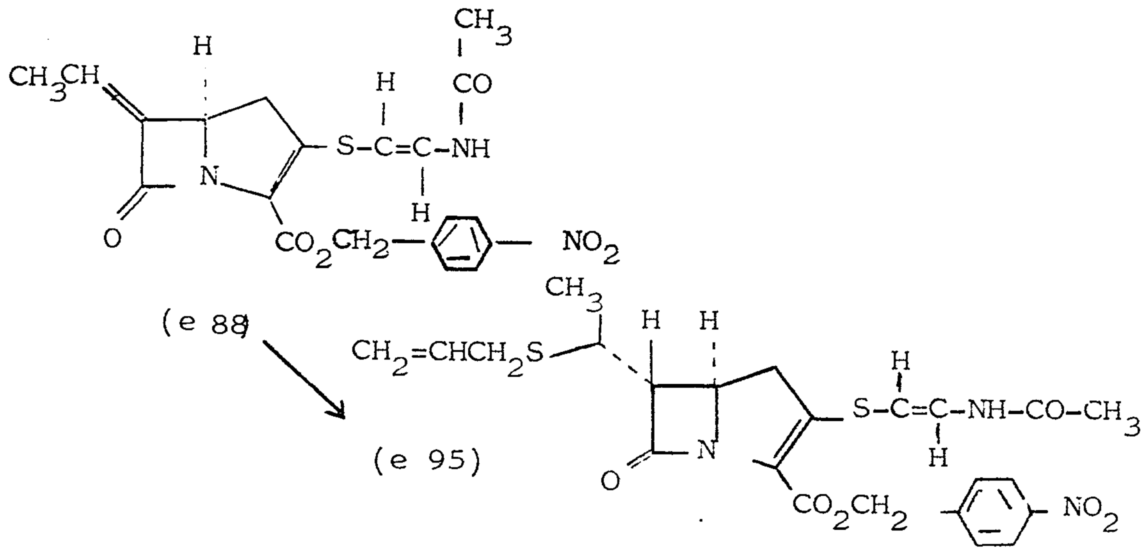

(e 88)

(e 95)

A solution of the ethylidene derivative (e88) (350 mg) in DMF (8ml) was treated with anhydrous $K_2CO_3$ (56 mg) and allyl mercaptan (60 mg) at $-20^o$ with vigorous stirring for 20 min. Ethyl acetate (40 ml) was added and the solution was washed with water (4 x 50 ml) and brine (20 ml), before drying ($MgSO_4$) and evaporating in vacuo. The crude product was chromatographed on silica gel using petroleum ether-ethyl acetate mixtures (from 3:2 to 1:9) as eluant.

Early fractions contained mainly the least polar isomer of the title thioether (e95) which was obtained as a foam (43 mg); νmax. 3430, 1775, 1700 and 1620 $cm^{-1}$; λmax (EtOH) 327 nm; δ($CDCl_3$) 1.45 (3H, $C\underline{H}_3CH$), 2.06 (3H,s,$C\underline{H}_3CO$), 2.9-3.3 (6H,m,$C\underline{H}_2S$, 4-$C\underline{H}_2$, 6-C$\underline{H}$ and $CH_3C\underline{H}S$), 4.10 (3H, br t, $\underline{J}$ 9 Hz), approx. 5.08 (2H, br d, allyl-$C\underline{H}_2$=) 5.22 and 5.48 (each 1H, d, $\underline{J}$ 14, $CO_2C\underline{H}_2$), approx. 5.6 - 6.0 (1H, m, allyl-C$\underline{H}$), 5.87 (1H, d, $\underline{J}$ 13.5 Hz, =C$\underline{H}$.S), 7.22 (1H, dd, $\underline{J}$ 13.5 and 10Hz, =C$\underline{H}$N), 7.63 and 8.19 (each 2H, d, $\underline{J}$ 9 Hz, $C_6\underline{H}_4$) and 7.83 (1H, br d, $\underline{J}$ 10 Hz, N$\underline{H}$).

The remaining fractions containing the product were combined and evaporated in vacuo to afford a gum (117 mg) which consisted of a mixture of the two diastereoisomers of (e95); i.r., u.v. and n.m.r. spectra as described for least polar isomer except for extra signals in the n.m.r. spectrum at $\delta(CDCl_3)$ 1.37 (d, J 7 Hz, $C\underline{H}_3CH$) and 3.45 (m, 5-C$\underline{H}$).

Example 73

Sodium (5R,6R)-3-[(E)-2-acetamidoethenylthio]-6-(1-allylthioethyl)-7-oxo-1-azabicyclo[3.2.0]hept-2-ene-2-carboxylate

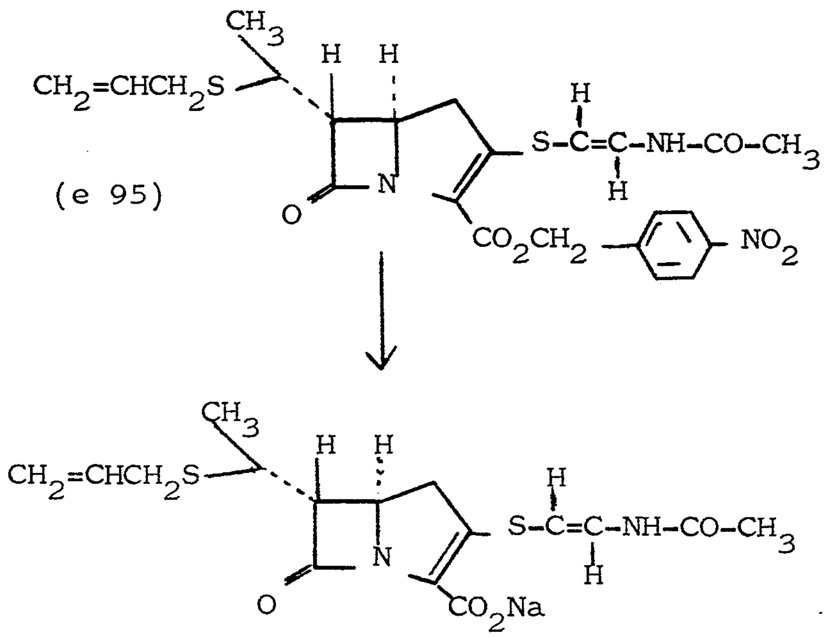

(e 95)

(e 96)

The diastereoisomeric ester (e95) (120 mg) was hydrogenolysed with 5% Pd on C (150 mg) in 20% aqueous dioxan (12 ml) in the manner described in Example 71. Work-up and chromatography on Biogel P2 afforded the title sodium salt (e96); λmax. ($H_2O$) 309 and 227 nm.

The minimum inhibitory concentrations (MIC) of this compound was determined using a standard DST agar + 10% horse blood substrate.

The results are shown below.

| ORGANISM | MIC (μg/ml) |
|---|---|
| Citrobacter freundii E8 | 50 |
| Enterobactor cloacae N1 | 25 |
| Escherichia coli 0111 | 50 |
| Escherichia coli JT 39 | 25 |
| Klebsiella aerogenes A | 6.2 |
| Proteus mirabilis C977 | 100 |
| Proteus morganii I580 | 50 |
| Proteus rettgeri WM16 | 50 |
| Proteus vulgaris W091 | 25 |
| Pseudomonas aeruginosa A | > 100 |
| Salmonella typhimurium CT10 | 25 |
| Serratia marcescens US20 | 50 |
| Shigella sonnei MB 11967 | 25 |
| Bacillus subtilis A | 1.6 |
| Staphylococcus aureus Oxford | 0.8 |
| Staphylococcus aureus Russell | 1.6 |
| Staphylococcus aureus 1517 | 12.5 |
| Streptococcus faecalis I | 50 |
| Streptococcus pneumoniae CN33 | ≤ 0.2 |
| Streptococcus pyogenes CN10 | 0.4 |
| E. coli ESS | 1.6 |

Example 74

p-Nitrobenzyl (5R,6R)-3-[(E)-2-acetamidoethenylthio]-6-[1-(2-acetamidoethylthio)ethyl]-7-oxo-1-azabicyclo-[3.2.0]hept-2-ene-2-carboxylate

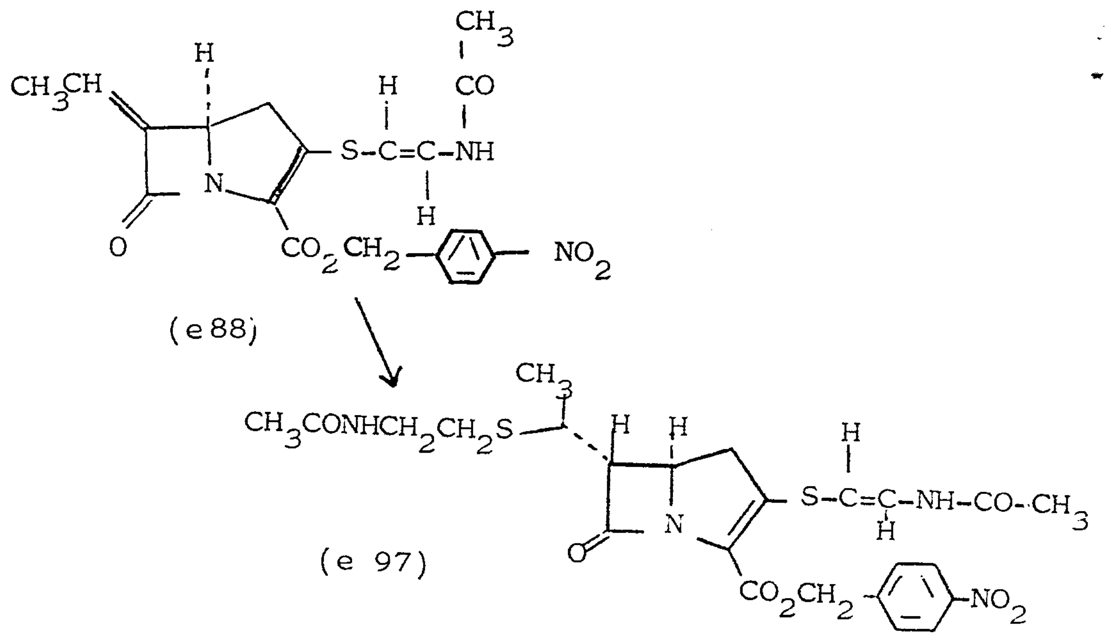

(e 88)

(e 97)

The ethylidene derivative (e 88) (200 mg) was dissolved in DMF (4 ml) and the solution was cooled to -25°. Anhydrous $K_2CO_3$ (32 mg) was added with vigorous stirring, followed by 2-acetamidoethylthiol (55 mg). Stirring was continued at -20° for 20 min and the mixture was diluted with ethyl acetate (50 ml). The solution was washed with water (4 x 30 ml) and brine (20 ml), then dried ($MgSO_4$) and evaporated in vacuo. The residue was chromatographed on silica gel using a gradient elution from chloroform to 20% EtOH in $CHCl_3$, to afford the title thioether (e97) as a foam (106 mg). Trituration of the product with ether gave a pale-yellow solid which consisted of a diastereoisomeric mixture (approx. 1:1) of the thioether derivative (e97); λmax. (EtOH) 327 (14,600) and 263 nm (17,200); νmax. (KBr) 1775, 1695, 1650 and 1620cm$^{-1}$; δ[$(CD_3)_2NCDO$ + $D_2O$] approx. 1.39 (3H, m, C<u>H</u>$_3$CH), 1.95 and 2.07 (each 3H, s, C<u>H</u>$_3$CO), 2.6 -

3.85 (8H, m, 4-C$\underline{H}_2$, NC$\underline{H}_2$C$\underline{H}_2$S, 6-C$\underline{H}$ and $CH_3$C$\underline{H}$), approx. 4.2 (1H, m, 5-C$\underline{H}$), 5.32 and 5.57 (each 1H, d, $\underline{J}$ 14 Hz, $CO_2$C$\underline{H}_2$), 6.07 (1H, d, $\underline{J}$ 13.5 Hz, =C$\underline{H}$.S), 7.19 (1H, d, $\underline{J}$ 13.5 Hz, = C$\underline{H}$N), 7.81 and 8.26 (each d, $\underline{J}$ 9 Hz, $C_6\underline{H}_4$). [$M^+$ , 548.1395. $C_{24}H_{28}N_4O_7S_2$ requires 548.1396].

Example 75

Sodium (5R,6R)-3-[(E)-2-acetamidoethenylthio]-6-[1-(2-acetamidoethylthio)ethyl]-7-oxo-1-azabicyclo[3.2.0]hept-2-ene-2-carboxylate

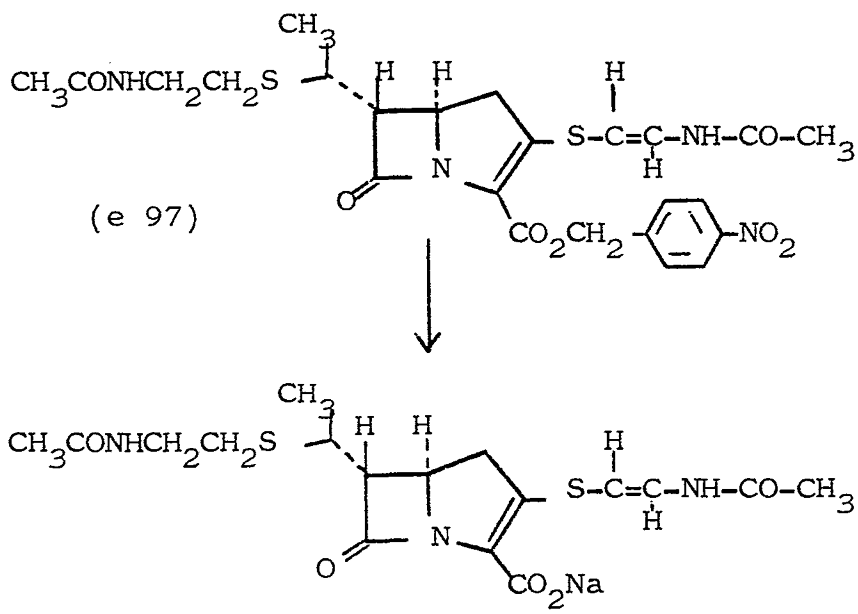

(e 97)

(e 98)

The ester (e97) (60 mg) was hydrogenolysed with 5% Pd on C (90 mg) in 20% aqueous dioxan (10 ml) as described in Example 71. Chromatography of the crude product on Biogel P2 afforded several fractions containing the desired sodium salt (e98) in aqueous solution ; λmax ($H_2O$) 309 and 228 nm.

The minimum inhibitory concentrations (MIC) of this compound was determined using a standard DST agar + 10% horse blood substrate.

The results are shown below.

| ORGANISM | MIC (μg/ml) |
|---|---|
| _Citrobacter freundii_ E8 | 5.0 |
| _Enterobactor cloacae_ N1 | 5.0 |
| _Escherichia coli_ 0111 | 5.0 |
| _Escherichia coli_ JT 39 | 5.0 |
| _Klebsiella aerogenes_ A | 5.0 |
| _Proteus mirabilis_ C977 | 12.5 |
| _Proteus morganii_ I580 | 25 |
| _Proteus rettgeri_ WM16 | 12.5 |
| _Proteus vulgaris_ W091 | 12.5 |
| _Pseudomonas aeruginosa_ A | >50 |
| _Salmonella typhimurium_ CT10 | 2.5 |
| _Serratia marcescens_ US20 | 12.5 |
| _Shigella sonnei_ MB 11967 | 5.0 |
| _Bacillus subtilis_ A | 2.5 |
| _Staphylococcus aureus_ Oxford | 1.2 |
| _Staphylococcus aureus_ Russell | 2.5 |
| _Staphylococcus aureus_ 1517 | 12.5 |
| _Streptococcus faecalis_ I | 50 |
| _Streptococcus pneumoniae_ CN33 | 0.2 |
| _Streptococcus pyogenes_ CN10 | 0.5 |
| _E. coli_ ESS | 1.2 |

Example 76

p-Nitrobenzyl (5R,6R)-3-[(E)-2-acetamidoethenylthio]-6-[1-(2-p-nitrobenzyloxycarbonylaminoethylthio)ethyl]-7-oxo-1-azabicyclo[3.2.0]hept-2-ene-2-carboxylate

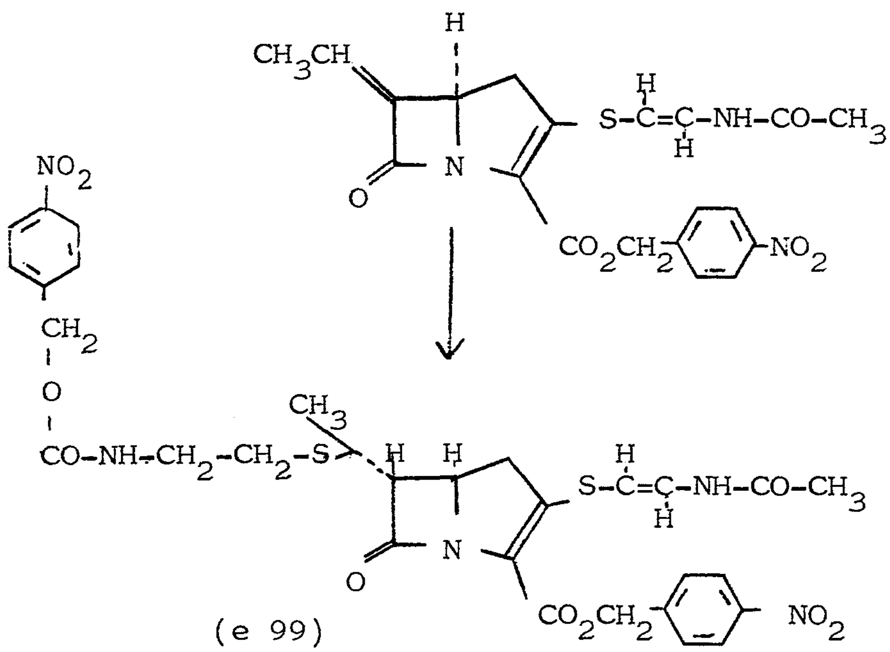

(e 88)

(e 99)

A solution of the ethylidene derivative (e88) (320 mg) in DMF (7 ml) was cooled to $-20^{o}$ and anhydrous $K_2CO_3$ (52 mg) was added. 2-p-nitrobenzyloxycarbonylamino-ethylthiol (191 mg) was added with efficient stirring, and the mixture was stirred at $-20^{o}$ for 20 min. Ethyl acetate (40 ml) was added and the solution was washed with water (3 x 50 ml) and brine ( 20 ml), then dried ($MgSO_4$) and evaporated in vacuo. The residue was chromatographed on silica gel using a gradient elution of petroleum ether-ethyl acetate 2:3 to ethyl acetate. Fractions containing the product (identified by t.l.c.) were combined and evaporated in vacuo to afford the title compound (e99)as a foam (169 mg); λmax (EtOH) 326 and 265 nm; νmax ($CHCl_3$) 1775, 1720 sh, 1700 and 1620 $cm^{-1}$; $\delta[(CD_3)_2CO]$ inter alia 1.42 (3H, d, J 6.5 Hz, $CH_3CH$),

1.97 (3H, s, $C\underline{H}_3CO$), 2.65 - 2.9 ( m, $C\underline{H}_2S$), approx. 3.05 - 3.5 (m, 4-$C\underline{H}_2$, $C\underline{H}_2N$, 6-C$\underline{H}$ and $C\underline{H}CH_3$), 4.15 (approx. dt, *J* approx. 3 and 9 Hz, 5-C$\underline{H}$), 5.02 (2H, s, $C\underline{H}_2OCO$), 5.27 and 5.53 (each 1H, d, *J* 14Hz, $C\underline{H}_2OCO$), 5.95 (1H, d, *J* 14 Hz, =C$\underline{H}$S), approx. 6.6 (1H, br, $CH_2N\underline{H}$), 7.21 (1H, dd, *J* 11 and 14 Hz, =C$\underline{H}$N), 7.55 - 7.9 and 8.22 (8H, m, aromatic protons).

Example 77

p-Nitrobenzyl (5R,6R)-3-[(E)-2-acetamidoethenylthio]-6-[(R)-1-methylthioethyl]-7-oxo-1-azabicyclo [3.2.0] hept-2-ene-2-carboxylate and p-nitrobenzyl (5R,6R)-3-[(E)-2-acetamidoethenylthio]-6-[(S)-1-methylthioethyl]-7-oxo-1-azabicyclo [3.2.0] hept-2-ene-2-carboxylate.

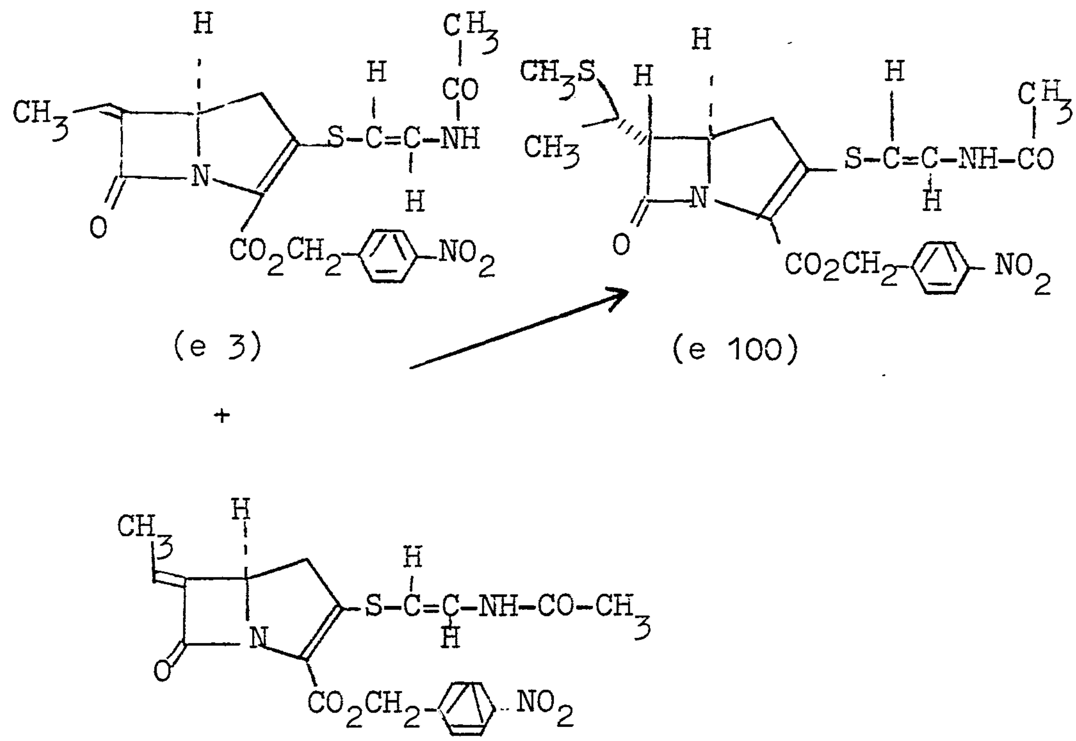

(e 3) (e 100)

+

(e 6)

A solution of the ethylidene derivatives (e3 and e6) (500 mg) in DMF (9ml) was cooled to -20°. Anhydrous $K_2CO_3$ (80mg) was added followed by solution of methyl mercaptan (53 mg) in DMF (0.6 ml) with vigorous stirring. Stirring was continued at -20° for 20 min. before adding ethyl acetate (50 ml). The solution was washed with dilute $NaHCO_3$ solution (30 ml), water (5 x 40 ml) and brine (20 ml) before drying ($MgSO_4$) and evaporating in vacuo. The residue was chromatographed

on a column of slica gel using a gradient elution of petroleum ether/ethyl acetate mixtures (3:2 to 1:9).

Early fractions containing the product were combined and evaporated in vacuo to afford the least polar isomer of the title ester (e 100) as a crystalline solid; λmax. (EtOH) 327, 264 and 232nm; δ(DMF-d7) 1.40 (3H, d, *J* 6.5 Hz, $CH_3CH$), 2.02 (3H, s, $CH_3CO$), 2.13 (3H,s,$CH_3S$), approx. 3.2 (1H,m,$CH_3CH$), 3.28 (2H, d, *J* 9 Hz, 4-$CH_2$), 3.58 (1H, dd, *J* 3 and 9 Hz, 6-CH), 4.15 (1H, dt, *J* 3 and 9 Hz, 5-CH), 5.33 and 5.58 (each 1H, d, *J* 14 Hz, $CO_2CH_2$), 5.98 (1H, d, *J* 13.5 Hz, = CHS), 7.21 (1H, dd, *J* 13.5 and 10 Hz, = CH.N), 7.82 and 8.27 (each 2H, d, *J* 9 Hz, $C_6H_4$) and 10.38 (1H, br d, *J* 10 Hz, NH).

The main bulk of the fractions were combined and evaporated in vacuo to afford a white solid which was triturated with ethyl acetate/ether and filtered. The solid consisted of a mixture (approx. 1:1) of the 6-[(R)-methylthioethyl] and 6-[(S)-methylthioethyl] derivatives (e 100) (199 mg); λmax. (EtOH) 327, 264 and 232nm; νmax. (KBr) 1778, 1700 br 1675 and 1625cm$^{-1}$; δ(DMF-d7) as described previously for the least polar isomer plus the following signals corresponding to the more polar isomer; 1.32 (3H, d, *J* 6Hz,$CH_3CH$), 2.17 (3H,s, $CH_3S$) and 3.79 (1H, dd, *J* 3 and 6 Hz, 6-CH).

The last few fractions contained the more polar isomer only. If desired, further quantities of each of the single isomers (e100) may be obtained by rechromatography of the isomeric mixture.

Example 78

Sodium (5R,6R)-3-[(E)-2-acetamidoethenylthio]-6-(1-methylthioethyl)-7-oxo-1-azabicyclo [3.2.0] hept-2-ene-2-carboxylate

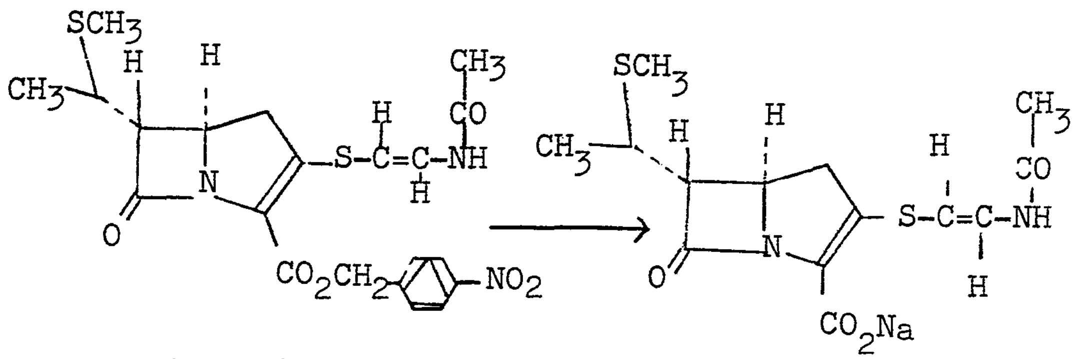

(e 100) (e 101)

A. A mixture of 5% Pd on C (40 mg) and 30% aqueous dioxan (3 ml) was hydrogenated at room temperature and atmospheric pressure for 0.5 h. A solution of the least polar isomer of the methylthio-derivative (e 100) (25 mg) in 30% aqueous dioxan (2 ml) was added to the hydrogenation vessel, and hydrogenation was continued for a further 4h. A solution of $NaHCO_3$ (5 mg) in water (2 ml) was added to the mixture which was filtered through Celite, washing with water. The solution was concentrated in vacuo to approximately 10 ml, and then washed with ethyl acetate (3 x 25 ml), before concentrating to approximately 3 ml. The aqueous solution was chromatographed on a column of Biogel P2 (2.5 x 15 cm), and fractions were monitored by u.v. Those which showed chromophores at both $\lambda$max. 309 and 228 nm contained a single isomer of the title sodium salt (e 101) (5 mg).

B. An isomeric mixture (approx. 1:1) of the thioether derivatives (e 100) (85 mg) was hydrogenated with 5% Pd on C catalyst (130 mg) in 30% aqueous dioxan (10 ml) exactly as described in section A, using

16 mg of $NaHCO_3$ during work-up. After Biogel P2 chromatography, fractions containing the product were combined and concentrated in vacuo using ethanol to azeotrope out water, and toluene to similarly remove ethanol. The title salt (e 101), a mixture of two diastereoisomers, was obtained as an off-white solid (30 mg); λmax. ($H_2O$) 309 and 228 nm; νmax. (KBr) 1755, 1675 and 1620 $cm^{-1}$.

The minimum inhibitory concentrations (MIC) of this compound was determined using a standard DST agar + 10% horse blood substrate. The tests used an undiluted inoculum of bacteria. The results are shown below.

| ORGANISM | MIC (μg/ml) |
|---|---|
| *Citrobacter freundii* E8 | 5.0 |
| *Enterobactor cloacae* N1 | 2.5 |
| *Escherichia coli* 0111 | 2.5 |
| *Escherichia coli* JT 39 | 2.5 |
| *Klebsiella aerogenes* A | 2.5 |
| *Proteus mirabilis* C977 | 12.5 |
| *Proteus morganii* I580 | 12.5 |
| *Proteus rettgeri* WM16 | 5.0 |
| *Proteus vulgaris* W091 | 5.0 |
| *Pseudomonas aeruginosa* A | 50 |
| *Salmonella typhimurium* CT10 | 1.2 |
| *Serratia marcescens* US20 | 5.0 |
| *Shigella sonnei* MB 11967 | 1.2 |
| *Bacillus subtilis* A | 1.2 |
| *Staphylococcus aureus* Oxford | 0.5 |
| *Staphylococcus aureus* Russell | 0.5 |
| *Staphylococcus aureus* 1517 | 12.5 |
| *Streptococcus faecalis* I | 12.5 |
| *Streptococcus pneumoniae* CN33 | 0.1 |
| *Streptococcus pyogenes* CN10 | 0.2 |
| *E. coli* ESS | 1.2 |

Example 79

p-Nitrobenzyl (5R, 6R)-3-[(E)-2-acetamidoethenylthio]-6-[(R)-1-phenylthioethyl]-7-oxo-1-azabicyclo[3.2.0]hept-2-ene-2-carboxylate

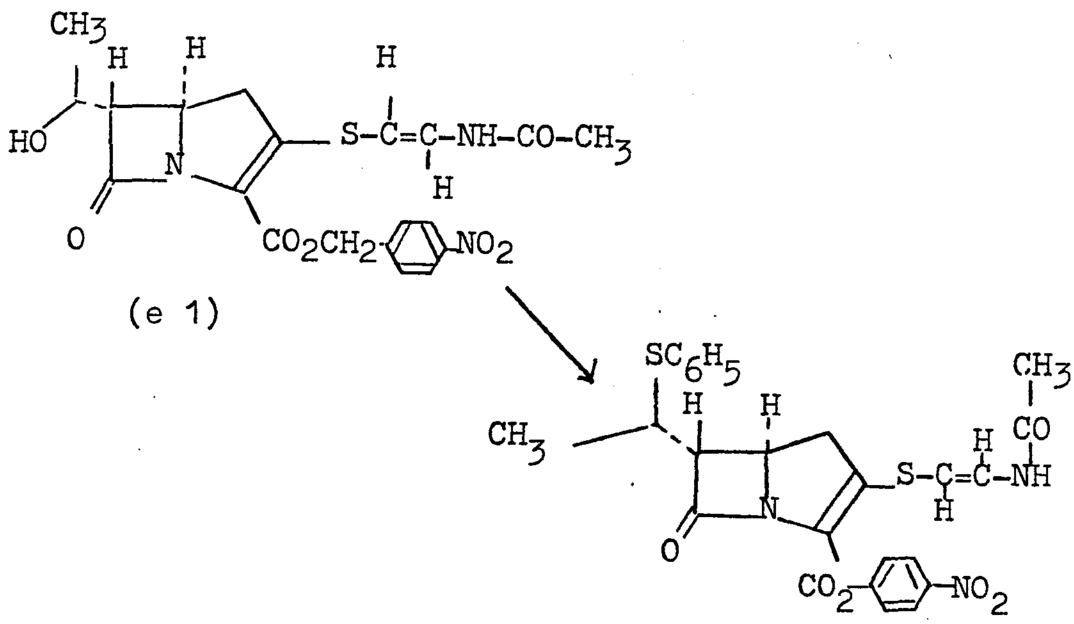

(e 1)

(e 102)

p-Nitrobenzyl (5R, 6S)-3-[(E)-2-acetamidoethenylthio]-6-[(S)-1-hydroxyethyl]-7-oxo-1-azabicyclo[3.2.0]hept-2-ene-2-carboxylate (e 1) (100 mg) in tetrahydrofuran (THF) (5 ml) was treated with N-phenylthiosuccinimide (43 mg) in THF (1 ml) followed by tri-n-butylphosphine (45 mg). After 1 hr. the solution was added to a solution of tri-n-butylphosphine (45 mg) in THF (4 ml) which had been treated with N-phenylthiosuccinimide (45 mg) in THF (1 ml) for 5 min. After a further 1 hr. the reaction mixture was added to tri-n -butylphosphine (150 mg) in THF (4 ml) which had been treated with N-phenylthiosuccinimide (145 mg) in THF (1 ml) and stirring was

continued for 3 hr. T.l.c. on silica gel using ethyl acetate elution indicated the formation of a $KMnO_4$ positive zone with $R_f$ slightly less than the sulphenimide/ phosphine complex. The THF was evaporated in vacuo, and ethyl acetate (100 ml) and water (100 ml) were added. The ethyl acetate layer was then washed with approx. 5% aqueous $NaHCO_3$ (20 ml), then with water, then dried ($MgSO_4$) and evaporated in vacuo. The residue was chromatographed on silica gel (230-400 mesh ASTM) (approx. 20 g) eluting with ethyl acetate/cyclohexane mixtures; 1:1 (50 ml); followed by 3:1. This gave the product, containing a contaminant (28 mg). The mixture was rechromatographed on silica gel, eluting with ethyl acetate/cyclohexane mixtures; 1:1 (50 ml), followed by 6:4, to give p-nitrobenzyl (5R, 6R)-3-[(E)-2-acetamidoethenylthio]-6-[(R)-1-phenylthioethyl]-7-oxo-1-azabicyclo[3.2.0]hept-2-ene-2-carboxylate (e 102) (22 mg) as a single isomer. λmax (EtOH) 327, 263 nm; νmax ($CHCl_3$) 1780, 1710, 1625 $cm^{-1}$; δ ($CDCl_3$ + $D_2O$) 1.46 (3H, d, _J_ 6 Hz), 2.07 (3H, s), 2.7 - 3.7 (4H, m), 3.8 - 4.2 (1H,m), 5.19 and 5.46 (2H, ABq, _J_ 14 Hz), 5.84 (1H, d, _J_ 14 Hz), 7.0 - 7.2 (8H,m), 8.20 (2H, d, _J_ 9Hz).

Example 80

p-Nitrobenzyl (5R, 6S)-3-(2-acetamidoethylthio)-6-[(R)-1-phenylthioethyl]-7-oxo-1-azabicyclo[3.2.0]hept-2-ene-2-carboxylate

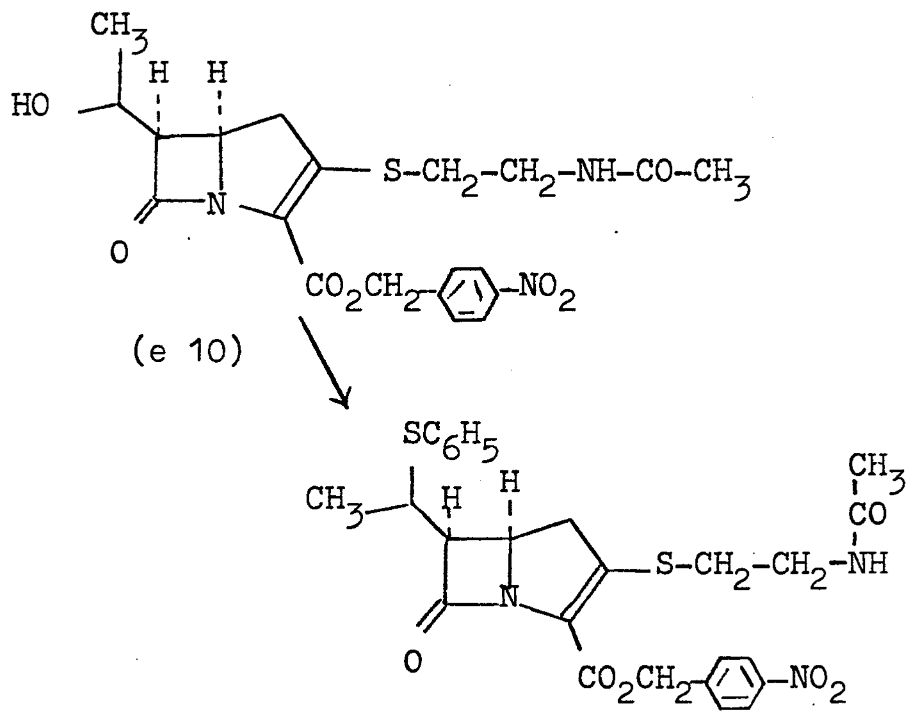

(e 10)

(e 103)

p-Nitrobenzyl (5R, 6R)-3-(2-acetamidoethylthio)-6-[(S)-1-hydroxyethyl]-7-oxo-1-azabicyclo[3.2.0]hept-2-ene-2-carboxylate (e 10) (100 mg) in suspension in tetrahydrofuran (THF) (5 ml) was added to tri-n-butylphosphine (240 mg) in THF (5 ml) which had been pretreated with N-phenylthiosuccinimide (240 mg) in THF (2 ml) for 5 min. After stirring at room temperature for 2.5 hr., the THF was evaporated in vacuo, ethyl acetate (50 ml) was added and the solution washed with water (2 x 50 ml), saturated aqueous $NaHCO_3$ (2 x 50 ml),

water (2 x 25 ml) and then with brine (25 ml). After drying ($MgSO_4$), the ethyl acetate was evaporated in vacuo and the residue chromatographed on silica gel (12 g) eluting with ethyl acetate/cyclohexane mixtures; 1:1 (50 ml), 6:4 (50 ml), 7:3 (50 ml), 8:2 (50 ml) and finally with ethyl acetate; this gave the thioether contaminated by by-products of the reaction, so it was rechromatographed on silica gel to give p-nitrobenzyl (5R, 6S)-3-(2-acetamidoethylthio)-6-[(R)-1-phenylthioethyl 7-oxo-1-azabicyclo[3.2.0]hept-2-ene-2-carboxylate (e 103) (11 mg). λmax (EtOH) 318, 264nm; νmax ($CHCl_3$) 1780, 1700, 1665 $cm^{-1}$.

Example 81

Sodium (5R, 6S)-3-(2-acetamidoethylthio)-6-[(R)-1-phenylthioethyl]-7-oxo-1-azabicyclo[3.2.0]hept-2-ene-2-carboxylate

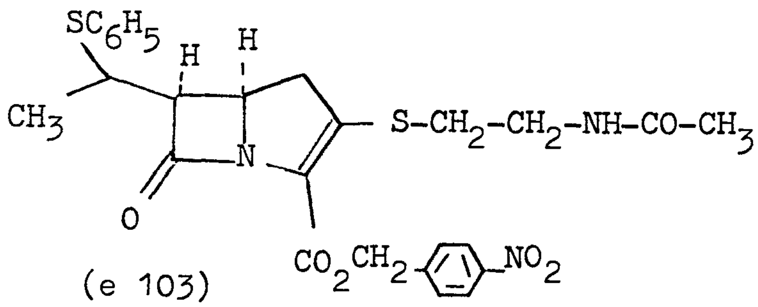

(e 103)

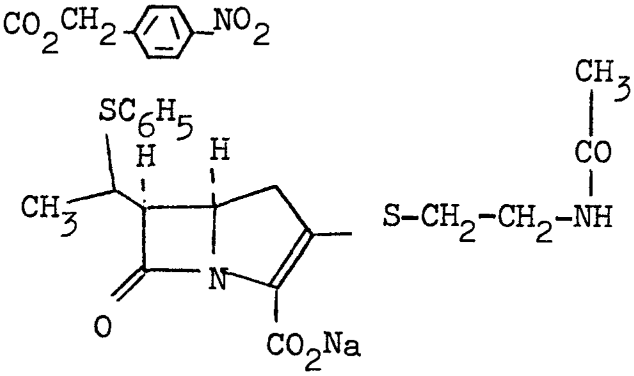

(e 104)

p-Nitrobenzyl (5R, 6S)-3-(2-acetamidoethylthio)-6-[(R)-1-phenylthioethyl]-7-oxo-1-azabicyclo[3.2.0]-hept-2-ene-2-carboxylate (e 103) (10 mg) in a mixture of dioxan and water (7:3, 5 ml) was added to 5% palladium on carbon catalyst which had been prehydrogenated for 30 min. in a mixture of dioxan and water (7:3, 5 ml). The mixture was hydrogenated at slightly super-atmospheric pressure for 4 hr., when sodium hydrogen carbonate (1.9 mg) was added to the mixture. The catalyst was filtered off and washed with water (5 ml). The filtrate was evaporated in vacuo to remove most of the dioxan and the resultant mixture was diluted with water to 25 ml and extracted with ethyl acetate. The aqueous extract was washed with ethyl acetate. The u.v. spectrum of the

aqueous layer showed a miximum of about 300 nm, indicating the presence of the sodium salt. The volume of the aqueous solution was reduced to about 1 ml, loaded, onto a Biogel P-2 column and eluted with water to give sodium (5R, 6S)-3-(2-acetamidoethylthio)-6-[(R)-1-phenylthioethyl]-7-oxo-1-azabicyclo[3.2.0]hept-2-ene-2-carboxylate (e 104).

Example 82

Sodium (5R, 6R)-3-[(E)-2-acetamidoethenylthio]-6-ethyl-7-oxo-1-azabicyclo[3.2.0]hept-2-ene-2-carboxylate

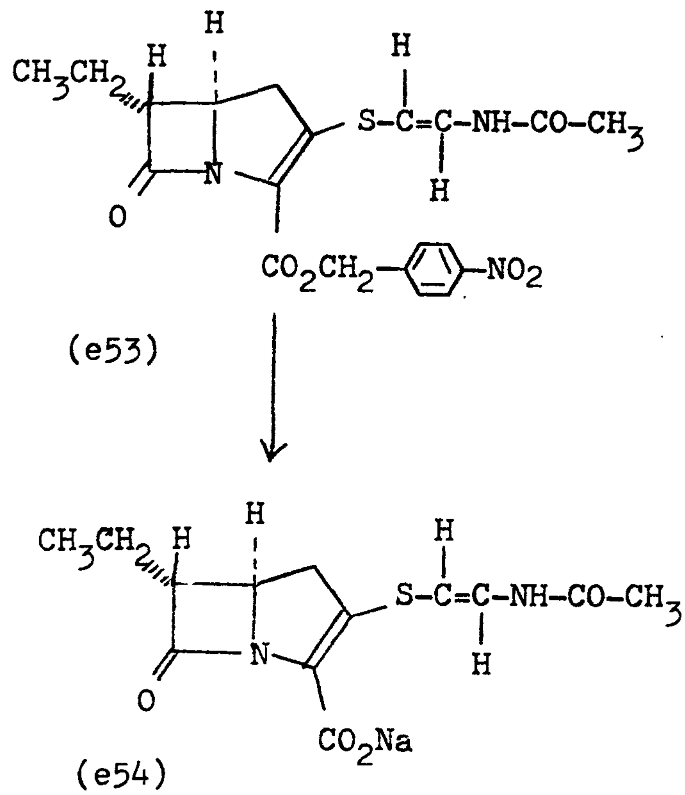

(e53)

(e54)

A solution of the ester (e53) (235 mg) in dioxan (5 ml) was added to a mixture of 5% Pd-C in 30% aqueous dioxan (20 ml) which had been prehydrogenated for 0.5 h. Hydrogenation was continued at ambient pressure and temperature for a further 4 h. A solution of $NaHCO_3$ (46 mg) in water (5 ml) was added to the mixture, which was then filtered through Celite, washing with water (10 ml). The solution was concentrated in vacuo to a volume of about 25 ml and was then washed with ethyl acetate (3 x 50 ml) before concentration further to about 5 ml. The aqueous solution was introduced onto a column of Biogel P2 (20 x 3.5 cm) which was then eluted with water. Fractions were monitored by uv and those containing the product were combined and evaporated in

vacuo. Ethanol (20 ml) was added and the solution was again evaporated in vacuo and the process repeated to remove the last traces of water. Toluene (2 x 20 ml) was added and evaporation in vacuo continued until the sodium salt (e 54) was obtained as a buff-coloured solid (62 mg); λ max. ($H_2O$) 307 and 228 nm. ν max. (KBr) 1755, 1675 and 1620 $cm^{-1}$.

Claims

1. A compound of the formula (I):

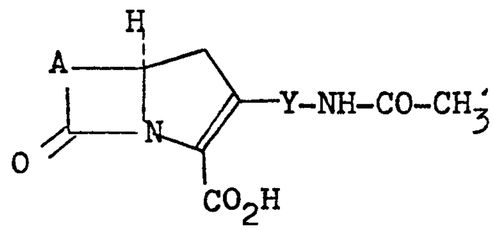

(I)

or a salt or cleavable ester thereof wherein A is a $C=CH-CH_3$ or $CH-CH_2-CH_3$ group and Y is a $-S-CH_2-CH_2-$, $-S-CH=CH-$ or $-SO-CH=CH-$ group; excluding biologically produced (5R, 6R)-3-(2-acetamidoethylthio)-6-ethyl-7-oxo-1-azabicyclo[3.2.0]hept-2-ene-2-carboxylic acid and its salts.

2. A compound as claimed in claim 1 wherein A is a $CH-CH_2-CH_3$ group.

3. The compound of the formula (II):

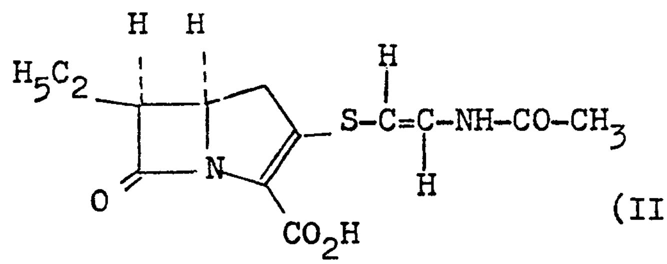

(II)

or a pharmaceutically acceptable salt or in-vivo hydrolysable ester thereof.

4. The compound of the formula (III):

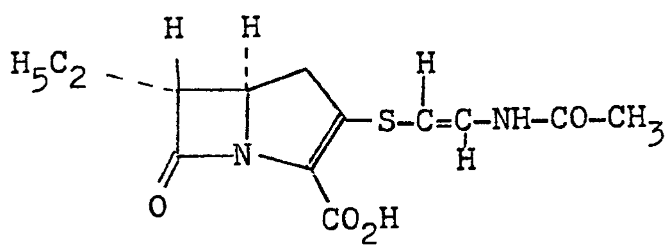

(III)

or a pharmaceutically acceptable salt or in-vivo hydrolysable ester thereof.

5. The compound of the formula (IV):

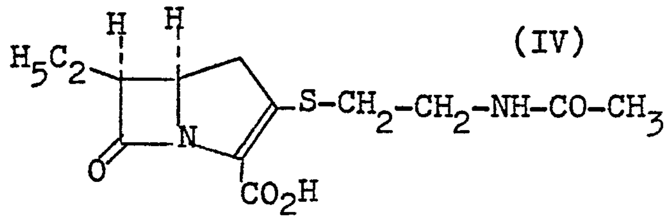

(IV)

or a pharmaceutically acceptable salt or in-vivo hydrolysable ester thereof.

6. The compound of the formula (V):

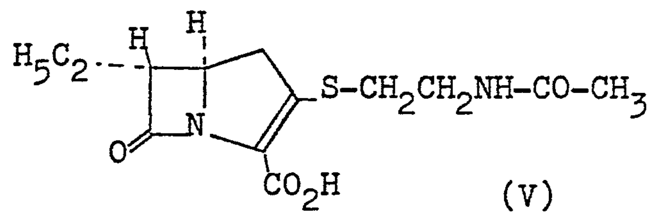

(V)

or a pharmaceutically acceptable salt in-vivo hydrolysable ester thereof with the proviso that the compound has been produced by chemical modification of the 6-position substituent.

7. A compound as claimed in any preceding claim in the form of an in-vivo hydrolysable ester.

8. A compound as claimed in any of claims 1 - 6 in the form of the free acid or a pharmaceutically acceptable salt.

9. A compound as claimed in claim 2 wherein the configuration about the β-lactam ring is _cis_ or as claimed in claim 3 or 5, when substantially free of the corresponding _trans_ isomer.

10. A compound as claimed in claim 2 wherein the configuration about the β-lactam ring is _trans_ or as claimed in claim 4 or claim 6, when substantially free of the corresponding _cis_ isomer.

11. A compound as claimed in claim 1 wherein A is a $C=CH-CH_3$ group.

12. The compound of the formula (VI):

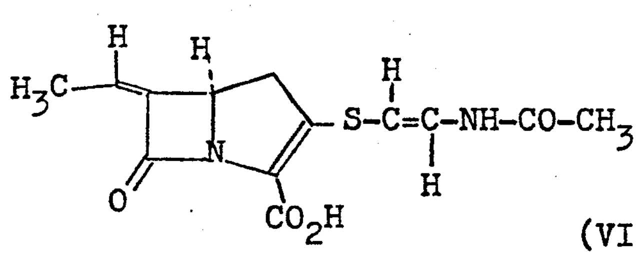

(VI)

or a salt or cleavable ester thereof.

13. The compound of the formula (VII):

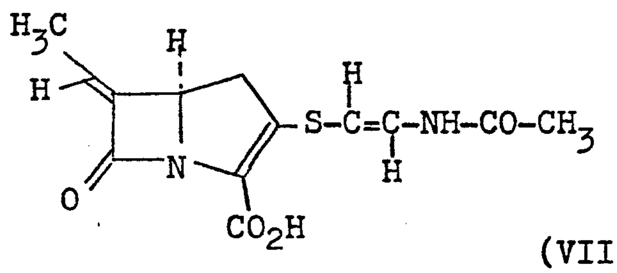

(VII)

or a salt or cleavable ester thereof.

14. The compound of the formula (VIII):

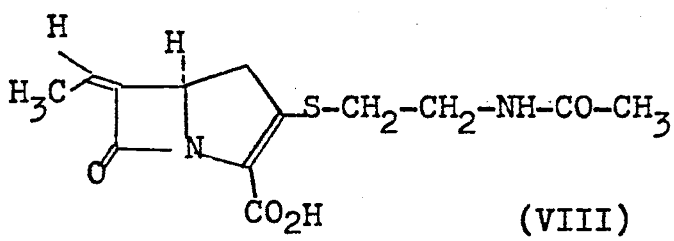

(VIII)

or a salt or cleavable ester thereof.

15. The compound of the formula (IX):

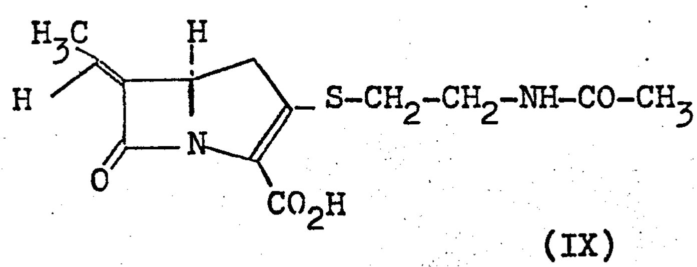

(IX)

or a salt or cleavable ester thereof.

16. A compound according to any one of claims 1, 2 or 9 - 15 in the form of a p-nitrobenzyl ester.

17. A pharmaceutical composition comprising a compound of the formula (I) - (IX) as defined in claims 1, 3 - 6 or 12 - 15 or a pharmaceutically acceptable salt or in-vivo hydrolysable ester thereof and a pharmaceutically acceptable carrier.

18. A composition as claimed in claim 17 comprising a compound according to any of claims 2 - 10.

19. A composition as claimed in claim 17 or claim 18 which also comprises a penicillin or cephalosporin.

20. A process for the preparation of a compound according to claim 1 wherein A is a $CH-CH_2-CH_3$ group, which process comprises the reduction of a corresponding compound of the formula (I) wherein A is a $CH=CH-CH_3$ group, and thereafter if desired de-esterifying the esterified carboxyl group.

21. A process as claimed in claim 20 wherein the reduction is effected by hydrogenation in the presence of a noble metal catalyst.

22. A process as claimed in claim 20 wherein the reduction is effected using a borohydride.

23. A process for the preparation of a compound according to claim 1 wherein A is a $C=CH-CH_3$ group which process comprises either:

(a) the elimination of the elements of a compound of the formula (XV):

$$R_7-(O)_n-SO_3H \qquad (XV)$$

wherein $R_7$ is a lower alkyl, aryl or lower aralkyl group and n is 0; or $R_7$ is a lower alkyl, aryl or

lower aralkyl group or a cation and n is 1, from a compound of the formula (XVI):

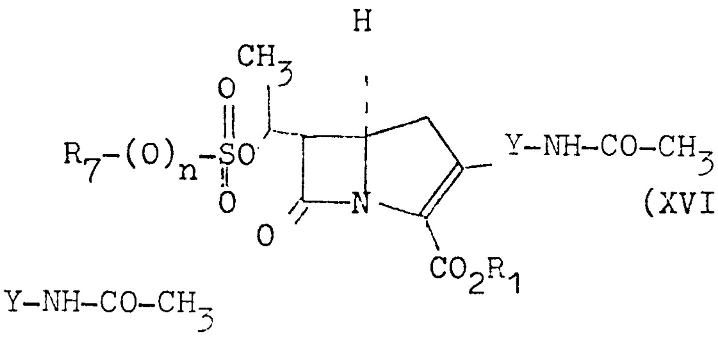

(XVI)

wherein Y-NH-CO-$CH_3$ is as defined in relation to formula (I); $CO_2R_1$ is a cleavable ester group; and $R_7$ and n are as defined in relation to formula (XV);

or

(b) the elimination of the elements of water from a compound of the formula (XVII):

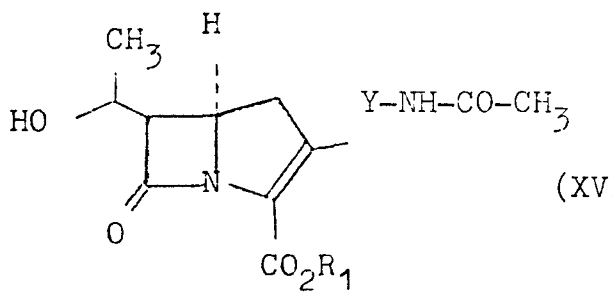

(XVII)

wherein Y-NH-CO-$CH_3$ is as defined in relation to formula (I) and $CO_2R_1$ is a cleavable ester group, in the presence of a compound of the formula (XVIII):

$$R_8O_2C-N=N-CO_2R_9$$

wherein $R_8$ and $R_9$ are independently lower alkyl, aryl or lower alkyl-aryl, and a compound of the formula (XIX):

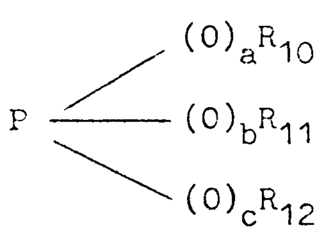

(XIX)

wherein a, b and c are independently 0 or 1, and $R_{10}$, $R_{11}$ and $R_{12}$ are independently lower alkyl, aryl or lower alkyl-aryl and thereafter, if desired, de-esterifying the carboxyl group.

24. A compound as claimed in claim 1 produced in any Example herein.

European Patent Office

# EUROPEAN SEARCH REPORT

0005349

Application number

EP 79 30 0712

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| P | DE - A - 2 815 157 (MERCK)<br>* claims and page 9, lines 1-5; page 16, lines 5-12 *<br>--- | 1,2,11, 16-23 | C 07 D 487/04<br>A 61 K 31/40//<br>(C 07 D 487/04<br>C 07 D 209/00<br>C 07 D 205/00)<br>(A 61 K 31/40<br>A 61 K 31/43)<br>(A 61 K 31/40<br>A 61 K 31/545) |
| E | EP - A - 0 001 567 (SANRAKU-OCEAN)<br>* claims *<br>--------- | 1-4,7-10,16-19 | |
| | | | TECHNICAL FIELDS SEARCHED (Int.Cl.²) |
| | | | C 07 D 487/04<br>A 61 K 31/40<br>A 61 K 31/43<br>A 61 K 31/545 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| The Hague | 17-07-1979 | CHOULY |

EPO Form 1503.1 06.78

BAD ORIGINAL